(19) Europäisches Patentamt
European Patent Office
Office européen des brevets

(11) EP 4 067 384 A1

(12) **EUROPEAN PATENT APPLICATION**
published in accordance with Art. 153(4) EPC

(43) Date of publication:
**05.10.2022 Bulletin 2022/40**

(21) Application number: **20893377.0**

(22) Date of filing: **27.11.2020**

(51) International Patent Classification (IPC):
***C07K 16/30*** *(2006.01)* ***C12N 15/13*** *(2006.01)*
***A61K 47/68*** *(2017.01)* ***G01N 33/68*** *(2006.01)*
***A61K 39/395*** *(2006.01)* ***A61P 35/00*** *(2006.01)*

(52) Cooperative Patent Classification (CPC):
**A61K 39/395; A61K 47/68; A61P 35/00; C07K 16/00; C07K 16/30; G01N 33/68**

(86) International application number:
**PCT/CN2020/132456**

(87) International publication number:
**WO 2021/104496 (03.06.2021 Gazette 2021/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.11.2019 CN 201911187949**

(71) Applicants:
- **Sumgen Mab (Beijing) Biotech Co., Ltd.**
  **Beijing 100176 (CN)**
- **Hangzhou Sumgen Biotech Co., Ltd.**
  **Hangzhou, Zhejiang 310056 (CN)**

(72) Inventors:
- **LV, Ming**
  **Beijing 100176 (CN)**
- **DING, Xiaoran**
  **Beijing 100176 (CN)**
- **MIAO, Shiwei**
  **Beijing 100176 (CN)**
- **TAN, Bin**
  **Beijing 100176 (CN)**
- **WANG, Xuegong**
  **Beijing 100176 (CN)**

(74) Representative: **Weickmann & Weickmann PartmbB**
**Postfach 860 820**
**81635 München (DE)**

(54) **SEPARATED ANTIGEN AXL BINDING PROTEIN AND USE THEREOF**

(57) Provided is a separated antigen binding protein, containing at least one CDR in VH with the amino acid sequence as shown in SEQ ID NO: 1 or SEQ ID NO: 46; and at least one CDR in VL with the amino acid sequence as shown in SEQ ID NO: 2. Also provided are an immunoconjugate containing the separated antigen binding protein, nucleic acid coding the separated antigen binding protein, a carrier containing the separated antigen binding protein, a cell containing the nucleic acid or the carrier, a method for preparing the separated antigen binding protein, and use of the separated antigen binding protein.

EP 4 067 384 A1

Sequence Listing

<110> SUMGEN MAB (BEIJING) BIOTECH CO., LTD. , HANGZHOU SUMGEN BIOTECH CO., LTD.

<120> SEPARATED ANTIGEN AXL BINDING PROTEIN AND USE THEREOF

<130> 0070-PA-022EP

<160> 46

<170> PatentIn version 3.5

<210> 1
<211> 119
<212> PRT
<213> Artificial Sequence

<220>
<223> VH general formula

<220>
<221> X
<222> (5)..(5)
<223> X is K or V

<220>
<221> X
<222> (23)..(23)
<223> X is S or A

<220>
<221> X
<222> (25)..(25)
<223> X is T or S

<220>
<221> X
<222> (27)..(27)
<223> X is F, Y, D or S

<220>
<221> X
<222> (41)..(41)
<223> X is F or S

<220>
<221> X
<222> (44)..(44)
<223> X is N or Q

<220>
<221> X
<222> (45)..(45)
<223> X is K or G

<220>
<221> X
<222> (88)..(88)
<223> X is T or S

<220>

<221> X
<222> (114)..(114)
<223> X is S or T

<400> 1

Gln Val Gln Leu Xaa Gln Ser Gly Pro Gly Leu Val Lys Pro Ser Gln
1 5 10 15

Ser Leu Ser Leu Thr Cys Xaa Val Xaa Gly Xaa Ser Ile Ser Ser Gly
20 25 30

Tyr Tyr Trp Asn Trp Ile Arg Gln Xaa Pro Gly Xaa Xaa Leu Glu Trp
35 40 45

Met Gly Tyr Arg Ser Tyr Asp Gly Ser Asn Asn Tyr Asn Pro Ser Leu
50 55 60

Lys Asn Arg Ile Ser Ile Thr Arg Asp Thr Ser Lys Asn Gln Phe Phe
65 70 75 80

Leu Lys Leu Asn Ser Val Thr Xaa Glu Asp Thr Ala Thr Tyr Tyr Cys
85 90 95

Ala Arg Gly Trp Leu Leu His Tyr Thr Met Asp Tyr Trp Gly Gln Gly
100 105 110

Thr Xaa Val Thr Val Ser Ser
115

<210> 2
<211> 112
<212> PRT
<213> Artificial Sequence

<220>
<223> VL general formula

<220>
<221> X
<222> (1)..(1)
<223> X is E or D

<220>
<221> X
<222> (2)..(2)
<223> X is L or I

<220>
<221> X
<222> (9)..(9)
<223> X is F or S

<220>

<221> X
<222> (11)..(11)
<223> X is N or V

<220>
<221> X
<222> (12)..(12)
<223> X is A or S

<220>
<221> X
<222> (17)..(17)
<223> X is T or Q

<220>
<221> X
<222> (42)..(42)
<223> X is L or Q

<220>
<221> X
<222> (69)..(69)
<223> X is S or G

<220>
<221> X
<222> (79)..(79)
<223> X is R or K

<220>
<221> X
<222> (105)..(105)
<223> X is A or G

<220>
<221> X
<222> (111)..(111)
<223> X is L or I

<400> 2

```
Xaa Xaa Val Met Thr Gln Ser Pro Xaa Ser Xaa Xaa Val Thr Leu Gly
1               5                   10                  15

Xaa Ser Ala Ser Ile Ser Cys Arg Ser Ser Arg Ser Leu Leu His Ser
            20                  25                  30

Asn Gly Phe Thr Tyr Leu Tyr Trp Tyr Xaa Gln Lys Pro Gly Gln Ser
        35                  40                  45

Pro Gln Leu Leu Ile Tyr Gln Met Ser Asn Leu Ala Ser Gly Val Pro
    50                  55                  60

Asp Arg Phe Ser Xaa Ser Gly Ser Gly Thr Asp Phe Thr Leu Xaa Ile
65                  70                  75                  80

Ser Arg Val Glu Ala Glu Asp Val Gly Val Tyr Tyr Cys Gly Gln Asn
                85                  90                  95
```

Leu Glu Leu Pro Leu Thr Phe Gly Xaa Gly Thr Lys Leu Glu Xaa Lys
100 105 110

<210> 3
<211> 119
<212> PRT
<213> Artificial Sequence

<220>
<223> 6G12M11 VH

<400> 3

Gln Val Gln Leu Val Gln Ser Gly Pro Gly Leu Val Lys Pro Ser Gln
1 5 10 15

Ser Leu Ser Leu Thr Cys Ala Val Thr Gly Phe Ser Ile Ser Ser Gly
20 25 30

Tyr Tyr Trp Asn Trp Ile Arg Gln Phe Pro Gly Gln Lys Leu Glu Trp
35 40 45

Met Gly Tyr Arg Ser Tyr Asp Gly Ser Asn Asn Tyr Asn Pro Ser Leu
50 55 60

Lys Asn Arg Ile Ser Ile Thr Arg Asp Thr Ser Lys Asn Gln Phe Phe
65 70 75 80

Leu Lys Leu Asn Ser Val Thr Ser Glu Asp Thr Ala Thr Tyr Tyr Cys
85 90 95

Ala Arg Gly Trp Leu Leu His Tyr Thr Met Asp Tyr Trp Gly Gln Gly
100 105 110

Thr Thr Val Thr Val Ser Ser
115

<210> 4
<211> 112
<212> PRT
<213> Artificial Sequence

<220>
<223> 6G12M11 VL

<400> 4

Glu Ile Val Met Thr Gln Ser Pro Ser Ser Val Ser Val Thr Leu Gly
1 5 10 15

Gln Ser Ala Ser Ile Ser Cys Arg Ser Ser Arg Ser Leu Leu His Ser

20 25 30

Asn Gly Phe Thr Tyr Leu Tyr Trp Tyr Leu Gln Lys Pro Gly Gln Ser
35 40 45

Pro Gln Leu Leu Ile Tyr Gln Met Ser Asn Leu Ala Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Asn
85 90 95

Leu Glu Leu Pro Leu Thr Phe Gly Gly Gly Thr Lys Leu Glu Ile Lys
100 105 110

<210> 5
<211> 119
<212> PRT
<213> Artificial Sequence

<220>
<223> 6G12M21 VH

<400> 5

Gln Val Gln Leu Val Gln Ser Gly Pro Gly Leu Val Lys Pro Ser Gln
1 5 10 15

Ser Leu Ser Leu Thr Cys Ser Val Thr Gly Phe Ser Ile Ser Ser Gly
20 25 30

Tyr Tyr Trp Asn Trp Ile Arg Gln Phe Pro Gly Gln Lys Leu Glu Trp
35 40 45

Met Gly Tyr Arg Ser Tyr Asp Gly Ser Asn Asn Tyr Asn Pro Ser Leu
50 55 60

Lys Asn Arg Ile Ser Ile Thr Arg Asp Thr Ser Lys Asn Gln Phe Phe
65 70 75 80

Leu Lys Leu Asn Ser Val Thr Ser Glu Asp Thr Ala Thr Tyr Tyr Cys
85 90 95

Ala Arg Gly Trp Leu Leu His Tyr Thr Met Asp Tyr Trp Gly Gln Gly
100 105 110

Thr Thr Val Thr Val Ser Ser
115

<210> 6
<211> 112
<212> PRT
<213> Artificial Sequence

<220>
<223> 6G12M21/6G12M31/6G12M41 VL

<400> 6

```
Asp Ile Val Met Thr Gln Ser Pro Ser Ser Val Ser Val Thr Leu Gly
1               5                   10                  15

Gln Ser Ala Ser Ile Ser Cys Arg Ser Ser Arg Ser Leu Leu His Ser
            20                  25                  30

Asn Gly Phe Thr Tyr Leu Tyr Trp Tyr Leu Gln Lys Pro Gly Gln Ser
        35                  40                  45

Pro Gln Leu Leu Ile Tyr Gln Met Ser Asn Leu Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Gly Gln Asn
                85                  90                  95

Leu Glu Leu Pro Leu Thr Phe Gly Gly Gly Thr Lys Leu Glu Ile Lys
            100                 105                 110
```

<210> 7
<211> 30
<212> PRT
<213> Artificial Sequence

<220>
<223> H-FR1 general formula

<220>
<221> X
<222> (5)..(5)
<223> X is K or V

<220>
<221> X
<222> (23)..(23)
<223> X is S or A

<220>
<221> X
<222> (25)..(25)
<223> X is T or S

<220>
<221> X
<222> (27)..(27)
<223> X is F, Y, D or S

<400> 7

Gln Val Gln Leu Xaa Gln Ser Gly Pro Gly Leu Val Lys Pro Ser Gln
1 5 10 15

Ser Leu Ser Leu Thr Cys Xaa Val Xaa Gly Xaa Ser Ile Ser
20 25 30

<210> 8
<211> 14
<212> PRT
<213> Artificial Sequence

<220>
<223> H-FR2 general formula

<220>
<221> X
<222> (5)..(5)
<223> X is F or S

<220>
<221> X
<222> (8)..(8)
<223> X is N or Q

<220>
<221> X
<222> (9)..(9)
<223> X is K or G

<400> 8

Trp Ile Arg Gln Xaa Pro Gly Xaa Xaa Leu Glu Trp Met Gly
1 5 10

<210> 9
<211> 32
<212> PRT
<213> Artificial Sequence

<220>
<223> H-FR3 general formula

<220>
<221> X
<222> (22)..(22)
<223> X is T or S

<400> 9

Arg Ile Ser Ile Thr Arg Asp Thr Ser Lys Asn Gln Phe Phe Leu Lys

1 5 10 15

Leu Asn Ser Val Thr Xaa Glu Asp Thr Ala Thr Tyr Tyr Cys Ala Arg
20 25 30

<210> 10
<211> 11
<212> PRT
<213> Artificial Sequence

<220>
<223> H-FR4 general formula

<220>
<221> X
<222> (6)..(6)
<223> X is S or T

<400> 10

Trp Gly Gln Gly Thr Xaa Val Thr Val Ser Ser
1 5 10

<210> 11
<211> 30
<212> PRT
<213> Artificial Sequence

<220>
<223> 6G12M11 H-FR1

<400> 11

Gln Val Gln Leu Val Gln Ser Gly Pro Gly Leu Val Lys Pro Ser Gln
1 5 10 15

Ser Leu Ser Leu Thr Cys Ala Val Thr Gly Phe Ser Ile Ser
20 25 30

<210> 12
<211> 14
<212> PRT
<213> Artificial Sequence

<220>
<223> 6G12M11/6G12M121/6G12M31/6G12M41 H-FR2

<400> 12

Trp Ile Arg Gln Phe Pro Gly Gln Lys Leu Glu Trp Met Gly
1 5 10

<210> 13
<211> 32
<212> PRT
<213> Artificial Sequence

<220>
<223> 6G12M11/6G12M21/6G12M31/6G12M41 H-FR3

<400> 13

Arg Ile Ser Ile Thr Arg Asp Thr Ser Lys Asn Gln Phe Phe Leu Lys
1 5 10 15

Leu Asn Ser Val Thr Ser Glu Asp Thr Ala Thr Tyr Tyr Cys Ala Arg
20 25 30

<210> 14
<211> 11
<212> PRT
<213> Artificial Sequence

<220>
<223> 6G12M11/6G12M21/6G12M31/6G12M41 H-FR4

<400> 14

Trp Gly Gln Gly Thr Thr Val Thr Val Ser Ser
1 5 10

<210> 15
<211> 30
<212> PRT
<213> Artificial Sequence

<220>
<223> 6G12M21/6G12M31/6G12M41 H-FR1

<400> 15

Gln Val Gln Leu Val Gln Ser Gly Pro Gly Leu Val Lys Pro Ser Gln
1 5 10 15

Ser Leu Ser Leu Thr Cys Ser Val Thr Gly Phe Ser Ile Ser
20 25 30

<210> 16
<211> 23
<212> PRT
<213> Artificial Sequence

<220>
<223> L-FR1 general formula

<220>
<221> X
<222> (1)..(1)
<223> X is E or D

<220>
<221> X
<222> (2)..(2)

<223>  X is L or I

<220>
<221>  X
<222>  (9)..(9)
<223>  X is F or S

<220>
<221>  X
<222>  (11)..(11)
<223>  X is N or V

<220>
<221>  X
<222>  (12)..(12)
<223>  X is A or S

<220>
<221>  X
<222>  (17)..(17)
<223>  X is T or Q

<400>  16

```
Xaa Xaa Val Met Thr Gln Ser Pro Xaa Ser Xaa Xaa Val Thr Leu Gly
1               5                   10                  15

Xaa Ser Ala Ser Ile Ser Cys
            20
```

<210>  17
<211>  15
<212>  PRT
<213>  Artificial Sequence

<220>
<223>  L-FR2 general formula

<220>
<221>  X
<222>  (3)..(3)
<223>  X is L or Q

<400>  17

```
Trp Tyr Xaa Gln Lys Pro Gly Gln Ser Pro Gln Leu Leu Ile Tyr
1               5                   10                  15
```

<210>  18
<211>  32
<212>  PRT
<213>  Artificial Sequence

<220>
<223>  L-FR3 general formula

<220>
<221>  X

<222> (8)..(8)
<223> X is S or G

<220>
<221> X
<222> (18)..(18)
<223> X is R or K

<400> 18

Gly Val Pro Asp Arg Phe Ser Xaa Ser Gly Ser Gly Thr Asp Phe Thr
1 5 10 15

Leu Xaa Ile Ser Arg Val Glu Ala Glu Asp Val Gly Val Tyr Tyr Cys
20 25 30

<210> 19
<211> 10
<212> PRT
<213> Artificial Sequence

<220>
<223> L-FR4 general formula

<220>
<221> X
<222> (3)..(3)
<223> X is A or G

<220>
<221> X
<222> (9)..(9)
<223> X is L or I

<400> 19

Phe Gly Xaa Gly Thr Lys Leu Glu Xaa Lys
1 5 10

<210> 20
<211> 23
<212> PRT
<213> Artificial Sequence

<220>
<223> 6G12M11 L-FR1

<400> 20

Glu Ile Val Met Thr Gln Ser Pro Ser Ser Val Ser Val Thr Leu Gly
1 5 10 15

Gln Ser Ala Ser Ile Ser Cys
20

<210> 21
<211> 15

<212> PRT
<213> Artificial Sequence

<220>
<223> 6G12M11/6G12M21/6G12M31/6G12M41 L-FR2

<400> 21

Trp Tyr Leu Gln Lys Pro Gly Gln Ser Pro Gln Leu Leu Ile Tyr
1 5 10 15

<210> 22
<211> 32
<212> PRT
<213> Artificial Sequence

<220>
<223> 6G12M11/6G12M21/6G12M31/6G12M41 L-FR3

<400> 22

Gly Val Pro Asp Arg Phe Ser Gly Ser Gly Ser Gly Thr Asp Phe Thr
1 5 10 15

Leu Lys Ile Ser Arg Val Glu Ala Glu Asp Val Gly Val Tyr Tyr Cys
20 25 30

<210> 23
<211> 10
<212> PRT
<213> Artificial Sequence

<220>
<223> 6G12M11/6G12M21/6G12M31/6G12M41 L-FR4

<400> 23

Phe Gly Gly Gly Thr Lys Leu Glu Ile Lys
1 5 10

<210> 24
<211> 23
<212> PRT
<213> Artificial Sequence

<220>
<223> 6G12M21/6G12M31/6G12M41 L-FR1

<400> 24

Asp Ile Val Met Thr Gln Ser Pro Ser Ser Val Ser Val Thr Leu Gly
1 5 10 15

Gln Ser Ala Ser Ile Ser Cys
20

<210> 25

<211> 6
<212> PRT
<213> Artificial Sequence

<220>
<223> 6G12M11/6G12M21/6G12/6G12M31/6G12M41 HCDR1

<400> 25

Ser Gly Tyr Tyr Trp Asn
1 5

<210> 26
<211> 16
<212> PRT
<213> Artificial Sequence

<220>
<223> 6G12M11/6G12M21/6G12 HCDR2

<400> 26

Tyr Arg Ser Tyr Asp Gly Ser Asn Asn Tyr Asn Pro Ser Leu Lys Asn
1 5 10 15

<210> 27
<211> 10
<212> PRT
<213> Artificial Sequence

<220>
<223> 6G12M11/6G12M21/6G12 /6G12M31/6G12M41 HCDR3

<400> 27

Gly Trp Leu Leu His Tyr Thr Met Asp Tyr
1 5 10

<210> 28
<211> 16
<212> PRT
<213> Artificial Sequence

<220>
<223> 6G12M11/6G12M21/6G12 /6G12M31/6G12M41 LCDR1

<400> 28

Arg Ser Ser Arg Ser Leu Leu His Ser Asn Gly Phe Thr Tyr Leu Tyr
1 5 10 15

<210> 29
<211> 7
<212> PRT
<213> Artificial Sequence

<220>
<223> 6G12M11/6G12M21/6G12/6G12M31/6G12M41 LCDR2

<400> 29

Gln Met Ser Asn Leu Ala Ser
1               5

<210> 30
<211> 9
<212> PRT
<213> Artificial Sequence

<220>
<223> 6G12M11/6G12M21/6G12/6G12M31/6G12M41 LCDR3

<400> 30

Gly Gln Asn Leu Glu Leu Pro Leu Thr
1               5

<210> 31
<211> 119
<212> PRT
<213> Artificial Sequence

<220>
<223> 6G12 VH

<400> 31

Gln Val Gln Leu Lys Gln Ser Gly Pro Gly Leu Val Lys Pro Ser Gln
1               5                   10                  15

Ser Leu Ser Leu Thr Cys Ser Val Thr Gly Phe Ser Ile Ser Ser Gly
            20                  25                  30

Tyr Tyr Trp Asn Trp Ile Arg Gln Phe Pro Gly Asn Lys Leu Glu Trp
        35                  40                  45

Met Gly Tyr Arg Ser Tyr Asp Gly Ser Asn Asn Tyr Asn Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Thr Glu Asp Thr Ala Thr Tyr Tyr Cys
                85                  90                  95

Ala Arg Gly Trp Leu Leu His Tyr Thr Met Asp Tyr Trp Gly Gln Gly
            100                 105                 110

Thr Ser Val Thr Val Ser Ser
        115

<210> 32

<211> 112
<212> PRT
<213> Artificial Sequence

<220>
<223> 6G12 VL

<400> 32

Glu Leu Val Met Thr Gln Ser Pro Phe Ser Asn Ala Val Thr Leu Gly
1 5 10 15

Thr Ser Ala Ser Ile Ser Cys Arg Ser Ser Arg Ser Leu Leu His Ser
20 25 30

Asn Gly Phe Thr Tyr Leu Tyr Trp Tyr Leu Gln Lys Pro Gly Gln Ser
35 40 45

Pro Gln Leu Leu Ile Tyr Gln Met Ser Asn Leu Ala Ser Gly Val Pro
50 55 60

Asp Arg Phe Ser Ser Ser Gly Ser Gly Thr Asp Phe Thr Leu Arg Ile
65 70 75 80

Ser Arg Val Glu Ala Glu Asp Val Gly Val Tyr Tyr Cys Gly Gln Asn
85 90 95

Leu Glu Leu Pro Leu Thr Phe Gly Ala Gly Thr Lys Leu Glu Leu Lys
100 105 110

<210> 33
<211> 330
<212> PRT
<213> Homo sapiens

<400> 33

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

```
Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330
```

<210> 34
<211> 107
<212> PRT
<213> Homo sapiens

<400> 34

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

Tyr Pro Arg Glu Ala Lys Val Gln Trp Lys Val Asp Asn Ala Leu Gln
35 40 45

Ser Gly Asn Ser Gln Glu Ser Val Thr Glu Gln Asp Ser Lys Asp Ser
50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 35
<211> 449
<212> PRT
<213> Artificial Sequence

<220>
<223> 6G12M11 heavy chain

<400> 35

Gln Val Gln Leu Val Gln Ser Gly Pro Gly Leu Val Lys Pro Ser Gln
1 5 10 15

Ser Leu Ser Leu Thr Cys Ala Val Thr Gly Phe Ser Ile Ser Ser Gly
20 25 30

Tyr Tyr Trp Asn Trp Ile Arg Gln Phe Pro Gly Gln Lys Leu Glu Trp
35 40 45

Met Gly Tyr Arg Ser Tyr Asp Gly Ser Asn Asn Tyr Asn Pro Ser Leu
50 55 60

```
Lys Asn Arg Ile Ser Ile Thr Arg Asp Thr Ser Lys Asn Gln Phe Phe
65                  70                  75                  80

Leu Lys Leu Asn Ser Val Thr Ser Glu Asp Thr Ala Thr Tyr Tyr Cys
                85                  90                  95

Ala Arg Gly Trp Leu Leu His Tyr Thr Met Asp Tyr Trp Gly Gln Gly
            100                 105                 110

Thr Thr Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320
```

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 36
<211> 219
<212> PRT
<213> Artificial Sequence

<220>
<223> 6G12M11 light chain

<400> 36

Glu Ile Val Met Thr Gln Ser Pro Ser Ser Val Ser Val Thr Leu Gly
1 5 10 15

Gln Ser Ala Ser Ile Ser Cys Arg Ser Ser Arg Ser Leu Leu His Ser
20 25 30

Asn Gly Phe Thr Tyr Leu Tyr Trp Tyr Leu Gln Lys Pro Gly Gln Ser
35 40 45

Pro Gln Leu Leu Ile Tyr Gln Met Ser Asn Leu Ala Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Asn
85 90 95

Leu Glu Leu Pro Leu Thr Phe Gly Gly Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 37
<211> 449
<212> PRT
<213> Artificial Sequence

<220>
<223> 6G12M21 heavy chain

<400> 37

Gln Val Gln Leu Val Gln Ser Gly Pro Gly Leu Val Lys Pro Ser Gln
1 5 10 15

Ser Leu Ser Leu Thr Cys Ser Val Thr Gly Phe Ser Ile Ser Ser Gly
20 25 30

Tyr Tyr Trp Asn Trp Ile Arg Gln Phe Pro Gly Gln Lys Leu Glu Trp
35 40 45

Met Gly Tyr Arg Ser Tyr Asp Gly Ser Asn Asn Tyr Asn Pro Ser Leu

```
        50                  55                  60

Lys Asn Arg Ile Ser Ile Thr Arg Asp Thr Ser Lys Asn Gln Phe Phe
65                  70                  75                  80

Leu Lys Leu Asn Ser Val Thr Ser Glu Asp Thr Ala Thr Tyr Tyr Cys
                85                  90                  95

Ala Arg Gly Trp Leu Leu His Tyr Thr Met Asp Tyr Trp Gly Gln Gly
            100                 105                 110

Thr Thr Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300
```

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 38
<211> 219
<212> PRT
<213> Artificial Sequence

<220>
<223> 6G12M21 light chain

<400> 38

Asp Ile Val Met Thr Gln Ser Pro Ser Ser Val Ser Val Thr Leu Gly
1 5 10 15

Gln Ser Ala Ser Ile Ser Cys Arg Ser Ser Arg Ser Leu Leu His Ser
20 25 30

Asn Gly Phe Thr Tyr Leu Tyr Trp Tyr Leu Gln Lys Pro Gly Gln Ser
35 40 45

Pro Gln Leu Leu Ile Tyr Gln Met Ser Asn Leu Ala Ser Gly Val Pro

50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Asn
85 90 95

Leu Glu Leu Pro Leu Thr Phe Gly Gly Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 39
<211> 894
<212> PRT
<213> Homo sapiens

<400> 39

Met Ala Trp Arg Cys Pro Arg Met Gly Arg Val Pro Leu Ala Trp Cys
1 5 10 15

Leu Ala Leu Cys Gly Trp Ala Cys Met Ala Pro Arg Gly Thr Gln Ala
20 25 30

Glu Glu Ser Pro Phe Val Gly Asn Pro Gly Asn Ile Thr Gly Ala Arg
35 40 45

Gly Leu Thr Gly Thr Leu Arg Cys Gln Leu Gln Val Gln Gly Glu Pro

```
        50                  55                  60

Pro Glu Val His Trp Leu Arg Asp Gly Gln Ile Leu Glu Leu Ala Asp
65                  70                  75                  80

Ser Thr Gln Thr Gln Val Pro Leu Gly Glu Asp Glu Gln Asp Asp Trp
                85                  90                  95

Ile Val Val Ser Gln Leu Arg Ile Thr Ser Leu Gln Leu Ser Asp Thr
            100                 105                 110

Gly Gln Tyr Gln Cys Leu Val Phe Leu Gly His Gln Thr Phe Val Ser
        115                 120                 125

Gln Pro Gly Tyr Val Gly Leu Glu Gly Leu Pro Tyr Phe Leu Glu Glu
    130                 135                 140

Pro Glu Asp Arg Thr Val Ala Ala Asn Thr Pro Phe Asn Leu Ser Cys
145                 150                 155                 160

Gln Ala Gln Gly Pro Pro Glu Pro Val Asp Leu Leu Trp Leu Gln Asp
                165                 170                 175

Ala Val Pro Leu Ala Thr Ala Pro Gly His Gly Pro Gln Arg Ser Leu
            180                 185                 190

His Val Pro Gly Leu Asn Lys Thr Ser Ser Phe Ser Cys Glu Ala His
        195                 200                 205

Asn Ala Lys Gly Val Thr Thr Ser Arg Thr Ala Thr Ile Thr Val Leu
    210                 215                 220

Pro Gln Gln Pro Arg Asn Leu His Leu Val Ser Arg Gln Pro Thr Glu
225                 230                 235                 240

Leu Glu Val Ala Trp Thr Pro Gly Leu Ser Gly Ile Tyr Pro Leu Thr
                245                 250                 255

His Cys Thr Leu Gln Ala Val Leu Ser Asp Asp Gly Met Gly Ile Gln
            260                 265                 270

Ala Gly Glu Pro Asp Pro Pro Glu Glu Pro Leu Thr Ser Gln Ala Ser
        275                 280                 285

Val Pro Pro His Gln Leu Arg Leu Gly Ser Leu His Pro His Thr Pro
    290                 295                 300
```

```
Tyr His Ile Arg Val Ala Cys Thr Ser Ser Gln Gly Pro Ser Ser Trp
305                 310                 315                 320

Thr His Trp Leu Pro Val Glu Thr Pro Glu Gly Val Pro Leu Gly Pro
                325                 330                 335

Pro Glu Asn Ile Ser Ala Thr Arg Asn Gly Ser Gln Ala Phe Val His
            340                 345                 350

Trp Gln Glu Pro Arg Ala Pro Leu Gln Gly Thr Leu Leu Gly Tyr Arg
        355                 360                 365

Leu Ala Tyr Gln Gly Gln Asp Thr Pro Glu Val Leu Met Asp Ile Gly
    370                 375                 380

Leu Arg Gln Glu Val Thr Leu Glu Leu Gln Gly Asp Gly Ser Val Ser
385                 390                 395                 400

Asn Leu Thr Val Cys Val Ala Ala Tyr Thr Ala Ala Gly Asp Gly Pro
                405                 410                 415

Trp Ser Leu Pro Val Pro Leu Glu Ala Trp Arg Pro Gly Gln Ala Gln
            420                 425                 430

Pro Val His Gln Leu Val Lys Glu Pro Ser Thr Pro Ala Phe Ser Trp
        435                 440                 445

Pro Trp Trp Tyr Val Leu Leu Gly Ala Val Val Ala Ala Ala Cys Val
    450                 455                 460

Leu Ile Leu Ala Leu Phe Leu Val His Arg Arg Lys Lys Glu Thr Arg
465                 470                 475                 480

Tyr Gly Glu Val Phe Glu Pro Thr Val Glu Arg Gly Glu Leu Val Val
                485                 490                 495

Arg Tyr Arg Val Arg Lys Ser Tyr Ser Arg Arg Thr Thr Glu Ala Thr
            500                 505                 510

Leu Asn Ser Leu Gly Ile Ser Glu Glu Leu Lys Glu Lys Leu Arg Asp
        515                 520                 525

Val Met Val Asp Arg His Lys Val Ala Leu Gly Lys Thr Leu Gly Glu
    530                 535                 540

Gly Glu Phe Gly Ala Val Met Glu Gly Gln Leu Asn Gln Asp Asp Ser
545                 550                 555                 560
```

```
Ile Leu Lys Val Ala Val Lys Thr Met Lys Ile Ala Ile Cys Thr Arg
                565                 570                 575

Ser Glu Leu Glu Asp Phe Leu Ser Glu Ala Val Cys Met Lys Glu Phe
            580                 585                 590

Asp His Pro Asn Val Met Arg Leu Ile Gly Val Cys Phe Gln Gly Ser
        595                 600                 605

Glu Arg Glu Ser Phe Pro Ala Pro Val Val Ile Leu Pro Phe Met Lys
    610                 615                 620

His Gly Asp Leu His Ser Phe Leu Leu Tyr Ser Arg Leu Gly Asp Gln
625                 630                 635                 640

Pro Val Tyr Leu Pro Thr Gln Met Leu Val Lys Phe Met Ala Asp Ile
                645                 650                 655

Ala Ser Gly Met Glu Tyr Leu Ser Thr Lys Arg Phe Ile His Arg Asp
            660                 665                 670

Leu Ala Ala Arg Asn Cys Met Leu Asn Glu Asn Met Ser Val Cys Val
        675                 680                 685

Ala Asp Phe Gly Leu Ser Lys Lys Ile Tyr Asn Gly Asp Tyr Tyr Arg
    690                 695                 700

Gln Gly Arg Ile Ala Lys Met Pro Val Lys Trp Ile Ala Ile Glu Ser
705                 710                 715                 720

Leu Ala Asp Arg Val Tyr Thr Ser Lys Ser Asp Val Trp Ser Phe Gly
                725                 730                 735

Val Thr Met Trp Glu Ile Ala Thr Arg Gly Gln Thr Pro Tyr Pro Gly
            740                 745                 750

Val Glu Asn Ser Glu Ile Tyr Asp Tyr Leu Arg Gln Gly Asn Arg Leu
        755                 760                 765

Lys Gln Pro Ala Asp Cys Leu Asp Gly Leu Tyr Ala Leu Met Ser Arg
    770                 775                 780

Cys Trp Glu Leu Asn Pro Gln Asp Arg Pro Ser Phe Thr Glu Leu Arg
785                 790                 795                 800

Glu Asp Leu Glu Asn Thr Leu Lys Ala Leu Pro Pro Ala Gln Glu Pro
                805                 810                 815
```

Asp Glu Ile Leu Tyr Val Asn Met Asp Glu Gly Gly Gly Tyr Pro Glu
820 825 830

Pro Pro Gly Ala Ala Gly Gly Ala Asp Pro Pro Thr Gln Pro Asp Pro
835 840 845

Lys Asp Ser Cys Ser Cys Leu Thr Ala Ala Glu Val His Pro Ala Gly
850 855 860

Arg Tyr Val Leu Cys Pro Ser Thr Thr Pro Ser Pro Ala Gln Pro Ala
865 870 875 880

Asp Arg Gly Ser Pro Ala Ala Pro Gly Gln Glu Asp Gly Ala
885 890

<210> 40
<211> 425
<212> PRT
<213> Artificial Sequence

<220>
<223> human AXL protein extracellular domain

<400> 40

Pro Arg Gly Thr Gln Ala Glu Glu Ser Pro Phe Val Gly Asn Pro Gly
1 5 10 15

Asn Ile Thr Gly Ala Arg Gly Leu Thr Gly Thr Leu Arg Cys Gln Leu
20 25 30

Gln Val Gln Gly Glu Pro Pro Glu Val His Trp Leu Arg Asp Gly Gln
35 40 45

Ile Leu Glu Leu Ala Asp Ser Thr Gln Thr Gln Val Pro Leu Gly Glu
50 55 60

Asp Glu Gln Asp Asp Trp Ile Val Val Ser Gln Leu Arg Ile Thr Ser
65 70 75 80

Leu Gln Leu Ser Asp Thr Gly Gln Tyr Gln Cys Leu Val Phe Leu Gly
85 90 95

His Gln Thr Phe Val Ser Gln Pro Gly Tyr Val Gly Leu Glu Gly Leu
100 105 110

Pro Tyr Phe Leu Glu Glu Pro Glu Asp Arg Thr Val Ala Ala Asn Thr
115 120 125

Pro Phe Asn Leu Ser Cys Gln Ala Gln Gly Pro Pro Glu Pro Val Asp
    130                 135                 140

Leu Leu Trp Leu Gln Asp Ala Val Pro Leu Ala Thr Ala Pro Gly His
145                 150                 155                 160

Gly Pro Gln Arg Ser Leu His Val Pro Gly Leu Asn Lys Thr Ser Ser
                165                 170                 175

Phe Ser Cys Glu Ala His Asn Ala Lys Gly Val Thr Thr Ser Arg Thr
            180                 185                 190

Ala Thr Ile Thr Val Leu Pro Gln Gln Pro Arg Asn Leu His Leu Val
        195                 200                 205

Ser Arg Gln Pro Thr Glu Leu Glu Val Ala Trp Thr Pro Gly Leu Ser
    210                 215                 220

Gly Ile Tyr Pro Leu Thr His Cys Thr Leu Gln Ala Val Leu Ser Asp
225                 230                 235                 240

Asp Gly Met Gly Ile Gln Ala Gly Glu Pro Asp Pro Pro Glu Glu Pro
                245                 250                 255

Leu Thr Ser Gln Ala Ser Val Pro Pro His Gln Leu Arg Leu Gly Ser
            260                 265                 270

Leu His Pro His Thr Pro Tyr His Ile Arg Val Ala Cys Thr Ser Ser
        275                 280                 285

Gln Gly Pro Ser Ser Trp Thr His Trp Leu Pro Val Glu Thr Pro Glu
    290                 295                 300

Gly Val Pro Leu Gly Pro Pro Glu Asn Ile Ser Ala Thr Arg Asn Gly
305                 310                 315                 320

Ser Gln Ala Phe Val His Trp Gln Glu Pro Arg Ala Pro Leu Gln Gly
                325                 330                 335

Thr Leu Leu Gly Tyr Arg Leu Ala Tyr Gln Gly Gln Asp Thr Pro Glu
            340                 345                 350

Val Leu Met Asp Ile Gly Leu Arg Gln Glu Val Thr Leu Glu Leu Gln
        355                 360                 365

Gly Asp Gly Ser Val Ser Asn Leu Thr Val Cys Val Ala Ala Tyr Thr
    370                 375                 380

Ala Ala Gly Asp Gly Pro Trp Ser Leu Pro Val Pro Leu Glu Ala Trp
385 390 395 400

Arg Pro Gly Gln Ala Gln Pro Val His Gln Leu Val Lys Glu Pro Ser
405 410 415

Thr Pro Ala Phe Ser Trp Pro Trp Trp
420 425

<210> 41
<211> 229
<212> PRT
<213> Homo sapiens

<400> 41

Glu Ser Lys Tyr Gly Pro Pro Cys Pro Pro Cys Pro Ala Pro Glu Phe
1 5 10 15

Leu Gly Gly Pro Ser Val Phe Leu Phe Pro Pro Lys Pro Lys Asp Thr
20 25 30

Leu Met Ile Ser Arg Thr Pro Glu Val Thr Cys Val Val Val Asp Val
35 40 45

Ser Gln Glu Asp Pro Glu Val Gln Phe Asn Trp Tyr Val Asp Gly Val
50 55 60

Glu Val His Asn Ala Lys Thr Lys Pro Arg Glu Glu Gln Phe Asn Ser
65 70 75 80

Thr Tyr Arg Val Val Ser Val Leu Thr Val Leu His Gln Asp Trp Leu
85 90 95

Asn Gly Lys Glu Tyr Lys Cys Lys Val Ser Asn Lys Gly Leu Pro Ser
100 105 110

Ser Ile Glu Lys Thr Ile Ser Lys Ala Lys Gly Gln Pro Arg Glu Pro
115 120 125

Gln Val Tyr Thr Leu Pro Pro Ser Gln Glu Glu Met Thr Lys Asn Gln
130 135 140

Val Ser Leu Thr Cys Leu Val Lys Gly Phe Tyr Pro Ser Asp Ile Ala
145 150 155 160

Val Glu Trp Glu Ser Asn Gly Gln Pro Glu Asn Asn Tyr Lys Thr Thr
165 170 175

Pro Pro Val Leu Asp Ser Asp Gly Ser Phe Phe Leu Tyr Ser Arg Leu
180 185 190

Thr Val Asp Lys Ser Arg Trp Gln Glu Gly Asn Val Phe Ser Cys Ser
195 200 205

Val Met His Glu Ala Leu His Asn His Tyr Thr Gln Lys Ser Leu Ser
210 215 220

Leu Ser Leu Gly Lys
225

<210> 42
<211> 119
<212> PRT
<213> Artificial Sequence

<220>
<223> 6G12M31 VH

<400> 42

Gln Val Gln Leu Val Gln Ser Gly Pro Gly Leu Val Lys Pro Ser Gln
1 5 10 15

Ser Leu Ser Leu Thr Cys Ser Val Thr Gly Phe Ser Ile Ser Ser Gly
20 25 30

Tyr Tyr Trp Asn Trp Ile Arg Gln Phe Pro Gly Gln Lys Leu Glu Trp
35 40 45

Met Gly Tyr Arg Ser Tyr Asp Gly Ser Asn Asn Tyr Asp Pro Ser Leu
50 55 60

Lys Asn Arg Ile Ser Ile Thr Arg Asp Thr Ser Lys Asn Gln Phe Phe
65 70 75 80

Leu Lys Leu Asn Ser Val Thr Ser Glu Asp Thr Ala Thr Tyr Tyr Cys
85 90 95

Ala Arg Gly Trp Leu Leu His Tyr Thr Met Asp Tyr Trp Gly Gln Gly
100 105 110

Thr Thr Val Thr Val Ser Ser
115

<210> 43
<211> 119
<212> PRT
<213> Artificial Sequence

<220>
<223> 6G12M41 VH

<400> 43

Gln Val Gln Leu Val Gln Ser Gly Pro Gly Leu Val Lys Pro Ser Gln
1               5                   10                  15

Ser Leu Ser Leu Thr Cys Ser Val Thr Gly Phe Ser Ile Ser Ser Gly
            20                  25                  30

Tyr Tyr Trp Asn Trp Ile Arg Gln Phe Pro Gly Gln Lys Leu Glu Trp
        35                  40                  45

Met Gly Tyr Arg Ser Tyr Asp Gly Ser Asn Asn Tyr Gly Pro Ser Leu
    50                  55                  60

Lys Asn Arg Ile Ser Ile Thr Arg Asp Thr Ser Lys Asn Gln Phe Phe
65                  70                  75                  80

Leu Lys Leu Asn Ser Val Thr Ser Glu Asp Thr Ala Thr Tyr Tyr Cys
                85                  90                  95

Ala Arg Gly Trp Leu Leu His Tyr Thr Met Asp Tyr Trp Gly Gln Gly
            100                 105                 110

Thr Thr Val Thr Val Ser Ser
        115

<210> 44
<211> 16
<212> PRT
<213> Artificial Sequence

<220>
<223> 6G12M31 HCDR2

<400> 44

Tyr Arg Ser Tyr Asp Gly Ser Asn Asn Tyr Asp Pro Ser Leu Lys Asn
1               5                   10                  15

<210> 45
<211> 16
<212> PRT
<213> Artificial Sequence

<220>
<223> 6G12M41 HCDR2

<400> 45

Tyr Arg Ser Tyr Asp Gly Ser Asn Asn Tyr Gly Pro Ser Leu Lys Asn

```
1               5                   10                  15


<210>  46
<211>  119
<212>  PRT
<213>  Artificial Sequence

<220>
<223>  6G12M31 VH; 6G12M41 VH

<220>
<221>  misc_feature
<222>  (61)..(61)
<223>  X is D or G

<400>  46

Gln Val Gln Leu Val Gln Ser Gly Pro Gly Leu Val Lys Pro Ser Gln
1               5                   10                  15


Ser Leu Ser Leu Thr Cys Ser Val Thr Gly Phe Ser Ile Ser Ser Gly
            20                  25                  30


Tyr Tyr Trp Asn Trp Ile Arg Gln Phe Pro Gly Gln Lys Leu Glu Trp
        35                  40                  45


Met Gly Tyr Arg Ser Tyr Asp Gly Ser Asn Asn Tyr Xaa Pro Ser Leu
    50                  55                  60


Lys Asn Arg Ile Ser Ile Thr Arg Asp Thr Ser Lys Asn Gln Phe Phe
65                  70                  75                  80


Leu Lys Leu Asn Ser Val Thr Ser Glu Asp Thr Ala Thr Tyr Tyr Cys
                85                  90                  95


Ala Arg Gly Trp Leu Leu His Tyr Thr Met Asp Tyr Trp Gly Gln Gly
            100                 105                 110


Thr Thr Val Thr Val Ser Ser
        115
```

## Description

## TECHNICAL FIELD

The present application relates to the field of biomedicine, and in particular, relates to an isolated antigen AXL binding protein and use thereof.

## BACKGROUND ART

Receptor tyrosine kinase (AXL), as a member of the Tyro-3 family of kinases, can be activated by the binding of a ligand Gas6 (a 70-kDa protein homologous to an anticoagulant factor protein S). The activation of AXL results in signal transduction by means of PI-3-kinase/Akt (Franke et al., Oncogene, 22: 8983-8998, 2003) and other major pathways such as Ras/Erk and β-catenin/TCF (Goruppi et al., Mol. Cell Biol., 21: 902-915, 2001).

In tumor cells, AXL plays an important role in regulating cell invasion and migration. Overexpression of AXL is not only associated with poor prognosis, but also with increased invasion of various human cancers reported in the breast cancer, colon cancer, esophagus cancer, hepatocellular cancer, gastric cancer, glioma, lung cancer, melanoma, osteosarcoma, ovarian cancer, prostate cancer, rhabdomyosarcoma, kidney cancer, thyroid cancer, and endometrial cancer (Linger R.M., Adv.Cancer Res., 2008, 100, 35-83 and Verma A., Mol.Cancer Ther., (2011). 10,1763-1773).

In view of the therapeutic potential of AXL, it is necessary to prepare an antibody that specifically binds to an AXL protein.

## SUMMARY OF THE INVENTION

In one aspect, the present application provides an isolated antigen-binding protein, containing at least one CDR in a VH as set forth in an amino acid sequence of SEQ ID NO: 1 or SEQ ID NO: 46; and comprising at least one CDR in a VH as set forth in an amino acid sequence of SEQ ID NO: 2.

In certain embodiments, said isolated antigen-binding protein defined in the present application has one or more of the following properties:

1) capability of binding to an AXL protein at a $K_D$ of $1 \times 10^{-7}$M or lower;
2) capability of specifically recognizing an AXL protein expressed on a cell surface; and
3) capability of mediating internalization after binding to the AXL protein expressed on the cell surface.

In certain embodiments, said AXL protein includes a human AXL protein.

In certain embodiments, said human AXL protein contains an amino acid sequence as set forth in SEQ ID NO: 39.

In certain embodiments, said AXL protein contains an extracellular domain.

In certain embodiments, said extracellular domain contains an amino acid sequence as set forth in SEQ ID NO: 40.

In certain embodiments, said cell includes a tumor cell.

In certain embodiments, said tumor includes an AXL positive tumor.

In certain embodiments, said tumor is selected from the group consisting of a lung cancer, a skin cancer, a kidney cancer, a pancreatic cancer, a hematologic tumor, a breast cancer, an ovarian cancer, a lymphoma, and a myeloma.

In certain embodiments, said tumor is selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

In certain embodiments, said cell includes a human cell.

In certain embodiments, said cell is selected from the group consisting of a human non-small cell lung cancer A549 cell, a human cutaneous squamous cell carcinoma A431 cell, a renal clear cell adenocarcinoma 786-O cell, a human pancreatic cancer MIA PaCa-2 cell, an erythroleukemia K562 cell, an acute T cell leukemia Jurkat cell, a human breast cancer MCF-7 cell, a human breast cancer MDA-MB-231 cell, a human breast cancer MDA-MB-468 cell, a human breast cancer SKBR3 cell, a human ovarian cancer SKOV3 cell, a lymphoma U-937 cell, a lymphoma Raji cell, a human myeloma U266, and a human multiple myeloma RPMI8226 cell.

In certain embodiments, said VH contains HCDR1, HCDR2, and HCDR3.

In certain embodiments, said HCDR1 contains an amino acid sequence as set forth in SEQ ID NO: 25.

In certain embodiments, said HCDR2 contains an amino acid sequence as set forth in any one of SEQ ID NOs: 26, 44, and 45.

In certain embodiments, said HCDR3 contains an amino acid sequence as set forth in SEQ ID NO: 27.

In certain embodiments, said VL contains LCDR1, LCDR2, and LCDR3.

In certain embodiments, said LCDR1 contains an amino acid sequence as set forth in SEQ ID NO: 28.

In certain embodiments, said LCDR2 contains an amino acid sequence as set forth in SEQ ID NO: 29.

In certain embodiments, said LCDR3 contains an amino acid sequence as set forth in SEQ ID NO: 30.

In certain embodiments, said VH contains framework regions H-FR1, H-FR2, H-FR3, and H-FR4.

In certain embodiments, a C-terminus of said H-FR1 is directly or indirectly linked to an N-terminus of said HCDR1, and said H-FR1 contains an amino acid sequence as set forth in SEQ ID NO: 7.

In certain embodiments, said H-FR1 contains an amino acid sequence as set forth in any one of SEQ ID NOs: 11 and 15.

In certain embodiments, said H-FR2 is located between said HCDR1 and said HCDR2, and said H-FR2 contains an amino acid sequence as set forth in SEQ ID NO: 8.

In certain embodiments, said H-FR2 contains an amino acid sequence as set forth in SEQ ID NO: 12.

In certain embodiments, said H-FR3 is located between said HCDR2 and said HCDR3, and said H-FR3 includes an amino acid sequence as set forth in SEQ ID NO: 9.

In certain embodiments, said H-FR3 contains an amino acid sequence as set forth in SEQ ID NO: 13.

In certain embodiments, an N-terminus of said H-FR4 is linked to a C-terminus of said HCDR3, and said H-FR4 contains an amino acid sequence as set forth in SEQ ID NO: 10.

In certain embodiments, said H-FR4 contains an amino acid sequence as set forth in SEQ ID NO: 14.

In certain embodiments, said VH contains an amino acid sequence as set forth in any one of SEQ ID NOs: 3, 5, 42, and 43.

In certain embodiments, said VL contains framework regions L-FR1, L-FR2, L-FR3, and L-FR4.

In certain embodiments, a C-terminus of said L-FR1 is directly or indirectly linked to an N-terminus of said LCDR1, and said L-FR1 contains an amino acid sequence as set forth in SEQ ID NO: 16.

In certain embodiments, said L-FR1 contains an amino acid sequence as set forth in any one of SEQ ID NOs: 20 and 24.

In certain embodiments, said L-FR2 is located between said LCDR1 and said LCDR2, and said L-FR2 contains an amino acid sequence as set forth in SEQ ID NO: 17.

In certain embodiments, said L-FR2 contains an amino acid sequence as set forth in SEQ ID NO: 21.

In certain embodiments, said L-FR3 is located between said LCDR2 and said LCDR3, and said L-FR3 contains an amino acid sequence as set forth in SEQ ID NO: 18.

In certain embodiments, said L-FR3 contains an amino acid sequence as set forth in SEQ ID NO: 22.

In certain embodiments, an N-terminus of said L-FR4 is linked to a C-terminus of said LCDR3, and said L-FR4 contains an amino acid sequence as set forth in SEQ ID NO: 19.

In certain embodiments, said L-FR4 contains an amino acid sequence as set forth in SEQ ID NO: 23.

In certain embodiments, said VL contains an amino acid sequence as set forth in any one of SEQ ID NOs: 4 and 6.

In certain embodiments, said isolated antigen-binding protein defined in the present application contains an antibody heavy-chain constant region, which is derived from a human IgG heavy-chain constant region.

In certain embodiments, said antibody heavy-chain constant region is derived from a human IgG1 heavy-chain constant region or a human IgG4 heavy-chain constant region.

In certain embodiments, said antibody heavy-chain constant region contains an amino acid sequence as set forth in any one of SEQ ID NOs: 33 and 41.

In certain embodiments, said isolated antigen-binding protein defined in the present application contains an antibody light-chain constant region, which contains a human Igκ constant region.

In certain embodiments, said antibody light-chain constant region contains an amino acid sequence as set forth in SEQ ID NO: 34.

In certain embodiments, said isolated antigen-binding protein defined in the present application contains an antibody heavy chain, which contains an amino acid sequence as set forth in any one of SEQ ID NOs: 35 and 37.

In certain embodiments, said isolated antigen-binding protein defined in the present application contains an antibody light chain, which contains an amino acid sequence as set forth in any one of SEQ ID NOs: 36 and 38.

In certain embodiments, said isolated antigen-binding protein defined in the present application includes an antibody or an antigen-binding fragment thereof.

In certain embodiments, said antibody is selected from the group consisting of a monoclonal antibody, a single chain antibody, a chimeric antibody, a multispecific antibody, a humanized antibody, and a fully human antibody.

In certain embodiments, said antigen-binding fragment is selected from the group consisting of: Fab, Fab', $F(ab)_2$, Fv, $F(ab')_2$, scFv, di-scFv, and dAb fragments.

In another aspect, the present application provides an immunoconjugate, containing said isolated antigen-binding protein defined in the present application.

In certain embodiments, said immunoconjugate further containing at least one additional agent selected from

the group consisting of a chemotherapeutic agent, a radioactive element, a cytostatic agent, and a cytotoxic agent.

In certain embodiments, said isolated antigen-binding protein is linked to said additional agent by a linker molecule.

In certain embodiments, said isolated antigen-binding protein and said additional agent are covalently linked to said linker molecule, respectively.

In certain embodiments, said additional agent includes maytansine or a derivative thereof.

In certain embodiments, said maytansine derivative includes a maytansine derivative DM1.

In another aspect, the present application provides one or more isolated nucleic acid molecules, encoding said isolated antigen-binding protein defined in the present application.

In another aspect, the present application provides a vector containing said nucleic acid molecule defined in the present application.

In another aspect, the present application provides a cell containing said nucleic acid molecule defined in the present application or said vector defined in the present application.

In another aspect, the present application provides a pharmaceutical composition, containing said isolated antigen-binding protein, said immunoconjugate, said nucleic acid molecule, said vector, and/or said cell defined in the present application, and optionally a pharmaceutically acceptable adjuvant.

In another aspect, the present application provides a preparation method for said isolated antigen-binding protein defined in the present application, wherein the method includes culturing said cell under a condition of allowing the expression of said isolated antigen-binding protein.

In another aspect, the present application provides use of said isolated antigen-binding protein, said immunoconjugate, said nucleic acid molecule, said vector, said cell, and/or said pharmaceutical composition in the preparation of a drug for preventing, relieving and/or treating a tumor.

In certain embodiments, said tumor includes an AXL positive tumor.

In certain embodiments, said tumor is selected from the group consisting of a lung cancer, a skin cancer, a kidney cancer, a pancreatic cancer, a hematologic tumor, a breast cancer, an ovarian cancer, a lymphoma, and a myeloma.

In certain embodiments, said tumor is selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

In another aspect, the present application provides use of said isolated antigen-binding protein in the preparation of a diagnostic agent for diagnosing a disease or condition associated with the expression of said AXL protein.

In another aspect, the present application provides a method for diagnosing a disease or condition associated with the expression of an AXL protein in a subject, including: bringing a sample derived from the subject and said isolated antigen-binding protein into contact, and determining the presence and/or amount of a substance capable of specifically binding the isolated antigen-binding protein, in said sample.

In another aspect, the present application provides a method for detecting AXL in a sample, including administering said isolated antigen-binding protein.

Other aspects and advantages of the present application can be readily perceived by those skilled in the art from the detailed description below. The detailed description below only shows and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

## BRIEF DESCRIPTION OF THE DRAWINGS

The specific features of the present invention involved in the present application are listed in the appended claims. The characteristics and advantages of the present invention involved in the present application can be better understood by referring to the exemplary embodiments and the accompanying drawings described in detail below. A brief description of the drawings is as follows:

FIG. 1 shows the antigen-binding capability of isolated antigen-binding proteins according to the present application;
FIG. 2 shows the binding, to different target antigens, of an antigen-binding protein 6G12M11 according to the present application;
FIG. 3 shows the binding, to different target antigens, of an antigen-binding protein 6G12M21 according to the present application;
FIG. 4 shows the binding, to different target antigens, of an antigen-binding protein 6G12M31 according to the present application;

FIG. 5 shows the binding, to different target antigens, of an antigen-binding protein 6G12M41 according to the present application;

FIG. 6 shows the binding, to A549 cell surface antigens, of the isolated antigen-binding proteins 6G12M11 and 6G12M21 according to the present application;

FIG. 7 shows the binding, to MDA-MB-231 cell surface antigens, of the isolated antigen-binding proteins 6G12M11 and 6G12M21 according to the present application;

FIG. 8 shows the binding, to 786-O cell surface antigens, of the isolated antigen-binding proteins 6G12M11 and 6G12M21 according to the present application;

FIG. 9 shows the binding, to MDA-MB-231 cell surface antigens, of the isolated antigen-binding proteins 6G12M21, 6G12M31, and 6G12M41 according to the present application;

FIG. 10 shows the binding, to 786-O cell surface antigens, of the isolated antigen-binding proteins 6G12M21, 6G12M31, and 6G12M41 according to the present application;

FIG. 11 shows the internalization efficiency, on A549 cells, of the isolated antigen-binding proteins 6G12M11 and 6G12M21 according to the present application;

FIG. 12 shows the internalization efficiency, on MDA-MB-231 cells, of the isolated antigen-binding proteins 6G12M11 and 6G12M21 according to the present application;

FIG. 13 shows the internalization efficiency, on 786-O cells, of the isolated antigen-binding proteins 6G12M11 and 6G12M21 according to the present application;

FIG. 14 shows the internalization efficiency, on MDA-MB-231 cells, of the isolated antigen-binding proteins 6G12M21, 6G12M31, and 6G12M41 according to the present application;

FIG. 15 shows the internalization efficiency, on 786-O cells, of the isolated antigen-binding proteins 6G12M21, 6G12M31, and 6G12M41 according to the present application;

FIG. 16 shows the inhibition curves, against MDA-MB-231, of immunoconjugates according to the present application;

FIG. 17 shows a tendency chart of tumor (human breast cancer MDA-MB-231) volume changes after administration of immunoconjugates according to the present application; and

FIG. 18 shows a tendency chart of changes in the body weight of mice after administration of the immunoconjugates according to the present application.

## DETAILED DESCRIPTION OF THE INVENTION

The embodiments of the invention of the present application will be illustrated by specific examples below. Those familiar with this technology can easily understand other advantages and effects of the invention of the present application from the content disclosed in the specification.

The present application is further described as follows: in the present invention, unless otherwise specified, the scientific and technical terms used herein have the meanings commonly understood by those skilled in the art. Moreover, as used herein, terms and laboratory operating steps in relation to the protein and nucleic acid chemistry, molecular biology, cell and tissue culture, microbiology, and immunology are all terms and routine steps widely used in the corresponding fields. At the same time, for a better understanding of the present invention, definitions and explanations of related terms are provided below.

In the present application, the term "isolated" generally refers to a state obtained from a natural state by artificial means. If a certain "isolated" substance or component is present in nature, it is possible because its natural environment changes, or the substance is isolated from the natural environment, or both. For example, a certain un-isolated polynucleotide or polypeptide naturally exists in a certain living animal body, and the same polynucleotide or polypeptide with a high purity isolated from such a natural state is called isolated polynucleotide or polypeptide. The term "isolated" excludes neither the mixed artificial or synthesized substance nor other impure substances that do not affect the activity of the isolated substance.

In the present application, the term "isolated antigen-binding protein" generally refers to an antigen-bindable protein obtained from a natural state by artificial means. The "isolated antigen-binding protein" may contain an antigen-binding portion and optionally, a scaffold or construct fraction allowing the antigen-binding fraction to adopt a conformation that facilitates the binding of the antigen-binding fraction to an antigen. The antigen-binding protein may contain, for example, an antibody-derived protein scaffold or alternative protein scaffolds or artificial scaffolds with grafted CDRs or CDR derivatives. Such scaffolds include, but are not limited to, an antibody-derived scaffold containing a mutation introduced, for example, to stabilize the three-dimensional structure of the antigen-binding protein, and a fully synthetic scaffold containing, for example, a biocompatible polymer. See, for example, Korndorfer et al., 2003, Proteins: Structure, Function, and Bioinformatics, 53(1): 121-129 (2003); and Roque et al., Biotechnol. Prog. 20: 639-654 (2004). In addition, peptide antibody mimics ("PAMs") and antibody mimics based scaffolds using fibronectin components may be used as scaffolds.

In the present application, the term "$K_D$" (similarly, "$K_D$" or "KD") generally refers to an "affinity constant" or

"equilibrium dissociation constant", and refers to a value obtained in titration measurement at equilibrium or by dividing a dissociation rate constant ($k_d$) by an association rate constant ($k_a$). The association rate constant ($k_a$), the dissociation rate constant ($k_d$), and the equilibrium dissociation constant ($K_D$) are used to represent the binding affinity of a binding protein (for example, the isolated antigen-binding protein defined in the present application) to an antigen (for example, the AXL protein). Methods for determining the association and dissociation rate constants are well known in the art. The use of fluorescence-based technology provides high sensitivity and the ability to check a sample at equilibrium in a physiological buffer. For example, the $K_D$ value may be measured by means of Octet, or by other laboratory approaches and instruments such as BIAcore (Biomolecular Interaction Analysis) assays (for example, instruments available from BIAcore International AB, a GE Healthcare company, Uppsala, Sweden). In addition, the $K_D$ value may also be measured by KinExA (Kinetic Exclusion Assay) available from Sapidyne Instruments (Boise, Idaho), or the $K_D$ value may be measured by a surface plasmon resonance (SPR) instrument.

In the present application, the term "EC50" or "$EC_{50}$", also known as the half-maximal effective concentration, generally refers to the concentration of an antibody which induces 50% of the maximum effect.

In the present application, the term "AXL protein" generally refers to a protein receptor tyrosine kinase encoded by an *axl* gene. AXL (Ark, UFO, and Tyro-7), as a member of the Tyro-3 family of kinases, can be activated by the binding of a ligand Gas6 (a 70-kDa protein homologous to an anticoagulant factor protein S). In some cancer (for example, the lung cancer, kidney cancer, or breast cancer) cells, there may be overexpression of the AXL protein. Human AXL protein, a protein of 894 amino acids, has an amino acid sequence that may be as set forth in SEQ ID NO: 39, wherein the amino acid residues 1-26 are signal peptides; and the amino acid residues 27-451 are the extracellular domains of AXL protein (with an amino acid sequence as set forth in SEQ ID NO: 40).

In the present application, the term "extracellular domain" generally refers to a polypeptide or protein domain located outside a cell. For example, the extracellular domain may be an extracellular domain of the AXL protein, and has an amino acid sequence as set forth in SEQ ID NO: 40. The extracellular domain of the AXL protein may have a structure close to that required by a cell adhesion molecule. The extracellular domain of the AXL protein may be a combination of two immunoglobulin-like domains, and is capable of binding to a Gas6 ligand (Sasaki T. et al., EMBO J. (2006). 25, 80-87).

In the present application, the term "specific binding" or "specific" generally refers to a measurable and reproducible interaction, such as the binding between a target and an antibody, and the existence of the target may be determined in a heterogeneous population of molecules (including biomolecules). For example, an antibody that specifically binds to a target (which may be an epitope) is an antibody that binds to the target with greater affinity, avidity, easiness, and/or duration than it binds to other targets. In one embodiment, the extent to which an antibody binds to an unrelated target is about 10% less than the extent to which the antibody binds to a target, as measured, for example, by radioimmunoassay (RIA). For example, in the present application, the isolated antigen-binding protein may bind to the AXL protein at a dissociation constant (KD) of $<1x10^{-7}$M or lower. In certain embodiments, the antibody specifically binds to an epitope on a protein, the epitope being conserved among proteins of different species. In another embodiment, the specific binding may include but does not require exclusive binding.

In the present application, the term "internalization" generally refers to a process that an antibody or an antigen-binding fragment thereof or a polypeptide specifically binds a receptor on the surface of a cell to form a receptor-antibody complex, and then enters the cell by virtue of endocytosis mediated by the receptor. Here, such antibody or the antigen-binding fragment thereof (such as a Fab fragment) may become an internalized antibody. The internalized antibody may act as a vector for targeted delivery of drugs, enzymes or DNA. In some cases, the internalization may inhibit the proliferation of tumor cells. For example, the internalized antibody may be used to couple an antitumor chemotherapeutic agent, radioactive element, cytostatic agent, and cytotoxic agent, and act as a candidate molecule for tumor biotherapy.

In the present application, the term "tumor" generally refers to a neoplasm or solid lesion resulting from abnormal cell growth. In the present application, the tumor may be a solid tumor or hematologic tumor. For example, in the present application, the tumor may be an AXL positive tumor, wherein the AXL positive tumor may be selected from the group consisting of a lung cancer, a skin cancer, a kidney cancer, a pancreatic cancer, a hematologic tumor, a breast cancer, an ovarian cancer, a lymphoma, and a myeloma. In certain embodiments, the AXL positive tumor may be selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

In the present application, the term "variable domain" generally refers to an amino terminal domain of an antibody heavy or light chain. The variable domains of the heavy and light chains may be referred to as "VH" and "VL" respectively (or "$V_H$" and "$V_L$" respectively). These domains are generally the most varied fractions of an antibody (with respect to other antibodies of the same type) and contain antigen-binding sites.

In the present application, the term "variable" generally refers to the fact that certain segments of the variable domains differ greatly in sequence between antibodies. The V domain mediates antigen binding and determines the specificity of a specific antibody to its specific antigen. However, the variability is not evenly distributed across the entire

range of variable domain. On the contrary, it is concentrated in three segments, referred to as hypervariable regions (CDRs or HVRs), in the variable domains of the light and heavy chains. The more highly conserved fraction of the variable domain is referred to as framework region (FR). The variable domains of the natural heavy and light chains each contain four FRs, most of which have a β-pleated configuration and which are linked by three CDRs to form a circular linkage, and to form a fraction of a β-pleated structure in some cases. The CDRs in each chain are held together in close proximity by the FRs, and, together with CDRs from the other chain, promote the formation of the antigen-binding site of the antibody (see Kabat et al., Sequences of Immunological Interest, Fifth Edition, National Institute of Health, Bethesda, Md. (1991)). The constant domain is not directly involved in the binding of an antibody to an antigen, but exhibits various effector functions, for example, allowing the antibody to participate in antibody-dependent cytotoxicity.

In the present application, the term "antibody" generally refers to an immunoglobulin or a fragment or derivative thereof, and encompasses any polypeptide, regardless of in vitro or in vivo production, that includes an antigen-binding site. The term includes, but is not limited to, polyclonal, monoclonal, monospecific, multispecific, non-specific, humanized, single-chain, chimeric, synthetic, recombinant, hybrid, mutant and grafted antibodies. Unless otherwise modified by the term "intact", as in "intact antibody", for the purposes of the present invention, the term "antibody" also includes antibody fragments, for example Fab, $F(ab')_2$, Fv, scFv, Fd, dAb, and other antibody fragments that maintain the antigen-binding function (for example, specific binding to AXL).Generally, such fragments should include an antigen-binding domain. A basic 4-chain antibody unit is a heterotetrameric glycoprotein composed of two identical light (L) chains and two identical heavy (H) chains. An IgM antibody is composed of 5 basic heterotetrameric units and another polypeptide called J chain, and contains 10 antigen-binding sites; and an IgA antibody includes 2-5 basic 4-chain units that may be polymerized with the J chain to form a multivalent combination. In the case of IgG, the 4-chain unit is generally of about 150,000 Daltons. Each L chain is linked to the H chain by a covalent disulfide bond, and two H chains are linked to each other by one or more disulfide bonds depending on the isotype of the H chains. Each of the H and L chains also has a regularly spaced intra-chain disulfide bridged bond. Each H chain has a variable domain (VH) at an N-terminus, followed by three constant domains (CH) for each of α and γ chains and four CH domains for the μ and ε isoforms. Each L chain has a variable domain (VL) at its N-terminus and a constant domain at the other end thereof. VL corresponds to VH, and CL corresponds to a first constant domain (CH1) of the heavy chain. Specific amino acid residues are considered to form an interface between the variable domains of the light and heavy chains. VH and VL are paired together to form a single antigen-binding site. For the structures and properties of different types of antibodies, see, for example, Basic and Clinical Immunology, 8th Edition, Daniel P. Sties, Abba I. Terr and Tristram G. Parsolw (eds), Appleton & Lange, Norwalk, Conn., 1994, p. 71 and Chapter 6. An L chain from any vertebrate species may be classified, based on the amino acid sequence of its constant domain, into one of two distinct types, called κ and λ. Immunoglobulins may be divided into different classes or isotypes depending on the amino acid sequences of constant domains of their heavy chains (CHs). There are five classes of immunoglobulins, namely, IgA, IgD, IgE, IgG, and IgM, which have heavy chains named α, δ, ε, γ, and μ, respectively. The classes γ and α are further divided into subclasses based on the relatively small differences in CH sequence and function. For example, the following subclasses are expressed in a human: IgG1, IgG2A, IgG2B, IgG3, IgG4, IgA1, and IgK1.

In the present application, the term "CDR" generally refers to a region of an antibody variable domain, with a sequence being highly variable and/or forming a structure defined loop. Generally, an antibody includes six CDRs, three (HCDR1, HCDR2, and HCDR3) in VH, and three (LCDR1, LCDR2, and LCDR3) in VL. In a natural antibody, HCDR3 and LCDR3 show most of the diversity of the six CDRs, and in particular, HCDR3 is considered to play a unique role in endowing the antibody with fine specificity. See, for example, Xu et al., Immunity, 13: 37-45 (2000); and Johnson and Wu, in Methods in Molecular Biology, 248: 1-25 (Lo, ed., Human Press, Totowa, N.J., 2003). In fact, a naturally occurring camelid antibody composed of only heavy chains function normally and stably in the absence of light chains. See, for example, Hamers-Casterman et al., Nature, 363: 446-448 (1993); and Sheriff et al., Nature Struct. Biol., 3: 733-736 (1996).

In the present application, the term "FR" generally refers to a more highly conserved fraction in the variable domain of an antibody, which is referred to as a framework region. Generally, the variable domain of each of the natural heavy and light chains contains four FRs, namely four (H-FR1, H-FR2, H-FR3, and H-FR4) in VH, and four (L-FR1, L-FR2, L-FR3, and L-FR4) in VL. For example, the VL of the isolated antigen-binding protein defined in the present application may include framework regions L-FR1, L-FR2, L-FR3, and L-FR4. The VH of the isolated antigen-binding protein defined in the present application may include framework regions H-FR1, H-FR2, H-FR3, and H-FR4.

In the present application, the term "antigen-binding fragment" generally refers to one or more fragments that have the ability to specifically bind an antigen (for example, the AXL protein). In the present application, the antigen-binding fragment may include Fab, Fab', $F(ab)_2$, a Fv fragment, a $F(ab')_2$, scFv, di-scFv, and/or dAb.

In the present application, the term "monoclonal antibody" or "McAb" or "monoclonal antibody composition" generally refers to an antibody molecule product of a single molecular composition. The monoclonal antibody composition exhibits single binding specificity and affinity to a specific epitope.

In the present application, the term "single chain antibody" generally refers to a molecule containing heavy-chain and light-chain variable regions of an antibody. For example, the single chain antibody may be created by linking

the heavy-chain variable regions of an antibody to the light-chain variable regions of the antibody by a linker molecule (for example, a linker peptide).

In the present application, the term "human antibody" generally refers to an antibody having variable-region framework and CDR regions derived from a human germline immunoglobulin sequence. In addition, if the antibody contains a constant region, it is also derived from the human germline immunoglobulin sequence. The human antibody of the present application may contain an amino acid residue that is not encoded by the human germline immunoglobulin sequence, but by, for example, a mutation introduced by a random or point mutation in vitro or a somatic mutation in vivo. However, the term "human antibody" does not include antibodies in which CDR sequences derived from other mammalian species are inserted into human framework sequences.

In the present application, the term "murine antibody" generally refers to an antibody having variable-region framework and CDR regions derived from a mouse germline immunoglobulin sequence. In addition, if the antibody contains a constant region, it is also derived from the mouse germline immunoglobulin sequence. The murine antibody of the present application may contain an amino acid residue that is not encoded by the mouse germline immunoglobulin sequence, but by, for example, a mutation introduced by a random or point mutation in vitro or a somatic mutation in vivo. However, the term "murine antibody" does not include antibodies in which CDR sequences derived from other mammalian species are inserted into mouse framework sequences.

In the present application, the term "chimeric antibody" generally refers to an antibody obtained by combining a non-human genetic material with a human genetic material. Or, more generally speaking, a chimeric antibody refers to an antibody that combines the genetic material of one species with the genetic material of another species.

In the present application, the term "multispecific antibody" generally refers to an antibody molecule capable of recognizing two or more antigens or epitopes at the same time. The multispecific antibody may be derived in a eukaryotic expression system or a prokaryotic expression system by methods such as a chemical coupling method, a hybrid-hybridoma method, and a genetic engineering antibody preparation method.

In the present application, the term "humanized antibody" generally refers to an antibody derived from a non-human species, but with a protein sequence that has been modified to increase its similarity to a human naturally-produced antibody.

In the present application, the term "fully human antibody" generally refers to a fully human antibody with both constant regions and variable regions derived from a human. The fully human antibody may be implemented by technologies such as the phage antibody library technology, preparation of human antibodies from transgenic mice, ribosome display technology, EBV transformed B cell cloning technology, and single B cell cloning.

In the present application, the terms "antibody recognizing an antigen" and "antibody specific to an antigen" are used interchangeably with the term "antibody specifically binding to an antigen" herein.

In the present application, the term "directly linked" is opposite to the term "indirectly linked", and the term "directly linked" generally refers to a direct linkage. For example, the direct linkage may be the situation where substances are directly linked without a spacer. The spacer may be a linker. For example, the linker may be a peptide linker. The term "indirectly linked" generally refers to the situation where substances are not directly linked. For example, the indirect linkage may be the situation where a linkage is performed via a spacer. For example, in the isolated antigen-binding protein defined in the present application, a C-terminus of the L-FR1 may be directly or indirectly linked to an N-terminus of the LCDR1.

In the present application, the term "immunoconjugate" generally refers to a conjugate produced by conjugating (for example, by covalent linkage via a linker molecule) the defined additional agent (for example, a chemotherapeutic agent, a radioactive element, a cytostatic agent, and a cytotoxic agent) to the defined isolated antigen-binding protein. This conjugate may deliver the defined additional agent to a target cell (for example, a tumor cell) by means of the specific binding of the isolated antigen-binding protein to an antigen on the target cell. Then, the immunoconjugate is internalized and finally enters the interior of the target cell (for example, into a lysosome and other vesicles). Here, the linker molecule in the immunoconjugate may be split to release the defined additional agent for exerting its cytotoxic effect. In addition, said antigen may also be secreted by the target cell and located in a space outside the target cell.

In the present application, the term "chemotherapeutic agent" generally refers to an agent for chemotherapy to inhibit tumor and/or tumor cell proliferation. The chemotherapeutic agent may be selected from the group consisting of a mitotic inhibitor, a kinase inhibitor, an alkylating agent, an antimetabolite, an embedded antibiotic, a growth factor inhibitor, a cell cycle inhibitor, an enzyme, a topoisomerase inhibitor, a histone deacetylase inhibitor, an anti-survival agent, a biological response modifier, and an anti-hormonogenesis agent such as an anti-androgen product agent and an anti-angiogenesis agent. For example, the chemotherapeutic agent may be selected from the group consisting of: capecitabine, daunorubicin, daunorubicin, actinomycin D, doxorubicin, epirubicin, idarubicin, esorubicin, bleomycin, mafosfamide, ifosfamide, cytarabine, dichloroethylnitrosourea, busulfan, mitomycin C, actinomycin D, plicamycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosourea, chlormethine, melphalan, cyclophosphamide, 6-mercaptopurine, 6-thioguanine, cytarabine (CA), 5-azacytidine, hydroxyurea, deoxycoformycin, 4-hydroxyperoxycyclophosphamide, 5-fluorouracil (5-FU), 5-fluorodeoxyuridine (5-FUdR), methotrexate (MTX), colchicine, taxol, vincristine, vinblastine, etoposide, trimetrexate, teniposide, and/or diethylstilbestrol (DES).

In the present application, the term "radioactive element" generally refers to an element for radiotherapy to inhibit tumor and/or tumor cell proliferation. The radioactive element may be selected from the group consisting of: $^{3}H$, $^{14}C$, $^{15}N$, $^{35}S$, $^{90}Y$, $^{99}Tc$, $^{111}In$, $^{125}I$ and/or $^{131}I$.

In the present application, the term "cytostatic agent" generally refers to an agent for inhibiting a tumor by suppressing growth factors that promote the growth and replication of tumor cells. The growth factors bind to receptors on the cell surface to consequently activate intracellular signaling pathways, and the complex pathways can promote uncontrolled cell growth, leading to excessive cell division and the development of a tumor. The cytostatic agent can inhibit the effects of these growth factors. The cytostatic agent may be selected from the group consisting of: an angiogenesis inhibiting factor, a deacetylase (HDAC) inhibiting factor, a Hedgehog signaling pathway blocker, an mTOR inhibitor, a p53/mdm2 inhibitor, a PARP inhibitor, a proteasome inhibitor, and/or a tyrosine kinase inhibitor.

In the present application, the term "cytotoxic agent" generally refers to an agent for inhibiting tumor and/or tumor cell proliferation by producing toxins on affected cells. The cytotoxic agent may be selected from the group consisting of: alkylating agents, such as, busulfan, hexamethylmelamine, thiotepa, cyclophosphamide, chlorambucil, uramustine, melphalan, chlorambucil, carmustine, streptozotocin, dacarbazine, temozolomide, and ifosfamide; antitumor agents, such as, mitomycin C; antimetabolites, such as, methotrexate, azathioprine, mercaptopurine, fludarabine, and 5-fluorouracil; platinum-containing anticancer agents, such as, cisplatin, and carboplatin; anthracyclines, such as, daunorubicin, doxorubicin, epirubicin, idarubicin, and mitoxantrone; plant alkaloids and terpenoids, such as, vincristine, vinblastine, vinorelbine, vindesine, podophyllotoxin, and docetaxel; topoisomerase inhibitors, such as, irinotecan, amsacrine, topotecan, etoposide, and teniposide; antibodies, such as, rituximab, trastuzumab, bevacizumab, erlotinib, and dactinomycin; finasteride; aromatase inhibitors; tamoxifen; goserelin; paclitaxel and/or imatinib mesylate. The cytotoxic agent can be administered orally, or by injection and other methods.

In the present application, the term "linker molecule" generally refers to a functional molecule liking or joining two molecules. For example, the linker molecule can link one molecule with another molecule (for example, one molecule is a protein molecule, and the other molecule is also a protein molecule or may be a small molecule drug). The linker molecule can be used in the construction of the defined immunoconjugate. In the immunoconjugate, the linker molecule may have two functional characteristics: 1. stability in the circulatory system, whereby the immunoconjugate is not split to release the defined additional agent in the circulatory system before reaching a target cell, thereby avoiding toxic effects; and 2. Rapidness and effectiveness in breakage after the linking molecule enters the target cell, whereby the defined additional agent may be effectively released to exert its due pharmacological activity. The linker molecule may be composed of a polar or non-polar amino acid. The linker molecule may also be a carbon chain containing a heteroatom (such as a nitrogen atom and a sulfur atom). The linker molecule may be 2-100 atoms (for example, between 2 atoms and 50 atoms) long, or 3, 5, 10, 15, 20, 25, 30, 35, 40, 45, or 50 atoms long. For another example, the linker may be 20 to 26 (20, 21, 22, 23, 24, 25, or 26) atoms long. The linker molecule may include substituents selected from the group consisting of: a hydrogen atom, alkyl, alkenyl, alkynyl, amino, alkylamino, dialkylamino, trialkylamino, hydroxyl, alkoxy, halogen, aryl, heterocycle, aromatic heterocycle, cyano, amide, carbamoyl, carboxylic acid, ester, thioether, alkyl sulfide, mercapto, and ureido. In addition, the linker molecule may be selected from the group consisting of: a pH-sensitive linker molecule, a protease-cleavable linker molecule, a nuclease-sensitive linker molecule, a lipase-sensitive linker molecule, a glycosidase linker molecule, a hypoxia linker molecule, a photocleavage linker molecule, a thermally unstable linker molecule, and an ultrasound-sensitive linker molecule.

In the present application, the term "covalent" generally refers to a covalent bond, that is, two or more atoms share a pair of electrons and reach a state of electronic saturation to form a relatively stable chemical structure. The covalent bond is formed by pairing electrons having opposite spin directions between two adjacent atoms. Here, atomic orbits overlap each other, and the electron cloud density between two nuclei increases relatively, thereby increasing the attraction to the two nuclei. The covalent bond may have saturability and directionality. The covalent bonds may be divided into non-polar covalent bonds, polar covalent bonds, and coordination covalent bonds. Compounds containing only covalent bonds may be referred to as covalent compounds.

In the present application, the term "maytansine" generally refers to a compound isolated from a Maytenus molina plant (see U.S. Patent No. US3896111). It is a kind of anti-mitotic cytotoxin, with a structural formula as follows:

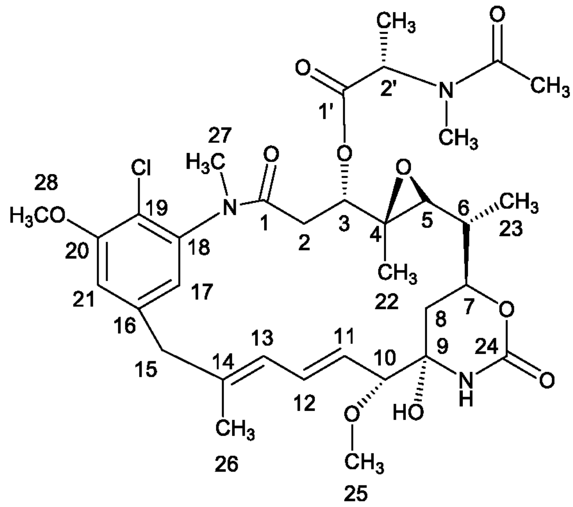

,

and the CAS Number of maytansine is 35846-53-8. Maytansine has a significant effect on various tumors, such as L-1210, P-388 leukemia, S-180, W-256, Lewis lung cancer and in vitro nasopharyngeal carcinoma. The maytansine derivative may include a compound, for example maytansine derivatives DM1 and DM4, having a maytansine ring structure with one or more substituent modifications on its ring.

In the present application, the term "maytansine derivative DM1" generally refers to a compound having the following structural formula:

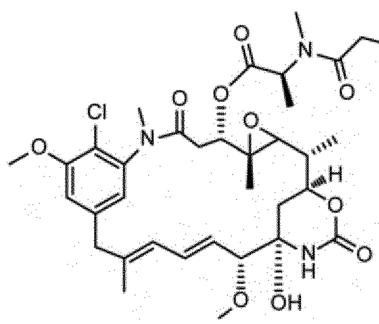

,

with the CAS Number of 139504-50-0. The maytansine derivative DM1 may be an anti-mitotic cytotoxin.

In the present application, the term "disease or condition associated with the expression of AXL protein" generally refers to a disease or condition which is associated with the expression of AXL protein, or which is induced by the up-regulation of the expression of AXL protein. For example, the disease or condition associated with the expression of the AXL protein may be a lung cancer, a skin cancer, a kidney cancer, a pancreatic cancer, a hematologic tumor, a breast cancer, an ovarian cancer, a lymphoma, and/or a myeloma.

In the present application, the term "isolated nucleic acid molecule" generally refers to a nucleotide, deoxyribonucleotide, or ribonucleotide in the isolated form of any length, or an analog, which is isolated from its natural environment or artificially synthesized.

In the present application, the term "vector" generally refers to a means for the delivery of nucleic acids, whereby a polynucleotide encoding a certain protein can be inserted therein to allow the protein to be expressed. The vector may be transformed, transduced, or transfected into a host cell, such that genetic material elements carried thereby can be expressed in the host cell. For instance, the vector includes: a plasmid; a phagemid; a cosmid; an artificial chromosome, such as a yeast artificial chromosome (YAC), a bacterial artificial chromosome (BAC), or a P1-derived artificial chromosome (PAC); and a bacteriophage, such as a λ phage or an M13 phage, an animal virus etc. The species of animal viruses used as vectors include retroviruses (including lentiviruses), adenoviruses, adeno-associated viruses, herpesviruses (for example, herpes simplex viruses), poxviruses, baculoviruses, papillomaviruses, and papovaviruses (for example, SV40). A vector may contain a variety of elements that control expression, including a promoter sequence, a transcription initiation sequence, an enhancer sequence, a selection element, and a reporter gene. In addition, the vector may also contain an origin of replication site. The vector may also include components, such as, but not limited only to, a virus particle, a liposome, or a protein coat, that assist the vector in entering the cell.

In the present application, the term "cell" generally refers to a single cell, cell line, or cell culture that may be or has been a recipient of a subj ect's plasmid or vector, including the nucleic acid molecule defined in the present invention or the vector defined in the present invention. The cell may include a progeny of a single cell. Due to natural, accidental, or deliberate mutations, the progeny may not necessarily be exactly the same as an original parent cell (in the form of total DNA complement or in the genome). The cell may include a cell transfected in vitro by using the vector defined in the present application. The cell may be a bacterial cells (for example, *E. coli*), a yeast cell, or other eukaryotic cells, such as a COS cell, a Chinese hamster ovary (CHO) cell, a CHO-K1 cell, an LNCAP cell, a HeLa cell, a HEK293 cell, a COS-1 cells, an NSO cell, a human non-small cell lung cancer A549 cell, a human cutaneous squamous cell carcinoma A431 cell, a renal clear cell adenocarcinoma 786-O cell, a human pancreatic cancer MIA PaCa-2 cell, an erythroleukemia K562 cell, an acute T cell leukemia Jurkat cell, a human breast cancer MCF-7 cell, a human breast cancer MDA-MB-231 cell, a human breast cancer MDA-MB-468 cell, a human breast cancer SKBR3 cell, a human ovarian cancer SKOV3 cell, a lymphoma U-937 cell, a lymphoma Raji cell, a human myeloma U266, or a human multiple myeloma RPMI8226 cell. In certain embodiments, the cell is a mammalian cell. In certain embodiments, the mammalian cell is ah HEK293 cell.

In the present application, the term "pharmaceutical composition" generally refers to a composition suitable for administration to a patient, preferably a human patient. For example, the pharmaceutical composition defined in the present application may contain the isolated antigen-binding protein defined in the present application, the immunoconjugate defined in the present application, the nucleic acid molecule defined in the present application, the vector defined in the present application, and/or the cell defined in the present application, and optionally a pharmaceutically acceptable adjuvant. In addition, the pharmaceutical composition may further include a suitable agent of one or more (pharmaceutically effective) carriers, stabilizers, excipients, diluents, solubilizers, surfactants, emulsifiers and/or preservatives. The acceptable ingredient of the composition is preferably nontoxic to a subject at a dose and concentration as used. The pharmaceutical composition of the present invention includes, but is not limited to, a liquid, frozen, and lyophilized composition.

In the present application, the term "pharmaceutically acceptable adjuvant" generally refers to any and all solvents, dispersion media, coatings, isotonic agents, and absorption retarders, etc. that are compatible with pharmaceutical administration. Such adjuvant is generally safe and non-toxic, and is neither biologically nor otherwise undesirable.

In the present application, the term "subject" generally refers to a human or non-human animal, including but not limited to a cat, a dog, a horse, a pig, a cow, a goat, a rabbit, a mouse, a rat, or a monkey.

In the present application, the term "comprising" generally refers to the inclusion of explicitly specified features, but not excluding other elements.

In the present application, the term "approximately" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

**Isolated Antigen-Binding Protein**

In one aspect, the present application provides an isolated antigen-binding protein, containing at least one CDR in a VH as set forth in an amino acid sequence of SEQ ID NO: 1 or SEQ ID NO: 46; and containing at least one CDR in a VH as set forth in an amino acid sequence of for example SEQ ID NO: 2.

QVQL $X_1$ QSGPGLVKPSQSLSLTC $X_2$ V $X_3$ G $X_4$ SISSGYYWNWIRQ $X_5$ PG $X_6$ $X_7$ LEWMGYRSYDGSNNYNPSLKNRISITRDTSKNQFFLKLNSVT $X_8$ EDTATYYCARGWLLHYTMDYWGQGT $X_9$ VTVSS (SEQ ID NO: 1), wherein $X_1$ may be K or V, $X_2$ may be S or A, $X_3$ may be T or S, $X_4$ may be F, Y, D or S, $X_5$ may be F or S, $X_6$ may be N or Q, $X_7$ may be K or G, $X_8$ may be T or S, and $X_9$ may be S or T.

QVQLVQSGPGLVKPSQSLSLTCSVTGFSISSGYYWNWIRQFPGQKLEWMGYRSYDGSN NYXiPSLKNRISITRDTSKNQFFLKLNSVTSEDTATYYCARGWLLHYTMDYWGQGTTVTVS S (SEQ ID NO: 46), wherein $X_1$ may be D or G.

$X_1$ $X_2$ VMTQSP $X_3$ S $X_4$ $X_5$ VTLG $X_6$ SASISCRSSRSLLHSNGFTYLYWY $X_7$ QKPGQSPQLLIYQMSNLASGVPDRFS $X_8$ SGSGTDFTL $X_9$ ISRVEAEDVGVYYCGQNLELPLTFG $X_{10}$ GTKLE $X_{11}$ K (SEQ ID NO: 2), wherein $X_1$ may be E or D, $X_2$ may be L or I, $X_3$ may be F or S, $X_4$ may be N or V, $X_5$ may be A or S, $X_6$ may be T or Q, $X_7$ may be L or Q, $X_8$ may be S or G, $X_9$ may be R or K, $X_{10}$ may be A may be G, and $X_{11}$ may be L or I.

The VH of the isolated antigen-binding protein defined in the present application may be obtained by amino acid mutation at one or more positions in the VH (SEQ ID NO: 31) of an antibody 6G12. Specifically, the isolated antigen-binding protein defined in the present application may be obtained either by amino acid mutation at one or more positions in a framework region (FR) of the VH of the antibody 6G12, or by amino acid mutation at one or more positions in a variable region (CDR) of the VH of the antibody 6G12. For example, in the VH of the antibody 6G12, from the N-terminus to the C-terminus, K at position 5 can be mutated to V; S at position 23 can be mutated to A; T at position 25 can be

mutated to S; F at position 27 can be mutated to Y, D or S; F at position 41 can be mutated to S; N at position 44 can be mutated to Q; K at position 45 can be mutated to G; T at position 88 can be mutated to S; and S at position 114 can be mutated to T.

For example, in the VH of the antibody 6G12, from the N-terminus to the C-terminus, K at position 5 can be mutated to V; S at position 23 can be mutated to A; T at position 25 can be mutated to S; F at position 27 can be mutated to Y, D or S; F at position 41 can be mutated to S; N at position 44 can be mutated to Q; K at position 45 can be mutated to G; N at position 61 can be mutated to D or G; T at position 88 can be mutated to S; and S at position 114 can be mutated to T.

The VL of the isolated antigen-binding protein defined in the present application may be obtained by amino acid mutation at one or more positions in the VL (SEQ ID NO: 32) of an antibody 6G12. Specifically, the isolated antigen-binding protein defined in the present application may be obtained by amino acid mutation at one or more positions in a framework region (FR) of the VL of the antibody 6G12.

For example, in the VL of the antibody 6G12, from the N-terminus to the C-terminus, E at position 1 can be mutated to D; L at position 2 can be mutated to I; F at position 9 can be mutated to S; N at position 11 can be mutated to V; A at position 12 can be mutated to S; T at position 17 can be mutated to Q; L at position 42 can be mutated to Q; S at position 69 can be mutated to G; R at position 79 can be mutated to K; A at position 105 can be mutated to G; and L at position 111 can be mutated to I.

Properties of Isolated Antigen-Binding Protein

In the present application, the isolated antigen-binding protein may have one or more of the following properties:

1) capability of binding to an AXL protein at a KD of $1 \times 10^{-7}$M or lower;
2) capability of specifically recognizing an AXL protein expressed on a cell surface; and
3) capability of mediating internalization after binding to the AXL protein expressed on the cell surface.

In the present application, the isolated antigen-binding protein may bind to the AXL protein at a $K_D$ of $1\times 10^{-7}$M or lower. For example, the $K_D$ value at which the isolated antigen-binding protein defined in the present application binds to a human-derived AXL protein may be $\leq 1\times10^{-7}$M, $\leq 9\times10^{-8}$M, $\leq 8\times10^{-8}$M, $\leq 7\times10^{-8}$M, $\leq 6\times10^{-8}$M, $\leq 5\times10^{-8}$M, $\leq 4\times10^{-8}$M, $\leq 3\times10^{-8}$M, $\leq 2\times10^{-8}$M, $\leq 1.5\times10^{-8}$M, $\leq 1.2\times10^{-8}$M, $\leq 1.15\times10^{-8}$M, $\leq 1.1\times10^{-8}$M, $\leq 1.05\times10^{-8}$M, $\leq 1\times10^{-8}$M, $<5\times10^{-9}$M or $\leq 1\times10^{-9}$M. For another example, the $K_D$ value at which the isolated antigen-binding protein defined in the present application binds to a human-derived AXL protein may be $\leq 1\times10^{-7}$M, $\leq 9\times10^{-8}$M, $\leq 8\times10^{-8}$M, $\leq 7\times10^{-8}$M, $\leq 6\times10^{-8}$M, $\leq 5\times10^{-8}$M, $\leq 4\times10^{-8}$M, $\leq 3\times10^{-8}$M, $\leq 2\times10^{-8}$M, $\leq 1.5\times10^{-8}$M, $\leq 1.2\times10^{-8}$M, $\leq 1.15\times10^{-8}$M, $\leq 1.1\times10^{-8}$M, $\leq 1.05\times10^{-8}$M, $\leq 1\times10^{-8}$M, $\leq 5\times10^{-9}$M or $\leq 1\times10^{-9}$M. For another example, the $K_D$ value at which the isolated antigen-binding protein defined in the present application binds to a human-derived AXL protein may be $\leq 1\times10^{-7}$M, $\leq 9\times10^{-8}$M, $\leq 8\times10^{-8}$M, $\leq 7\times10^{-8}$M, $\leq 6\times10^{-8}$M, $\leq 5\times10^{-8}$M, $\leq 4\times10^{-8}$M, $\leq 3\times10^{-8}$M, $\leq 2\times10^{-8}$M, $\leq 1.5\times10^{-8}$M, $\leq 1.5\times10^{-8}$M, $\leq 1.5\times10^{-8}$M, $\leq 1.5\times10^{-8}$M, $\leq 1.5\times10^{-8}$M, $\leq 1\times10^{-8}$M, $\leq 5\times10^{-9}$M or $\leq 1\times10^{-9}$M.

In the present application, the $K_D$ may also be determined by ELISA, competitive ELISA or BIACORE or KINEXA.

In the present application, the capability of competitive binding may be measured by determining the dissociation equilibrium constant of an antibody-antigen interaction of the isolated antigen binding protein. The method for detecting the dissociation equilibrium constant may be selected from the group consisting of an enzyme-linked immunosorbent assay method, a surface plasmon resonance (SRP) method, a potentiometric titration method, a spectrophotometric method, a capillary electrophoresis method, a fluorescence method, and a thin-layer chromatography pH method. For example, the isolated antigen-binding protein can be detected by the SRP method (for example, by a biomacromolecule interaction instrument).

In the present application, the isolated antigen-binding protein may specifically bind to the AXL protein expressed on a cell surface. The specific binding may be determined by FACS. For example, the specific binding of the isolated antigen-binding protein, defined in the present application, to the AXL protein on the cell surface may be embodied by EC50 in the FACS assay. For example, a lower EC50 indicates better specific binding. For example, the EC50 value at which the isolated antigen-binding protein binds to the AXL protein on the surface of a non-small-cell lung cancer A549 cell in the FACS assay may be 0.01μg/ml~0.10μg/ml, 0.01μg/ml~0.15μg/ml, 0.01μg/ml~0.20μg/ml, 0.01μg/ml~0.25μg/ml, 0.01μg/ml~0.30μg/ml, 0.01μg/ml~0.35μg/ml, 0.01μg/ml~0.40μg/ml, 0.01μg/ml~0.45μg/ml, 0.01μg/ml~0.50μg/ml, 0.01μg/ml~0.55μg/ml, 0.01μg/ml~0.60μg/ml, 0.01ug/ml~0.65μg/ml, 0.01ug/ml~0.70μg/ml, 0.01ug/ml~0.75μg/ml, or 0.01ug/ml~0.80μg/ml. For another example, the EC50 value at which the isolated antigen-binding protein binds to the AXL protein on the surface of a human breast cancer MDA-MB-231 cell in the FACS assay may be 0.01μg/ml~0.10μg/ml, 0.01μg/ml~0.15μg/ml, 0.01μg/ml~0.20μg/ml, 0.01μg/ml~0.25μg/ml, 0.01μg/ml~0.30μg/ml, 0.01μg/ml~0.35μg/ml, 0.01μg/ml~0.40μg/ml, 0.01μg/ml~0.45μg/ml, 0.01μg/ml~0.50μg/ml, 0.01μg/ml~0.55μg/ml, 0.01μg/ml~0.60μg/ml, 0.01μg/ml~0.65μg/ml, 0.01μg/ml~0.70μg/ml, 0.01μg/ml~0.75μg/ml, or

0.01ug/ml~0.80μg/ml. For another example, the EC50 value at which the isolated antigen-binding protein binds to the AXL protein on the surface of a renal clear cell adenocarcinoma 786-O cell in the FACS assay may be 0.01μg/ml~0.10μg/ml, 0.01μg/ml~0.15μg/ml, 0.01μg/ml~0.20μg/ml, 0.01μg/ml~0.25μg/ml, 0.01μg/ml~0.30μg/ml, 0.01μg/ml~0.35μg/ml, 0.01μg/ml~0.40μg/ml, 0.01μg/ml~0.45μg/ml, 0.01μg/ml~0.50μg/ml, 0.01μg/ml~0.55μg/ml, 0.01μg/ml~0.60μg/ml, 0.01μg/ml~0.65μg/ml, 0.01μg/ml-0.70μg/ml, 0.01μg/ml~0.75μg/ml, or 0.01ug/ml~0.80μg/ml.

In the present application, the isolated antigen-binding protein may mediate internalization after binding to the AXL protein expressed on the cell surface. For example, the internalization may include the following step: when the isolated antigen-binding protein can bind to a plasma membrane of a cell (for example, a tumor cell), or can be released in the cell in response to a proteolytic activity in a cellular microenvironment (for example, a tumor cell microenvironment), consequently, the isolated antigen-binding protein can be engulfed by the cell membrane and absorbed into the cell. In certain embodiments, the isolated antigen-binding protein in the immunoconjugate, and/or the additional agent conjugated thereto, may also be engulfed by the cell membrane and absorbed into the cell after the isolated antigen-binding protein binds to the plasma membrane of the cell.

In the present application, the AXL protein may be either a human AXL protein (NP_068713), or a cynomolgus monkey AXL protein (Genbank Accession Number HB387229.1). For example, the AXL protein may be a human AXL protein whose amino acid sequence as set forth in SEQ ID NO:39.

The AXL protein may include a variant of the AXL protein. For example, the variant may be: 1) a protein or polypeptide having one or more amino acids substituted, deleted or added to the amino acid sequence of the AXL protein; and 2) a protein or polypeptide having at least about 85% (for example, at least about 85%, about 90%, about 91%, about 92%, about 93%, about 94%, about 95%, about 96%, about 97%, about 98%, about 99% or higher) sequence homology with the AXL protein.

The AXL protein may further include a fragment of the AXL protein. For example, the AXL protein may be an extracellular domain of the human AXL protein, and has an amino acid sequence as set forth in SEQ ID NO: 40.

In the present application, the cell may include a tumor cell. For example, the tumor may be an AXL positive tumor. For example, the tumor may be selected from the group consisting of a lung cancer, a skin cancer, a kidney cancer, a pancreatic cancer, a hematologic tumor, a breast cancer, an ovarian cancer, a lymphoma, and a myeloma. For another example, the tumor may be selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

In the present application, the cell may include a human cell. For example, the cell may include a cell selected from the group consisting of a human non-small cell lung cancer A549 cell, a human cutaneous squamous cell carcinoma A431 cell, a renal clear cell adenocarcinoma 786-O cell, a human pancreatic cancer MIA PaCa-2 cell, an erythroleukemia K562 cell, an acute T cell leukemia Jurkat cell, a human breast cancer MCF-7 cell, a human breast cancer MDA-MB-231 cell, a human breast cancer MDA-MB-468 cell, a human breast cancer SKBR3 cell, a human ovarian cancer SKOV3 cell, a lymphoma U-937 cell, a lymphoma Raji cell, a human myeloma U266, and a human multiple myeloma RPMI8226 cell.

Types of Isolated Antigen-Binding Protein

In the present application, the isolated antigen-binding protein may include an antibody or an antigen-binding fragment thereof. For example, the isolated antigen-binding protein defined in the present application may include, but is not limited to, a recombinant antibody, a monoclonal antibody, a human antibody, a murine antibody, a humanized antibody, a chimeric antibody, a bispecific antibody, a single chain antibody, a double antibody, a triple antibody, a quadruple antibody, a Fv fragment, a scFv fragment, a Fab fragment, a Fab' fragment, a $F(ab')_2$ fragment, and a camelized single-domain antibody.

In the present application, the antibody may be a humanized antibody. In other words, the isolated antigen-binding protein defined in the present application may be an antibody, which is immunospecifically bound to a relevant antigen (for example, human AXL) and contains a framework region (FR) substantially having the amino acid sequence of a human antibody and a complementarity determining region (CDR) substantially having the amino acid sequence of a non-human antibody, or a variant, derivative, analog or fragment thereof. Here, "substantially" in the context of a CDR means that the amino acid sequence of the CDR has at least 80%, preferably at least 85%, at least 90%, at least 95%, at least 98%, or at least 99% identity to the amino acid sequence of the CDR of a non-human antibody. The humanized antibody may comprise substantially all at least one and generally two variable domains (Fab, Fab', F(ab')2, FabC, or Fv), wherein all or substantially all CDR regions correspond to the CDR regions of a non-human immunoglobulin (i.e., an antibody), and all or substantially all framework regions are framework regions having consensus sequences to a human immunoglobulin. Preferably, the humanized antibody also contains at least a fraction of a constant region (for example, Fc) of an immunoglobulin, which is typically the constant region of human immunoglobulin. In some embodiments, the humanized antibody contains a light chain and at least the variable domain of a heavy chain. The

antibody may also include the CH1, hinge, CH2, CH3, and CH4 regions of a heavy chain. In some embodiments, the humanized antibody contains only a humanized light chain. In some embodiments, the humanized antibody contains only a humanized heavy chain. In a particular embodiment, the humanized antibody contains a light chain and/or the humanized variable domain of a humanized heavy chain.

In the present application, the antigen-binding fragment may include Fab, Fab', $F(ab)_2$, a Fv fragment, a $F(ab')_2$, scFv, di-scFv, and/or dAb.

CDR

CDR of an antibody, also known as a complementarity determining region, in a part of a variable region. An amino acid residue in this region makes contact with an antigen or antigenic epitope. The CDR of antibody can be determined by a variety of coding systems, such as CCG, Kabat, Chothia, IMGT, the combination of Kabat/Chothia, etc. These encoding systems are known in the art. See http://www.bioinf.org.uk/abs/index.html#kabatnum for details. Those skilled in the art can determine a CDR region by using different coding systems according to the sequence and structure of an antibody. The CDR region may be different depending on the use of different coding systems. The CDR of the isolated antigen-binding protein defined in the present application can be determined by busing Kabat.

In the present application, the VH of the isolated antigen-binding protein may contain HCDR1, HCDR2 and HCDR3.

In the present application, the HCDR1 may contain an amino acid sequence as set forth in SEQ ID NO: 25.

In the present application, the HCDR2 may contain amino acid sequences as set forth in SEQ ID NOs: 26, 44, and 45.

In the present application, the HCDR3 may contain an amino acid sequence as set forth in SEQ ID NO: 27.

For example, the HCDR1 of the isolated antigen-binding protein defined in the present application may contain an amino acid sequence as set forth in SEQ ID NO: 25, the HCDR2 may contain an amino acid sequence as set forth in SEQ ID NO: 26, and the HCDR3 may contain an amino acid sequence as set forth in SEQ ID NO: 27.

For example, the HCDR1 of the isolated antigen-binding protein defined in the present application may contain an amino acid sequence as set forth in SEQ ID NO: 25, the HCDR2 may contain an amino acid sequence as set forth in SEQ ID NO: 44, and the HCDR3 may contain an amino acid sequence as set forth in SEQ ID NO: 27.

The HCDR1 of the isolated antigen-binding protein defined in the present application may contain an amino acid sequence as set forth in SEQ ID NO: 25, the HCDR2 may contain an amino acid sequence as set forth in SEQ ID NO: 45, and the HCDR3 may contain an amino acid sequence as set forth in SEQ ID NO: 27.

In the present application, the LCDR1 may contain an amino acid sequence as set forth in SEQ ID NO: 28.

In the present application, the LCDR2 may contain an amino acid sequence as set forth in SEQ ID NO: 29.

In the present application, the LCDR3 may contain an amino acid sequence as set forth in SEQ ID NO: 30.

For example, the LCDR1 of the isolated antigen-binding protein defined in the present application may contain an amino acid sequence as set forth in SEQ ID NO: 28, the LCDR2 may contain an amino acid sequence as set forth in SEQ ID NO: 29, and the LCDR3 may contain an amino acid sequence as set forth in SEQ ID NO: 30.

For another example, in the isolated antigen-binding protein defined in the present application, the HCDR1 may contain an amino acid sequence as set forth in SEQ ID NO: 25, the HCDR2 may contain an amino acid sequence as set forth in SEQ ID NO: 26, and the HCDR3 may contain an amino acid sequence as set forth in SEQ ID NO: 27; and the LCDR1 may contain an amino acid sequence as set forth in SEQ ID NO: 28, the LCDR2 may contain an amino acid sequence as set forth in SEQ ID NO: 29, and the LCDR3 may contain an amino acid sequence as set forth in SEQ ID NO: 30.

For another example, in the isolated antigen-binding protein defined in the present application, the HCDR1 may contain an amino acid sequence as set forth in SEQ ID NO: 25, the HCDR2 may contain an amino acid sequence as set forth in SEQ ID NO: 44, and the HCDR3 may contain an amino acid sequence as set forth in SEQ ID NO: 27; and the LCDR1 may contain an amino acid sequence as set forth in SEQ ID NO: 28, the LCDR2 may contain an amino acid sequence as set forth in SEQ ID NO: 29, and the LCDR3 may contain an amino acid sequence as set forth in SEQ ID NO: 30.

For another example, in the isolated antigen-binding protein defined in the present application, the HCDR1 may contain an amino acid sequence as set forth in SEQ ID NO: 25, the HCDR2 may contain an amino acid sequence as set forth in SEQ ID NO: 45, and the HCDR3 may contain an amino acid sequence as set forth in SEQ ID NO: 27; and the LCDR1 may contain an amino acid sequence as set forth in SEQ ID NO: 28, the LCDR2 may contain an amino acid sequence as set forth in SEQ ID NO: 29, and the LCDR3 may contain an amino acid sequence as set forth in SEQ ID NO: 30.

FR

In the present application, the VH of the isolated antigen-binding protein may contain framework regions H-FR1, H-FR2, H-FR3, and H-FR4.

In the present application, a C-terminus of the H-FR1 is directly or indirectly linked to an N-terminus of the HCDR1, and the H-FR1 may contain an amino acid sequence as set forth in SEQ ID NO: 7.

QVQL $X_1$ QSGPGLVKPSQSLSLTC $X_2$ V $X_3$ G $X_4$ SIS (SEQ ID NO: 7), wherein $X_1$ may be K or V, $X_2$ may be S or A, $X_3$ may be T or S, and $X_4$ may be F, Y, D, or S.

For example, in the present application, the H-FR1 may contain an amino acid sequence as set forth in any one of SEQ ID NOs: 11 and 15.

In the present application, the H-FR2 may be located between the HCDR1 and the HCDR2, and the H-FR2 may contain an amino acid sequence as set forth in SEQ ID NO: 8.

WIRQ $X_1$ PG $X_2$ $X_3$ LEWMG (SEQ ID NO: 8), wherein $X_1$ may be F or S, $X_2$ may be N or Q, and $X_3$ may be K or G.

For example, in the present application, the H-FR2 may contain an amino acid sequence as set forth in SEQ ID NO: 12.

In the present application, the H-FR3 may be located between the HCDR2 and the HCDR3, and the H-FR3 may contain an amino acid sequence as set forth in SEQ ID NO: 9.

RISITRDTSKNQFFLKLNSVT $X_1$ EDTATYYCAR (SEQ ID NO: 9), wherein $X_1$ may be T or S.

For example, in the present application, the H-FR3 may contain an amino acid sequence as set forth in SEQ ID NO: 13.

In the present application, an N-terminus of the H-FR4 may be linked to a C-terminus of the HCDR3, and the H-FR4 may contain an amino acid sequence as set forth in SEQ ID NO: 10.

WGQGT $X_1$ VTVSS (SEQ ID NO: 10), wherein $X_1$ may be S or T.

For example, in the present application, the H-FR4 may contain an amino acid sequence as set forth in SEQ ID NO: 14.

For another example, the H-FR1 of the isolated antigen-binding protein defined in the present application may contain an amino acid sequence as set forth in SEQ ID NO: 11, the H-FR2 may contain an amino acid sequence as set forth in SEQ ID NO: 12, the H-FR3 may contain an amino acid sequence as set forth in SEQ ID NO: 13, and the H-FR4 may contain an amino acid sequence as set forth in SEQ ID NO: 14.

For still another example, the H-FR1 of the isolated antigen-binding protein defined in the present application may contain an amino acid sequence as set forth in SEQ ID NO: 15, the H-FR2 may contain an amino acid sequence as set forth in SEQ ID NO: 12, the H-FR3 may contain an amino acid sequence as set forth in SEQ ID NO: 13, and the H-FR4 may contain an amino acid sequence as set forth in SEQ ID NO: 14.

In the present application, the VL of the isolated antigen-binding protein may contain framework regions L-FR1, L-FR2, L-FR3, and L-FR4.

In the present application, a C-terminus of the L-FR1 may be directly or indirectly linked to an N-terminus of the LCDR1, and the L-FR1 may contain an amino acid sequence as set forth in SEQ ID NO: 16.

$X_1$ $X_2$ VMTQSP $X_3$ S $X_4$ $X_5$ VTLG $X_6$ SASISC (SEQ ID NO: 16), wherein $X_1$ may be E or D, $X_2$ may be L or I, $X_3$ may be F or S, $X_4$ may be N or V, $X_5$ may be A or S, and $X_6$ may be T or Q. For example, in the present application, the L-FR1 may contain an amino acid sequence as set forth in any one of SEQ ID NOs: 20 and 24.

In the present application, the L-FR2 may be located between the LCDR1 and the LCDR2, and the L-FR2 may contain an amino acid sequence as set forth in SEQ ID NO: 17.

WY $X_1$ QKPGQSPQLLIY (SEQ ID NO: 17), wherein $X_1$ may be L or Q.

For example, in the present application, the L-FR2 may contain an amino acid sequence as set forth in SEQ ID NO: 21.

In the present application, the L-FR3 may be located between the LCDR2 and the LCDR3, and the L-FR3 may contain an amino acid sequence as set forth in SEQ ID NO: 18.

GVPDRFS $X_1$ SGSGTDFTL $X_2$ ISRVEAEDVGVYYC (SEQ ID NO: 18), wherein $X_1$ may be S or G, and $X_2$ may be R or K.

For example, in the present application, the L-FR3 may contain an amino acid sequence as set forth in SEQ ID NO: 22.

In the present application, an N-terminus of the L-FR4 may be linked to a C-terminus of the LCDR3, and the L-FR4 may contain an amino acid sequence as set forth in SEQ ID NO: 19.

FG $X_1$ GTKLE $X_2$ K (SEQ ID NO: 19), wherein $X_1$ may be A or G, and $X_2$ may be L or I.

For example, in the present application, the L-FR4 may contain an amino acid sequence as set forth in SEQ ID NO: 23.

For another example, the L-FR1 of the isolated antigen-binding protein defined in the present application may contain an amino acid sequence as set forth in SEQ ID NO: 20, the L-FR2 may contain an amino acid sequence as set

forth in SEQ ID NO: 21, the L-FR3 may contain an amino acid sequence as set forth in SEQ ID NO: 22, and the L-FR4 may contain an amino acid sequence as set forth in SEQ ID NO: 23.

For another example, the L-FR1 of the isolated antigen-binding protein defined in the present application may contain an amino acid sequence as set forth in SEQ ID NO: 24, the L-FR2 may contain an amino acid sequence as set forth in SEQ ID NO: 21, the L-FR3 may contain an amino acid sequence as set forth in SEQ ID NO: 22, and the L-FR4 may contain an amino acid sequence as set forth in SEQ ID NO: 23.

For another example, in the isolated antigen-binding protein defined in the present application, the H-FR1 may contain an amino acid sequence as set forth in SEQ ID NO: 11, the H-FR2 may contain an amino acid sequence as set forth in SEQ ID NO: 12, the H-FR3 may contain an amino acid sequence as set forth in SEQ ID NO: 13, and the H-FR4 may contain an amino acid sequence as set forth in SEQ ID NO: 14; and the L-FR1 may contain an amino acid sequence as set forth in SEQ ID NO: 20, the L-FR2 may contain an amino acid sequence as set forth in SEQ ID NO: 21, the L-FR3 may contain an amino acid sequence as set forth in SEQ ID NO: 22, and the L-FR4 may contain an amino acid sequence as set forth in SEQ ID NO: 23.

For another example, in the isolated antigen-binding protein defined in the present application, the H-FR1 may contain an amino acid sequence as set forth in SEQ ID NO: 15, the H-FR2 may contain an amino acid sequence as set forth in SEQ ID NO: 12, the H-FR3 may contain an amino acid sequence as set forth in SEQ ID NO: 13, and the H-FR4 may contain an amino acid sequence as set forth in SEQ ID NO: 14; and the L-FR1 may contain an amino acid sequence as set forth in SEQ ID NO: 24, the L-FR2 may contain an amino acid sequence as set forth in SEQ ID NO: 21, the L-FR3 may contain an amino acid sequence as set forth in SEQ ID NO: 22, and the L-FR4 may contain an amino acid sequence as set forth in SEQ ID NO: 23.

## VH and VL

The isolated antigen-binding protein defined in the present application may contain an antibody light-chain variable region VH and an antibody heavy-chain variable region VL.

In the present application, the VH may contain an amino acid sequence as set forth in any one of SEQ ID NOs: 3, 5, 42, and 43.

In the present application, the VL may contain an amino acid sequence as set forth in any one of SEQ ID NOs: 4 and 6.

For example, the VH may contain an amino acid sequence as set forth in SEQ ID NO: 3, and the VL may contain an amino acid sequence as set forth in SEQ ID NO: 4.

For another example, the VH may contain an amino acid sequence as set forth in SEQ ID NO: 5, and the VL may contain an amino acid sequence as set forth in SEQ ID NO: 6.

For another example, the VH may contain an amino acid sequence as set forth in SEQ ID NO: 42, and the VL may contain an amino acid sequence as set forth in SEQ ID NO: 6.

For another example, the VH may contain an amino acid sequence as set forth in SEQ ID NO: 43, and the VL may contain an amino acid sequence as set forth in SEQ ID NO: 6.

## Constant Region, Heavy Chain, and Light Chain

In the present application, the isolated antigen-binding protein may include an antibody heavy-chain constant region, which may be derived from a human IgG heavy-chain constant region.

In certain embodiments, the isolated antigen-binding protein may include an antibody heavy-chain constant region, which may be derived from a human IgG1 heavy-chain constant region. In some other embodiments, the isolated antigen-binding protein may include an antibody heavy-chain constant region, which may be derived from a human IgG4 heavy-chain constant region.

For example, the antibody heavy-chain constant region may contain an amino acid sequence as set forth in SEQ ID NO: 33 and 41.

In the present application, the isolated antigen-binding protein may include an antibody light-chain constant region, which may include a human Igκ constant region. For example, the antibody light-chain constant region may contain an amino acid sequence as set forth in SEQ ID NO: 34.

In the present application, the isolated antigen-binding protein may contain an antibody heavy chain HC, which may contain an amino acid sequence as set forth in any one of SEQ ID NOs: 35 and 37.

In the present application, the isolated antigen-binding protein may contain an antibody light chain LC, which may contain an amino acid sequence as set forth in any one of SEQ ID NOs: 36 and 38.

The isolated antigen-binding protein defined in the present application may contain an antibody heavy chain and an antibody light chain.

For example, the heavy chain may contain an amino acid sequence as set forth in SEQ ID NO: 35, and the

light chain may contain an amino acid sequence as set forth in SEQ ID NO: 36.

For example, the heavy chain may contain an amino acid sequence as set forth in SEQ ID NO: 37, and the light chain may contain an amino acid sequence as set forth in SEQ ID NO: 38.

In the present application, in the isolated antigen-binding protein, the heavy chain may contain an amino acid sequence as set forth in SEQ ID NO: 35, and the light chain may contain an amino acid sequence as set forth in SEQ ID NO: 36. In the isolated antigen-binding protein, the HCDR1 may contain an amino acid sequence as set forth in SEQ ID NO: 25, the HCDR2 may contain an amino acid sequence as set forth in SEQ ID NO: 26, and the HCDR3 may contain an amino acid sequence as set forth in SEQ ID NO: 27; and the LCDR1 may contain an amino acid sequence as set forth in SEQ ID NO: 28, the LCDR2 may contain an amino acid sequence as set forth in SEQ ID NO: 29, and the LCDR3 may contain an amino acid sequence as set forth in SEQ ID NO: 30. In addition, in the isolated antigen-binding protein, the H-FR1 may contain an amino acid sequence as set forth in SEQ ID NO: 11, the H-FR2 may contain an amino acid sequence as set forth in SEQ ID NO: 12, the H-FR3 may contain an amino acid sequence as set forth in SEQ ID NO: 13, and the H-FR4 may contain an amino acid sequence as set forth in SEQ ID NO: 14; and the L-FR1 may contain an amino acid sequence as set forth in SEQ ID NO: 20, the L-FR2 may contain an amino acid sequence as set forth in SEQ ID NO: 21, the L-FR3 may contain an amino acid sequence as set forth in SEQ ID NO: 22, and the L-FR4 may contain an amino acid sequence as set forth in SEQ ID NO: 23. In addition, the VH may contain an amino acid sequence as set forth in SEQ ID NO: 3, and the VL may contain an amino acid sequence as set forth in SEQ ID NO: 4. For example, the isolated antigen-binding protein may be a 6G12M11 antibody.

In the present application, in the isolated antigen-binding protein, the heavy chain may contain an amino acid sequence as set forth in SEQ ID NO: 37, and the light chain may contain an amino acid sequence as set forth in SEQ ID NO: 38. In the isolated antigen-binding protein, the HCDR1 may contain an amino acid sequence as set forth in SEQ ID NO: 25, the HCDR2 may contain an amino acid sequence as set forth in SEQ ID NO: 26, and the HCDR3 may contain an amino acid sequence as set forth in SEQ ID NO: 27; and the LCDR1 may contain an amino acid sequence as set forth in SEQ ID NO: 28, the LCDR2 may contain an amino acid sequence as set forth in SEQ ID NO: 29, and the LCDR3 may contain an amino acid sequence as set forth in SEQ ID NO: 30. In addition, in the isolated antigen-binding protein, the H-FR1 may contain an amino acid sequence as set forth in SEQ ID NO: 15, the H-FR2 may contain an amino acid sequence as set forth in SEQ ID NO: 12, the H-FR3 may contain an amino acid sequence as set forth in SEQ ID NO: 13, and the H-FR4 may contain an amino acid sequence as set forth in SEQ ID NO: 14; and the L-FR1 may contain an amino acid sequence as set forth in SEQ ID NO: 24, the L-FR2 may contain an amino acid sequence as set forth in SEQ ID NO: 21, the L-FR3 may contain an amino acid sequence as set forth in SEQ ID NO: 22, and the L-FR4 may contain an amino acid sequence as set forth in SEQ ID NO: 23. In addition, the VH may contain an amino acid sequence as set forth in SEQ ID NO: 5, and the VL may contain an amino acid sequence as set forth in SEQ ID NO: 6. For example, the isolated antigen-binding protein may be a 6G12M21 antibody.

In the present application, in the isolated antigen-binding protein, the HCDR1 may contain an amino acid sequence as set forth in SEQ ID NO: 25, the HCDR2 may contain an amino acid sequence as set forth in SEQ ID NO: 44, and the HCDR3 may contain an amino acid sequence as set forth in SEQ ID NO: 27; and the LCDR1 may contain an amino acid sequence as set forth in SEQ ID NO: 28, the LCDR2 may contain an amino acid sequence as set forth in SEQ ID NO: 29, and the LCDR3 may contain an amino acid sequence as set forth in SEQ ID NO: 30. In addition, in the isolated antigen-binding protein, the H-FR1 may contain an amino acid sequence as set forth in SEQ ID NO: 15, the H-FR2 may contain an amino acid sequence as set forth in SEQ ID NO: 12, the H-FR3 may contain an amino acid sequence as set forth in SEQ ID NO: 13, and the H-FR4 may contain an amino acid sequence as set forth in SEQ ID NO: 14; and the L-FR1 may contain an amino acid sequence as set forth in SEQ ID NO: 24, the L-FR2 may contain an amino acid sequence as set forth in SEQ ID NO: 21, the L-FR3 may contain an amino acid sequence as set forth in SEQ ID NO: 22, and the L-FR4 may contain an amino acid sequence as set forth in SEQ ID NO: 23. In addition, the VH may contain an amino acid sequence as set forth in SEQ ID NO: 42, and the VL may contain an amino acid sequence as set forth in SEQ ID NO: 4. For example, the isolated antigen-binding protein may be a 6G12M31 antibody.

In the present application, in the isolated antigen-binding protein, the HCDR1 may contain an amino acid sequence as set forth in SEQ ID NO: 25, the HCDR2 may contain an amino acid sequence as set forth in SEQ ID NO: 45, and the HCDR3 may contain an amino acid sequence as set forth in SEQ ID NO: 27; and the LCDR1 may contain an amino acid sequence as set forth in SEQ ID NO: 28, the LCDR2 may contain an amino acid sequence as set forth in SEQ ID NO: 29, and the LCDR3 may contain an amino acid sequence as set forth in SEQ ID NO: 30. In addition, in the isolated antigen-binding protein, the H-FR1 may contain an amino acid sequence as set forth in SEQ ID NO: 15, the H-FR2 may contain an amino acid sequence as set forth in SEQ ID NO: 12, the H-FR3 may contain an amino acid sequence as set forth in SEQ ID NO: 13, and the H-FR4 may contain an amino acid sequence as set forth in SEQ ID NO: 14; and the L-FR1 may contain an amino acid sequence as set forth in SEQ ID NO: 24, the L-FR2 may contain an amino acid sequence as set forth in SEQ ID NO: 21, the L-FR3 may contain an amino acid sequence as set forth in SEQ ID NO: 22, and the L-FR4 may contain an amino acid sequence as set forth in SEQ ID NO: 23. In addition, the VH may contain an amino acid sequence as set forth in SEQ ID NO: 43, and the VL may contain an amino acid sequence

as set forth in SEQ ID NO: 6. For example, the isolated antigen-binding protein may be a 6G12M41 antibody.

In addition, it should be noted that the isolated antigen-binding protein defined in the present application may contain a heavy and/or light chain sequence with one or more conservative sequence modifications in relation to the 6G12M11, 6G12M21, 6G12M31 and 6G12M41 antibodies. The socalled "conservative sequence modifications" mean amino acid modifications that would not significantly affect or alter the binding properties of an antibody. Such conservative modifications include amino acid substitutions, additions, and deletions. These modifications may be introduced into the isolated antigen-binding protein defined in the present application by standard techniques known in the art, such as point mutation and PCR-mediated mutation. The conservative amino acid substitutions refer to substitutions of amino acid residues with amino acid residues having similar side chains. There are sets of amino acid residues, having similar side chains, that are known in the art. These sets of amino acid residues include amino acids with basic side chains (for example, lysine, arginine, and histidine), acidic side chains (for example, aspartic acid, and glutamic acid), uncharged polar side chains (for example, glycine, asparagine, glutamine, serine, threonine, tyrosine, cysteine, and tryptophan), non-polar side chains (for example, alanine, valine, leucine, isoleucine, proline, phenylalanine, and methionine), β-branched side chains (for example, threonine, valine, and isoleucine), and aromatic side chains (for example, tyrosine, phenylalanine, tryptophan, and histidine). In certain embodiments, one or more amino acid residues in the CDR region of the isolated antigen-binding protein defined in the present application may be substituted with other amino acid residues in the same side chain set. Those skilled in the art know that some conservative sequence modifications do not abolish antigen binding. For details, see, for example, Brummell et al., (1993) Biochem 32:1180-8; de Wildt et al., (1997) Prot. Eng. 10:835-41; Komissarov et al., (1997), J. Biol. Chem. 272:26864-26870; Hall et al., (1992) J. Immunol. 149:1605-12; Kelley and O'Connell (1993) Biochem. 32:6862-35; Adib-Conquy et al., (1998) Int. Immunol. 10:341-6; and Beers et al., (2000) Clin. Can. Res. 6:2835-43.

The protein, polypeptide, and/or amino acid sequence involved in the present application should also be understood to cover at least the following range: a variant or homologue that has the same or similar functions as said protein or polypeptide.

In the present application, the variant may be a protein or polypeptide formed by substituting, deleting or adding one or more amino acids in an amino acid sequence of the defined protein and/or polypeptide (for example, the isolated antigen-binding protein defined in the present application). For example, the variant may include a protein or polypeptide with amino acid changes induced by substituting, deleting, and/or inserting at least one amino acid, for example, 1-30, 1-20, or 1-10 amino acids, and for another example, 1, 2, 3, 4, or 5 amino acids. The functional variant may substantially maintain the biological properties of said protein or polypeptide before the changes (for example, substitution, deletion, or addition). For example, the functional variant may maintain at least 60%, 70%, 80%, 90%, or 100% of the biological activity (for example, the capability of specifically binding to the AXL protein) of the defined protein or polypeptide before the changes.

In the present application, the homologue may be a protein or polypeptide that has at least about 80% (for example, at least about 85%, about 90%, about 91%, about 92%, about 93%, about 94%, about 95%, about 96%, about 97%, about 98%, about 99% or higher) sequence homology with the amino acid sequence of the defined protein and/or polypeptide (for example, the antibody defined in the present application, or an antigen-binding fragment thereof).

In the present application, the homology generally refers to the level of similarity or association between two or more sequences. The "percentage of sequence homology" may be calculated in the following way: comparing two sequences to be aligned in a comparison window, determining in the two sequences the number of positions at which identical nucleic acid bases (for example, A, T, C, G and I) or identical amino acid residues (for example, Ala, Pro, Ser, Thr, Gly, Val, Leu, Ile, Phe, Tyr, Trp, Lys, Arg, His, Asp, Glu, Asn, Gln, Cys, and Met) exist to acquire the number of matching positions; and dividing the number of matching positions by the total number of positions in the comparison window (i.e., the window size), and multiplying a result by 100 to produce the sequence homology percentage. The alignment for determining the sequence homology percentage may be achieved in a variety of ways known in the art, for example, by using publicly available computer software such as BLAST, BLAST-2, ALIGN or Megalign (DNASTAR) software. Those skilled in the art may determine appropriate parameters for sequence alignment, including any algorithm required to implement the maximum alignment undergoing comparison, within a full-length sequence range or within a target sequence region. The homology may also be determined by the following methods: FASTA and BLAST. For the description of the FASTA algorithm, a reference may be made to W. R. Pearson and D. J. Lipman, "Improved Tools for Biological Sequence Comparison", Proc. Natl. Acad. Sci.) , 85: 2444-2448, 1988; and D. J. Lipman and W. R. Pearson, "Rapid and Sensitive Protein Similarity Searches", Science, 227: 1435-1441, 1989. For the description of the BLAST algorithm, a reference may be made to S. Altschul, W. Gish, W. Miller, E. W. Myers and D. Lipman, "Basic Local Alignment Search Tool", J. Mol. Biol., 215: 403-410, 1990.

**Immunoconjugate, Nucleic Acid Molecule, Vector, Cell, and Pharmaceutical Composition**

Immunoconjugate

In another aspect, the present application provides an immunoconjugate, containing the defined isolated antigen-binding protein.

For example, the immunoconjugate may contain at least one additional agent selected from the group consisting of a chemotherapeutic agent, a radioactive element, a cytostatic agent, and a cytotoxic agent. In certain embodiments, the isolated antigen-binding protein in the immunoconjugate is linked to the at least one additional agent by a linker molecule. For example, in the immunoconjugate, isolated antigen-binding protein and the at least one additional agent may be covalently linked to the linker molecule, respectively.

In the present application, the defined additional agent may include maytansine or a derivative thereof. For example, the maytansine derivative may include a maytansine derivative DM1.

Nucleic Acid Molecule

In another aspect, the present application further provides one or more isolated nucleic acid molecules, which can encode the isolated antigen-binding protein defined in the present application. The one or more isolated nucleic acid molecules defined in the present application may be nucleotides, deoxyribonucleotides, or ribonucleotides in the isolated form of any length, or analogs, which are isolated from their natural environment or artificially synthesized, but can encode the isolated antigen-binding protein defined in the present application.

Vector

In another aspect, the present application further provides a vector, which may contain the nucleic acid molecule defined in the present application. The vector may be transformed, transduced, or transfected into a host cell, such that genetic material elements carried thereby can be expressed in a host cell. For example, the vector may include: a plasmid; a phagemid; a cosmid; an artificial chromosome, such as a yeast artificial chromosome (YAC), a bacterial artificial chromosome (BAC), or a P1-derived artificial chromosome (PAC); and a bacteriophage, such as a λ phage or an M13 phage, an animal virus etc. The species of animal viruses used as vectors include retroviruses (including lentiviruses), adenoviruses, adeno-associated viruses, herpesviruses (for example, herpes simplex viruses), poxviruses, baculoviruses, papillomaviruses, and papovaviruses (for example, SV40). For another example, the vector may contain a variety of elements that control expression, including a promoter sequence, a transcription initiation sequence, an enhancer sequence, a selection element, and a reporter gene. In addition, the vector may further contain an origin of replication site. In addition, the vector may further include components, such as, but not limited only to, a virus particle, a liposome, or a protein coat, that assist the vector in entering the cell.

Cell

In another aspect, the present application further provides a cell, which may contain the nucleic acid molecule defined in the present application or said vector defined in the present application. The cell may include a progeny of a single cell. Due to natural, accidental, or deliberate mutations, the progeny may not necessarily be exactly the same as an original parent cell (in the form of total DNA complement or in the genome). In certain embodiments, the cell may further include a cell transfected in vitro by using the vector defined in the present application. In certain embodiments, the cell may be a bacterial cells (for example, *E. coli),* a yeast cell, or other eukaryotic cells, such as a COS cell, a Chinese hamster ovary (CHO) cell, a CHO-K1 cell, an LNCAP cell, a HeLa cell, a HEK293 cell, a COS-1 cells, an NS0 cell, a myeloma cell, a human non-small cell lung cancer A549 cell, a human cutaneous squamous cell carcinoma A431 cell, a renal clear cell adenocarcinoma 786-O cell, a human pancreatic cancer MIA PaCa-2 cell, an erythroleukemia K562 cell, an acute T cell leukemia Jurkat cell, a human breast cancer MCF-7 cell, a human breast cancer MDA-MB-231 cell, a human breast cancer MDA-MB-468 cell, a human breast cancer SKBR3 cell, a human ovarian cancer SKOV3 cell, a lymphoma U-937 cell, a lymphoma Raji cell, a human myeloma U266, or a human multiple myeloma RPMI8226 cell. In certain embodiments, the cell may be a mammalian cell. In certain embodiments, the mammalian cell may be a HEK293 cell.

Pharmaceutical Composition

In another aspect, the present application further provides a pharmaceutical composition, which may contain the isolated antigen-binding protein defined in the present application, the immunoconjugate defined in the present

application, the nucleic acid molecule defined in the present application, the vector defined in the present application, and/or the cell defined in the present application, and optionally a pharmaceutically acceptable adjuvant.

In certain embodiments, the pharmaceutical composition may further contain a suitable agent of one or more (pharmaceutically effective) carriers, stabilizers, excipients, diluents, solubilizers, surfactants, emulsifiers and/or preservatives. The acceptable ingredient of the composition is preferably nontoxic to a subject at a dose and concentration as used. The pharmaceutical composition of the present invention includes, but is not limited to, a liquid, frozen, and lyophilized composition. In certain embodiments, the pharmaceutically acceptable adjuvant generally may include any and all solvents, dispersion media, coatings, isotonic agents, and absorption retarders, that are compatible with pharmaceutical administration. Such adjuvant is generally safe and non-toxic, and is neither biologically nor otherwise undesirable.

In certain embodiments, the pharmaceutical composition may be administered parenterally, transdermally, intraluminally, intraarterially, intrathecally and/or intranasally, or may be directly injected into a tissue. For example, the pharmaceutical composition may be administered to a patient or subject by infusion or injection. In certain embodiments, the pharmaceutical composition can be administered by different means, for example, intravenous, intratumoral, intraperitoneal, subcutaneous, intramuscular, topical or intradermal administration. In certain embodiments, the pharmaceutical composition may be administered uninterruptedly. The uninterrupted (or continuous) administration may be achieved by a small pump system, worn by a patient, which is used to measure a therapeutic agent flowing into the patient, as described in WO2015/036583.

A dosing regimen for the pharmaceutical composition may include administering the pharmaceutical composition as a single bolus, administering the pharmaceutical composition in multiple fractional doses over time, or reducing or increasing a dose in proportion to the degree of exigency of treatment condition. In certain embodiments, a therapeutic regimen may include administering the pharmaceutical composition once a week, once every two weeks, once every three weeks, once every four weeks, once a month, once every 3 months, or once every 3-6 months. In certain embodiments, the dosing regimen includes intravenously administering the antibody at 1 mg/kg body weight or 3 mg/kg body weight based on one of the following dosing schedules: (i) six doses every four weeks, and then once every three months; (ii) once every three weeks; and (iii) once at 3 mg/kg body weight, and then once every three weeks at 1 mg/kg body weight. In certain embodiments, the dose is adjusted to achieve a blood concentration of about 1-1000 μg/ml, for example, about 25-300 μg/ml.

## Preparation Method and Use

### Preparation Method

In another aspect, the present application further provides a preparation method for the isolated antigen-binding protein defined in the present application, wherein the method may include culturing the cell defined in the present application, under a condition of allowing the expression of the isolated antigen-binding protein defined in the present application.

### Use

In another aspect, the present application further provides use of the isolated antigen-binding protein, the immunoconjugate, the nucleic acid molecule, the vector, the cell, and/or the pharmaceutical composition in the preparation of a drug for preventing, relieving and/or treating a tumor.

In another aspect, the present application further provides a method for preventing, relieving or treating a tumor, wherein the method may include administering the isolated antigen-binding protein, immunoconjugate, nucleic acid molecule, vector, cell, and/or pharmaceutical composition defined in the present application to a subject in need thereof. In the present application, the administration may be carried out by different means, for example, intravenous, intratumoral, intraperitoneal, subcutaneous, intramuscular, topical or intradermal administration.

In another aspect, the isolated antigen-binding protein, immunoconjugate, nucleic acid molecule, vector, cell, and/or pharmaceutical composition defined in the present application may be used for preventing, relieving and/or treating a tumor.

In the present application, the tumor may include a solid tumor or hematologic tumor.

In the present application, the tumor may include a AXL positive tumor. For example, the AXL positive tumor may be selected from the group consisting of a lung cancer, a skin cancer, a kidney cancer, a pancreatic cancer, a hematologic tumor, a breast cancer, an ovarian cancer, a lymphoma, and a myeloma. For another example, the AXL positive tumor may be selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

In the present application, the subject may include a human or non-human animal. For example, the subject may include, but is not limited to, a cat, a dog, a horse, a pig, a cow, a goat, a rabbit, a mouse, a rat, or a monkey.

In the present application, the isolated antigen-binding protein may be administered with one or more additional antibodies to effectively inhibit tumor growth in a subject. In certain embodiments, the isolated antigen-binding protein and the one or more additional antibodies (for example, an antibody LAG-3, an antibody PD-1, and/or an antibody CTLA-4) may be administered to a subject. In the present application, the isolated antigen-binding protein may be administered together with a chemotherapeutic agent, which may be a cytotoxic agent, for example, SN-38, epirubicin, oxaliplatin, and/or 5-FU.

In another aspect, the present application provides use of the defined isolated antigen-binding protein in the diagnosis of a disease or condition associated with the expression of the AXL protein. In another aspect, the present application provides use of the defined antibody or the antigen-binding fragment thereof in the preparation of a diagnostic agent for diagnosing a disease or condition associated with the expression of the AXL protein.

In the present application, the diagnostic agent may be used alone or in combination with an instrument, appliance, device, or system. During the prevention, diagnosis, treatment and monitoring, and prognosis observation for a disease, the evaluation of health status, and the prediction of hereditary disease, the diagnostic agent may be used for in vitro detection of a human sample (for example, various body fluids, cells, tissue samples, etc.). The diagnostic agent may be selected from the group consisting of: a reagent, a kit, a calibrator, and a quality control.

The in vitro detection method may be selected from the group consisting of: Western Blot, ELISA, and immunohistochemistry. For example, the reagent may include a reagent capable of measuring the level of expression of the AXL protein. For example, the reagent may be selected from the group consisting of: a reagent for performing the Western Blot, a reagent for performing the ELISA, and a reagent for performing the immunohistochemistry.

In another aspect, the present application provides a method for diagnosing a disease or condition associated with the expression of an AXL protein in a subject, including: bringing a sample derived from the subject and said isolated antigen-binding protein into contact, and determining the presence and/or amount of a substance capable of specifically binding the isolated antigen-binding protein, in said sample.

In another aspect, the present application provides a method for detecting AXL in a sample, comprising administering the isolated antigen-binding protein. In the present application, the administration may be carried out by different means, for example, intravenous, intratumoral, intraperitoneal, subcutaneous, intramuscular, topical or intradermal administration.

In the present application, the disease or condition associated with the expression of the AXL protein may be selected from the group consisting of a lung cancer, a skin cancer, a kidney cancer, a pancreatic cancer, a hematologic tumor, a breast cancer, an ovarian cancer, a lymphoma, and a myeloma; or may be selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

In addition, the present application may further include the following embodiments.

1 An isolated antigen-binding protein, comprising at least one CDR in a VH as set forth in an amino acid sequence of SEQ ID NO: 1 or SEQ ID NO: 46; and comprising at least one CDR in a VL as set forth in an amino acid sequence of SEQ ID NO: 2.

2 The isolated antigen-binding protein according to embodiment 1, having one or more of the following properties:

1) capability of binding to an AXL protein at a $K_D$ of $1\times10^{-7}$M or lower;
2) capability of specifically recognizing an AXL protein expressed on a cell surface; and
3) capability of mediating internalization after binding to the AXL protein expressed on the cell surface.

3 The isolated antigen-binding protein according to embodiment 2, wherein said AXL protein comprises a human AXL protein.

4 The isolated antigen-binding protein according to embodiment 3, wherein said human AXL protein includes an amino acid sequence as set forth in SEQ ID NO: 39.

5 The isolated antigen-binding protein according to any one of embodiments 2-4, wherein said AXL protein comprises an extracellular domain.

6 The isolated antigen-binding protein according to embodiment 5, wherein said extracellular domain comprises an amino acid sequence as set forth in SEQ ID NO: 40.

7 The isolated antigen-binding protein according to embodiment 2, wherein said cell comprises a tumor cell.

8 The isolated antigen-binding protein according to embodiment 7, wherein said tumor comprises an AXL positive tumor.

9 The isolated antigen-binding protein according to embodiment 8, wherein said tumor is selected from the group consisting of a lung cancer, a skin cancer, a kidney cancer, a pancreatic cancer, a hematologic tumor, a breast

cancer, an ovarian cancer, a lymphoma, and a myeloma.

10 The isolated antigen-binding protein according to embodiment 9, wherein said tumor is selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

11 The isolated antigen-binding protein according to embodiment 2, wherein said cell comprises a human cell.

12 The isolated antigen-binding protein according to embodiment 7 or 11, wherein said cell is selected from the group consisting of a human non-small cell lung cancer A549 cell, a human cutaneous squamous cell carcinoma A431 cell, a renal clear cell adenocarcinoma 786-O cell, a human pancreatic cancer MIA PaCa-2 cell, an erythroleukemia K562 cell, an acute T cell leukemia Jurkat cell, a human breast cancer MCF-7 cell, a human breast cancer MDA-MB-231 cell, a human breast cancer MDA-MB-468 cell, a human breast cancer SKBR3 cell, a human ovarian cancer SKOV3 cell, a lymphoma U-937 cell, a lymphoma Raji cell, a human myeloma U266, and a human multiple myeloma RPMI8226 cell.

13 The isolated antigen-binding protein according to embodiment 1, wherein said VH comprises HCDR1, HCDR2 and HCDR3.

14 The isolated antigen-binding protein according to embodiment 13, wherein said HCDR1 comprises an amino acid sequence as set forth in SEQ ID NO: 25.

15 The isolated antigen-binding protein according to embodiment 13, wherein said HCDR2 comprises an amino acid sequence as set forth in any one of SEQ ID NOs: 26, 44, and 45.

16 The isolated antigen-binding protein according to embodiment 13, wherein said HCDR3 comprises an amino acid sequence as set forth in SEQ ID NO: 27.

17 The isolated antigen-binding protein according to embodiment 1, wherein said VL comprises LCDR1, LCDR2, and LCDR3.

18 The isolated antigen-binding protein according to embodiment 17, wherein said LCDR1 comprises an amino acid sequence as set forth in SEQ ID NO: 28.

19 The isolated antigen-binding protein according to embodiment 17, wherein said LCDR2 comprises an amino acid sequence as set forth in SEQ ID NO: 29.

20 The isolated antigen-binding protein according to embodiment 17, wherein said LCDR3 comprises an amino acid sequence as set forth in SEQ ID NO: 30.

21 The isolated antigen-binding protein according to embodiment 1 or 13, wherein said VH comprises framework regions H-FR1, H-FR2, H-FR3, and H-FR4.

22 The isolated antigen-binding protein according to embodiment 21, wherein a C-terminus of said H-FR1 is directly or indirectly linked to an N-terminus of said HCDR1, and said H-FR1 comprises an amino acid sequence as set forth in SEQ ID NO: 7.

23 The isolated antigen-binding protein according to embodiment 22, wherein said H-FR1 comprises an amino acid sequence as set forth in any one of SEQ ID NOs: 11 and 15.

24 The isolated antigen-binding protein according to embodiment 21, wherein said H-FR2 is located between said HCDR1 and said HCDR2, and said H-FR2 comprises an amino acid sequence as set forth in SEQ ID NO: 8.

25 The isolated antigen-binding protein according to embodiment 24, wherein said H-FR2 comprises an amino acid sequence as set forth in SEQ ID NO: 12.

26 The isolated antigen-binding protein according to embodiment 21, wherein said H-FR3 is located between said HCDR2 and said HCDR3, and said H-FR3 comprises an amino acid sequence as set forth in SEQ ID NO: 9.

27 The isolated antigen-binding protein according to embodiment 26, wherein said H-FR3 comprises an amino acid sequence as set forth in SEQ ID NO: 13.

28 The isolated antigen-binding protein according to embodiment 21, wherein an N-terminus of said H-FR4 is linked to a C-terminus of said HCDR3, and said H-FR4 comprises an amino acid sequence as set forth in SEQ ID NO: 10.

29 The isolated antigen-binding protein according to embodiment 28, wherein said H-FR4 comprises an amino acid sequence as set forth in SEQ ID NO: 14.

30 The isolated antigen-binding protein according to any one of embodiments 1, 13-16, and 22-29, wherein said VH comprises an amino acid sequence as set forth in any one of SEQ ID NOs: 3, 5, 42, and 43.

31 The isolated antigen-binding protein according to embodiment 1 or 17, wherein said VL comprises framework regions L-FR1, L-FR2, L-FR3, and L-FR4.

32 The isolated antigen-binding protein according to embodiment 31, wherein a C-terminus of said L-FR1 is directly or indirectly linked to an N-terminus of said LCDR1, and said L-FR1 comprises an amino acid sequence as set forth in SEQ ID NO: 16.

33 The isolated antigen-binding protein according to embodiment 32, wherein said L-FR1 comprises an amino acid sequence as set forth in any one of SEQ ID NOs: 20 and 24.

34 The isolated antigen-binding protein according to embodiment 31, wherein said L-FR2 is located between said

LCDR1 and said LCDR2, and said L-FR2 comprises an amino acid sequence as set forth in SEQ ID NO: 17.

35 The isolated antigen-binding protein according to embodiment 34, wherein said L-FR2 comprises an amino acid sequence as set forth in SEQ ID NO: 21.

36 The isolated antigen-binding protein according to embodiment 31, wherein said L-FR3 is located between said LCDR2 and said LCDR3, and said L-FR3 comprises an amino acid sequence as set forth in SEQ ID NO: 18.

37 The isolated antigen-binding protein according to embodiment 36, wherein said L-FR3 comprises an amino acid sequence as set forth in SEQ ID NO: 22.

38 The isolated antigen-binding protein according to embodiment 31, wherein an N-terminus of said L-FR4 is linked to a C-terminus of said LCDR3, and said L-FR4 comprises an amino acid sequence as set forth in SEQ ID NO: 19.

39 The isolated antigen-binding protein according to embodiment 38, wherein said L-FR4 comprises an amino acid sequence as set forth in SEQ ID NO: 23.

40 The isolated antigen-binding protein according to any one of embodiments 1, 17-20, and 32-39, wherein said VL comprises an amino acid sequence as set forth in any one of SEQ ID NOs: 4 and 6.

41 The isolated antigen-binding protein according to any one of embodiments 1-4, 6-11, 13-20, 22-29, and 32-39, comprising an antibody heavy-chain constant region, which is derived from a human IgG heavy-chain constant region.

42 The isolated antigen-binding protein according to embodiment 41, wherein said antibody heavy-chain constant region is derived from a human IgG1 heavy-chain constant region or a human IgG4 heavy-chain constant region.

43 The isolated antigen-binding protein according to embodiment 42, wherein said antibody heavy-chain constant region comprises an amino acid sequence as set forth in any one of SEQ ID NOs: 33 and 41.

44 The isolated antigen-binding protein according to any one of embodiments 1-4, 6-11, 13-20, 22-29, 32-39, and 42-43, comprising an antibody light-chain constant region, which comprises a human Igκ constant region.

45 The isolated antigen-binding protein according to embodiment 44, wherein said antibody light-chain constant region comprises an amino acid sequence as set forth in SEQ ID NO: 34.

46 The isolated antigen-binding protein according to any one of embodiments 1-4, 6-11, 13-20, 22-29, 32-39, 42-43, and 45, comprising an antibody heavy chain, which comprises an amino acid sequence as set forth in any one of SEQ ID NOs: 35 and 37.

47 The isolated antigen-binding protein according to any one of embodiments 1-4, 6-11, 13-20, 22-29, 32-39, 42-43, and 45, comprising an antibody light chain, which comprises an amino acid sequence as set forth in any one of SEQ ID NOs: 36 and 38.

48 The isolated antigen-binding protein according to any one of embodiments 1-4, 6-11, 13-20, 22-29, 32-39, 42-43, and 45, comprising an antibody or an antigen-binding fragment thereof.

49 The isolated antigen-binding protein according to embodiment 48, wherein said antibody is selected from the group consisting of a monoclonal antibody, a single chain antibody, a chimeric antibody, a multispecific antibody, a humanized antibody, and a fully human antibody.

50 The isolated antigen-binding protein according to embodiment 48, wherein said antigen-binding fragment is selected from the group consisting of: Fab, Fab', $F(ab)_2$, Fv, $F(ab')_2$, scFv, di-scFv, and dAb fragments.

51 An immunoconjugate, comprising the isolated antigen-binding protein of any one of embodiments 1-50.

52 The immunoconjugate according to embodiment 51, further comprising at least one additional agent selected from the group consisting of a chemotherapeutic agent, a radioactive element, a cytostatic agent, and a cytotoxic agent.

53 The immunoconjugate according to embodiment 52, wherein said isolated antigen-binding protein is linked to said additional agent by a linker molecule.

54 The immunoconjugate according to embodiment 53, wherein said isolated antigen-binding protein and said additional agent are covalently linked to said linker molecule, respectively.

55 The immunoconjugate according to any one of embodiments 52-54, wherein said additional agent comprises maytansine or a derivative thereof.

56 The immunoconjugate according to embodiment 55, wherein said maytansine derivative comprises a maytansine derivative DM1.

57 One or more isolated nucleic acid molecules, encoding said isolated antigen-binding protein of any one of embodiments 1-50.

58 A vector, comprising the nucleotide molecule of embodiment 57.

59 A cell, comprising said nucleic acid molecule of embodiment 57 or said vector of embodiment 58.

60 A pharmaceutical composition, comprising said isolated antigen-binding protein of any one of embodiments 1-50, said immunoconjugate of any one of embodiments 51-56, said nucleic acid molecule of embodiment 57, said vector of embodiment 58, and/or said cell of embodiment 59, and optionally a pharmaceutically acceptable adjuvant.

61 A preparation method for said isolated antigen-binding protein of any one of embodiments 1-50, wherein the method comprises culturing said cell of embodiment 59 under a condition of allowing the expression of said isolated antigen-binding protein of any one of embodiments 1-50.

62 Use of said isolated antigen-binding protein of any one of embodiments 1-50, said immunoconjugate of any one of embodiments 51-56, said nucleic acid molecule of embodiment 57, said vector of embodiment 58, said cell of embodiment 59, and/or said pharmaceutical composition of embodiment 60 in the preparation of a drug for preventing, relieving and/or treating a tumor.

63 The use according to embodiment 62, wherein said tumor comprises an AXL positive tumor.

64 The use according to embodiment 63, wherein said tumor is selected from the group consisting of a lung cancer, a skin cancer, a kidney cancer, a pancreatic cancer, a hematologic tumor, a breast cancer, an ovarian cancer, a lymphoma, and a myeloma.

65 The use according to embodiment 64, wherein said tumor is selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

66 Use of said isolated antigen-binding protein of any one of embodiments 1-50 in the preparation of a diagnostic agent for diagnosing a disease or condition associated with the expression of said AXL protein.

67 A method for diagnosing a disease or condition associated with the expression of an AXL protein in a subject, comprising: bringing a sample derived from the subject and said isolated antigen-binding protein of any one of embodiments 1-50 into contact, and determining the presence and/or amount of a substance capable of specifically binding the isolated antigen-binding protein, in the sample.

68 A method for detecting AXL in a sample, comprising administering said isolated antigen-binding protein of any one of embodiments 1-50.

Not wishing to be bound by any particular theory, the following examples are merely to illustrate the protein molecule, preparation methods and uses and the like according to the present application, and are not intended to limit the scope of the present invention. The examples do not include detailed descriptions of traditional methods, such as a method for constructing a vector and a plasmid, a method for inserting a protein-encoding gene into such a vector and plasmid, or a method for introducing a plasmid into a host cell. Such methods are well known to those ordinarily skilled in the art and have been described in many publications, including Sambrook, J., Fritsch, E. F. and Maniais, T. (1989) Molecular Cloning: A Laboratory Manual, 2nd edition, Cold spring Harbor Laboratory Press.

## Examples

### Example 1. Recognition of target antigens by antibodies

A target antigen AXL-His (Sino Biological Inc.) was coated at 1 μ g/ml on an ELISA strip, which was held at 4°C overnight. After the ELISA strip was washed with PBST, 10% fetal bovine serum was added to the ELISA strip, which was blocked for 1 hour at 37°C. Different concentrations of antibodies 6G12M11, 6G12M21, 6G12M31 and 6G12M41 were added to react at 37°C for 1 hour. After the ELISA strip was washed with PBST, a horseradish peroxidase-labeled goat anti-mouse secondary antibody (Goat Anti-mouse IgG HRP, Abcam) was added to react for 30 minutes at 37°C. The ELISA strip was washed 5 times with PBST, and patted and dried on absorbent paper to remove residual droplets as much as possible. 100 μl of TMB (eBioscience) was added to each well, and the ELISA strip was held for 1.5 min at room temperature (20±5°C) in the dark. 100 μl of 2N $H_2SO_4$ stop solution was added to each well to terminate substrate reaction. The OD value was read at 450 nm by a microplate reader to analyze the binding ability of each antibody to the target antigen AXL. The amino acid sequence of the heavy chain of antibody 6G12M11 is as set forth in SEQ ID NO: 35, and the amino acid sequence of the light chain of the same is as set forth in SEQ ID NO: 36; the amino acid sequence of the heavy chain of antibody 6G12M21 is as set forth in SEQ ID NO: 37, and the amino acid sequence of the light chain of the same is as set forth in SEQ ID NO: 38; the VH of antibody 6G12M31 is as set forth in SEQ ID NO: 42, and the VL of the same is as set forth in SEQ ID NO: 6; and the VH of antibody 6G12M41 is as set forth in SEQ ID NO: 43, and the VL of the same is as set forth in SEQ ID NO: 6.

Test results are as shown in FIGs. 1A and IB. It can be seen that both antibodies 6G12M11 and 6G12M21 can specifically recognize the target antigen AXL. This recognition activity is significantly dose-dependent, with 6G12M11 having an $EC_{50}$ value of 27.03 ng/mL, 6G12M21 having an $EC_{50}$ value of 25.29 ng/mL, 6G12M31 having an $EC_{50}$ value of 21.11ng/mL, and 6G12M41 having an $EC_{50}$ value of 17.56 ng/mL.

### Example 2. Affinity assay of antibodies

In this test, the affinity of AXL-His to each of the antibodies 6G12M11, 6G12M21, 6G12M31, and 6G12M41 was detected by the surface plasmon resonance technology. The test instrument used was BIACOR biomacromolecule interaction instrument (GE Healthcare, T-200). The antibodies 6G12M11, 6G12M21, 6G12M31, and 6G12M41 were immobilized on the surface of a chip by an antibody capturing method, and with all the concentrations set to 0.5 μg/mL,

were injected for 60 seconds at a flow rate of 10 μL/min. The prepared AXL-His (Sino Biological Inc., 6×His labels, molecular weight: 47.5 Kda) as a mobile phase was let to flow through the surface of the chip under 5 concentration gradients (8, 4, 2, 1, 0.5 μg/mL) respectively for interaction determination. The AXL-His underwent association for 120 s and dissociation for 1800 s at the flow rate of 30 μL/min. The affinity assay results of antibodies are shown in Table 1. It can be seen that the isolated antigen-binding protein defined in the present application can bind to the AXL protein at a KD of $1 \times 10^{-7}$M or lower.

Table 1. Affinity assay results of antibodies

| Antibody | Association constant Ka (1/Ms) | Dissociation constant Kd (1/s) | Relative affinity KD (M) |
|---|---|---|---|
| 6G12M11 | 9.742E+04 | 1.136E-03 | 1.166E-08 |
| 6G12M21 | 1.119E+05 | 1.137E-03 | 1.016E-08 |
| 6G12M31 | 3.97E+04 | 1.16E-03 | 2.92E-08 |
| 6G12M41 | 4.15E+04 | 1.19E-03 | 2.87E-08 |

**Example 3. Specific recognition of target antigens by antibodies**

AXL, TRYO3, and MER are all members of the TYRO3 receptor tyrosine kinase subfamily. To verify the recognition specificity of the antibodies 6G12M11, 6G12M21, 6G12M31 and 6G12M41, the binding ability of these antibodies to other members of the AXL family is tested by the ELISA method.

TYRO3-Fc (ACRO Biosystems), MER-His (ACRO Biosystems), and AXL-His (Sino Biological Inc.) each were coated at 1 μg/ml on an ELISA strip, which was held at 4°C overnight. After the ELISA strips were washed with PBST, 10% fetal bovine serum was added to the ELISA strip, which was blocked for 1 hour at 37°C. Antibodies 6G12M11, 6G12M21, 6G12M31 and 6G12M41 were added to react at 37° C for 1 hour. After the ELISA strips were washed with PBST, horseradish peroxidase-labeled goat anti-human IgG Fab secondary antibodies (Goat Anti-Human IgG (Fab')2 (HRP), Abcam) were added to react for 30 minutes at 37°C. The ELISA strips were washed 5 times with PBST, and patted and dried on absorbent paper to remove residual droplets as much as possible. 100 μl of TMB (eBioscience, #85-00-420) was added to each well, and the ELISA strips were held for 1.5 min at room temperature (20±5°C) in the dark. 100 μl of 2N $H_2SO_4$ stop solution was added to each well to terminate substrate reaction. The OD value was read at 450 nm by a microplate reader to analyze the binding ability of antibodies to the proteins.

Results are shown in FIGs. 2-5. It can be seen that the antibodies 6G12M11, 6G12M21, 6G12M31, and 6G12M41 can specifically recognize the target antigen AXL, but do not bind to all the other proteins TYRO3 and MER in the same family as AXL.

**Example 4. Specific recognition of cell surface antigens by antibodies**

The binding of AXLs on the surfaces of human non-small cell lung cancer A549 cells, human breast cancer MDA-MB-231 cells, and renal clear cell adenocarcinoma 786-O cells to antibodies 6G12M11, 6G12M21, 6G12M31, and 6G12M41 was tested by flow cytometry. Cells in a logarithmic growth phase were collected; the cell density was regulated to $5 \times 10^6$ cells/mL; and pre-cooling was performed on ice. The antibodies 6G12M11, 6G12M21, 6G12M31, and 6G12M41 were diluted to 20 μg/ml with pre-cooled normal saline containing 2% FBS. 100 μl of cells were taken, and an equal volume of each of the foregoing diluted antibodies was added to the cells to react for 30 minutes at 4° C in the dark. After the completion of reaction, the cells were washed twice with pre-cooled physiological saline containing 2% FBS (6000 rpm, 45 s). The secondary antibody PE Mouse Anti-Human IgG (BD Pharmingen) was diluted at 1:5 with pre-cooled physiological saline containing 2% FBS, and 100 μL of the secondary antibody was taken for resuspending the cells, with a reaction conducted for 30 minutes at 4° C in the dark. After the reaction, the cells were washed twice with pre-cooled physiological saline containing 2% FBS (6000 rpm, 45 s). The cells were resuspended by using 400 μl of 1% paraformaldehyde. The binding of antibodies to cell surface antigens was analyzed by means of a flow cytometer (BD Calibur).

The results are shown in FIGs. 6, 7 and 8. It can be seen that the antibodies 6G12M11 and 6G12M21 can specifically recognize, in a dose-dependent way, the AXLs on the surfaces of human non-small cell lung cancer A549 cells, human breast cancer MDA-MB-231 cells and renal clear cell adenocarcinoma 786-O cells, and the antibodies 6G12M11 and 6G12M21 have similar recognition capabilities. The antibodies 6G12M11 and 6G12M21 bind to the A549 cells at EC50 values of 0.46 μg/mL and 0.78 μg/mL, respectively; to the MDA-MB-231 cells at EC50 values of 0.59 μg/mL and 0.39 μg/mL, respectively; and to the 786-O cells at EC50 values of 0.43 μg/mL and 0.31 μg/mL, respectively.

The results are as shown in FIGs. 9 and 10. The results show that the antibodies 6G12M21, 6G12M31, and

6G12M41 can specifically recognize, in a dose-dependent way, the AXLs on the surfaces of human breast cancer MDA-MB-231 cells and renal clear cell adenocarcinoma 786-O cells, and the antibodies 6G12M21, 6G12M31, and 6G12M41 have similar recognition capabilities. The binding to the MDA-MB-231 cells occurs at an EC50 value of 0.64 μg/mL in the case of 6G12M21, 0.60 μg/mL in the case of 6G12M31, and 0.54 μg/mL in the case of 6G12M41. The binding to the 786-O cells occurs at an EC50 value of 0.40 μg/mL in the case of 6G12M21, 0.53 μg/mL in the case of 6G12M31, and 0.47 μg/mL in the case of 6G12M41.

**Example 5. Internalization activities of antibodies**

The internalization activities of antibodies 6G12M11, 6G12M21, 6G12M31, and 6G12M41 on the human non-small cell lung cancer A549 cells, human breast cancer MDA-MB-231 cells, and renal clear cell adenocarcinoma 786-O cells were tested by flow cytometry. Cells in a logarithmic growth phase were collected; the cell density was regulated to $5\times10^6$ cells/mL; and pre-cooling was performed on ice. The antibodies 6G12M11, 6G12M21, 6G12M31, and 6G12M41 were diluted to different concentrations with pre-cooled normal saline containing 2% FBS. 100 μl of cells were taken, and an equal volume of each of the foregoing diluted antibodies was added to the cells for incubation for 30 minutes at 4° C. After the completion of incubation, the cells were washed three times with pre-cooled physiological saline containing 2% FBS. The cells were continuously held at 4° C or 37° C for incubation for 2 hours, and then washed twice. The secondary antibody PE Mouse Anti-Human IgG (BD Pharmingen) was diluted at 1:5 with pre-cooled physiological saline containing 2% FBS, and 100 μL of the secondary antibody was taken for resuspending the cells, with a reaction conducted for 30 minutes at 4° C in the dark. After the completion of reaction, the cells were washed three times. The cells were resuspended by using 400 μl of 1% paraformaldehyde. The fluorescence intensity on the surfaces of cells cultured at different temperatures was analyzed for the antibodies by the flow cytometer (BD Calibur), and the internalization efficiency of each antibody was calculated according to the following formula:

$$\text{Internalization efficiency} = (\text{total surface MFI at } 4°\text{C - total surface MFI at } 37°\text{C})/\text{total surface MFI at } 4°\text{C} \times 100\%.$$

The results are shown in FIGs. 11, 12 and 13. It can be seen that the antibodies 6G12M11 and 6G12M21 can be effectively internalized on the human non-small cell lung cancer A549 cells, human breast cancer MDA-MB-231 cells and renal clear cell adenocarcinoma 786-O cells, at similar efficiency, which indicates that the antibodies 6G12M11 and 6G12M21 have good internalization activities.

As shown in FIGs. 14 and 15, it can be seen that the antibodies 6G12M21, 6G12M31, and 6G12M41 can be effectively internalized on both the human breast cancer MDA-MB-231 cells and renal clear cell adenocarcinoma 786-O cells, at similar efficiency. It can be seen that the antibodies 6G12M21, 6G12M31, and 6G12M41 have good internalization activities.

**Example 6. Inhibition of proliferation of tumor cells by immunoconjugates**

An immunoconjugate 6G12M41-ADC was constructed as an example of ADC by using a small molecule linker and MMAF, the biological activity of the 6G12M41-based immunoconjugate was evaluated, and the potential of the antibody 6G12M41 to construct a small-molecule antibody-drug conjugate (ADC) and other immunoconjugates was analyzed.

A certain number of cells (human breast cancer MDA-MB-231 cells) in a logarithmic growth phase were inoculated in a 96-well culture plate, and after 24 hours of adherent growth, different concentrations of drugs were added to take effect for 72 hours. After the drug effect ended, CCK-8 (Dojindo, Dojindo Laboratories, Japan) was added to the culture plate at 10 μl per well, and incubation was carried out in a 37°C incubator containing 5% carbon dioxide for 3-5 hours. The OD value was determined at 450 nm wavelength with a microplate reader, and the cell growth inhibition rate was calculated according to the following formula:

$$\text{Inhibition rate} = (\text{OD value of control hole-OD value of dosing hole})/\text{OD value of control hole} \times 100\%$$

According to the inhibition rate at each concentration, the median inhibitory concentration IC50 was then calculated.

Results are as shown in FIG. 16. The results show that the antibody 6G12M41 conjugated with MMAF (6G12M41-ADC) exhibits a significant proliferation inhibitory effect on the breast cancer MDA-MB-231, with an IC50 of 8.298 nM.

**Example 7. In vivo tumor-inhibiting activity of immunoconjugate**

Taking the immunoconjugate 6G12M41-ADC as an example, its in vivo tumor-inhibiting activity was analyzed.

B-NDG mice were subcutaneously inoculated with breast cancer MDA-MB-231 cells to establish subcutaneous breast cancer animal models for evaluating the in vivo tumor-inhibiting activity of 6G12M41-ADC. 8-week-old female B-NDG mice (Biocytogen Jiangsu Gene Biotechnology Co., Ltd.) were selected, and MDA-MB-231 cells were cultured in a GIBCO Leibovitz's L-15 medium containing 10% inactivated fetal bovine serum. The MDA-MB-231 cells in an exponential growth phase were collected and inoculated subcutaneously on the right side of each B-NDG mouse at a concentration of $1\times10^7$ cells/0.1 mL and a volume of 0.1 mL/mouse. After inoculation, when the mean tumor volume reached 92 $mm^3$, the mice were randomly divided into 4 groups according to the tumor size, each with 6 animals, namely G1 PBS, G2 6G12M41 (10 mg/kg), G3 6G12M41-ADC (10 mg/kg), and G4 6G12M41-ADC (5 mg/kg). The administration was performed by intraperitoneal injection, once a week, twice consecutively. The experiment ended on the 25th day of grouped administration. The body weight and tumor volume of each mouse were measured twice a week during administration and observation, and measured values were recorded. At the end of the experiment, the animals were euthanized, and the mice were dissected to obtain tumors, which were then weighed. The therapeutic effect was evaluated based on the relative tumor inhibition rate (TGI), and the safety was evaluated based on the changes in body weight and death of animals. $TGI_{TV}$ (%) = [1-(Ti-T0)/(Vi-V0)]$\times$100% (Ti: mean tumor volume of the treatment group on Day i, and T0: mean tumor volume of the treatment group on Day 0; Vi: mean tumor volume of the solvent control group on Day i of administration, and V0: mean tumor volume of the solvent control group on Day 0 of administration).

Results are shown in FIGs. 17 and 18. During the experiment, experimental animals in the administration group were in good activity and eating state during the administration, with body weight increased to a certain extent. This indicates that the experimental animals have a better tolerance to the test product. On Day 25 of grouped administration, the mean tumor volume of the PBS control group was $668\pm45$ $mm^3$, and the mean tumor volumes of the administration groups G2-G4 were $458\pm14$ $mm^3$, $311\pm26$ $mm^3$, and $395\pm14$ $mm^3$, respectively, with the $TGI_{TV}$S of 36.5%, 62.0, and 47.5%, respectively.

Each test product has a significant inhibitory effect on the growth of subcutaneous transplanted tumors of MDA-MB-231 cells, without any obvious toxic and side effects on animals while exerting drug effects, exhibiting good safety. In addition, the tumor-inhibiting effect of the test product 6G12M41-ADC is concentration-dependent.

The foregoing detailed description is provided by way of explanation and examples, and is not intended to limit the scope of the appended claims. Various changes of the embodiments listed in the present application until now would be obvious to those of ordinary skills in the art, and should be kept within the scope of the appended claims and equivalents thereof.

## Claims

1. An isolated antigen-binding protein, comprising at least one CDR in a VH as set forth in an amino acid sequence of SEQ ID NO: 1 or SEQ ID NO: 46; and comprising at least one CDR in a VL as set forth in an amino acid sequence of SEQ ID NO: 2.

2. The isolated antigen-binding protein according to claim 1, having one or more of the following properties:

   1) capability of binding to an AXL protein at a $K_D$ of $1 \times 10^{-7}$M or lower;
   2) capability of specifically recognizing an AXL protein expressed on a cell surface; and
   3) capability of mediating internalization after binding to the AXL protein expressed on the cell surface.

3. The isolated antigen-binding protein according to claim 2, wherein said AXL protein comprises a human AXL protein.

4. The isolated antigen-binding protein according to claim 3, wherein said human AXL protein comprises an amino acid sequence as set forth in SEQ ID NO: 39.

5. The isolated antigen-binding protein according to any one of claims 2-4, wherein said AXL protein comprises an extracellular domain.

6. The isolated antigen-binding protein according to claim 5, wherein said extracellular domain comprises an amino acid sequence as set forth in SEQ ID NO: 40.

7. The isolated antigen-binding protein according to claim 2, wherein said cell comprises a tumor cell.

8. The isolated antigen-binding protein according to claim 7, wherein said tumor comprises an AXL positive tumor.

9. The isolated antigen-binding protein according to claim 8, wherein said tumor is selected from the group consisting of a lung cancer, a skin cancer, a kidney cancer, a pancreatic cancer, a hematologic tumor, a breast cancer, an ovarian cancer, a lymphoma, and a myeloma.

10. The isolated antigen-binding protein according to claim 9, wherein said tumor is selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

11. The isolated antigen-binding protein according to claim 2, wherein said cell comprises a human cell.

12. The isolated antigen-binding protein according to claim 7 or 11, wherein said cell is selected from the group consisting of a human non-small cell lung cancer A549 cell, a human cutaneous squamous cell carcinoma A431 cell, a renal clear cell adenocarcinoma 786-O cell, a human pancreatic cancer MIA PaCa-2 cell, an erythroleukemia K562 cell, an acute T cell leukemia Jurkat cell, a human breast cancer MCF-7 cell, a human breast cancer MDA-MB-231 cell, a human breast cancer MDA-MB-468 cell, a human breast cancer SKBR3 cell, a human ovarian cancer SKOV3 cell, a lymphoma U-937 cell, a lymphoma Raji cell, a human myeloma U266, and a human multiple myeloma RPMI8226 cell.

13. The isolated antigen-binding protein according to claim 1, wherein said VH comprises HCDR1, HCDR2 and HCDR3.

14. The isolated antigen-binding protein according to claim 13, wherein said HCDR1 comprises an amino acid sequence as set forth in SEQ ID NO: 25.

15. The isolated antigen-binding protein according to claim 13, wherein said HCDR2 comprises an amino acid sequence as set forth in any one of SEQ ID NOs: 26, 44, and 45.

16. The isolated antigen-binding protein according to claim 13, wherein said HCDR3 comprises an amino acid sequence as set forth in SEQ ID NO: 27.

17. The isolated antigen-binding protein according to claim 1, wherein said VL comprises LCDR1, LCDR2, and LCDR3.

18. The isolated antigen-binding protein according to claim 17, wherein said LCDR1 comprises an amino acid sequence as set forth in SEQ ID NO: 28.

19. The isolated antigen-binding protein according to claim 17, wherein said LCDR2 comprises an amino acid sequence as set forth in SEQ ID NO: 29.

20. The isolated antigen-binding protein according to claim 17, wherein said LCDR3 comprises an amino acid sequence as set forth in SEQ ID NO: 30.

21. The isolated antigen-binding protein according to claim 1 or 13, wherein said VH comprises framework regions H-FR1, H-FR2, H-FR3, and H-FR4.

**22.** The isolated antigen-binding protein according to claim 21, wherein a C-terminus of said H-FR1 is directly or indirectly linked to an N-terminus of said HCDR1, and said H-FR1 comprises an amino acid sequence as set forth in SEQ ID NO: 7.

**23.** The isolated antigen-binding protein according to claim 22, wherein said H-FR1 comprises an amino acid sequence as set forth in any one of SEQ ID NOs: 11 and 15.

**24.** The isolated antigen-binding protein according to claim 21, wherein said H-FR2 is located between said HCDR1 and said HCDR2, and said H-FR2 comprises an amino acid sequence as set forth in SEQ ID NO: 8.

**25.** The isolated antigen-binding protein according to claim 24, wherein said H-FR2 comprises an amino acid sequence as set forth in SEQ ID NO: 12.

**26.** The isolated antigen-binding protein according to claim 21, wherein said H-FR3 is located between said HCDR2 and said HCDR3, and said H-FR3 comprises an amino acid sequence as set forth in SEQ ID NO: 9.

**27.** The isolated antigen-binding protein according to claim 26, wherein said H-FR3 comprises an amino acid sequence as set forth in SEQ ID NO: 13.

**28.** The isolated antigen-binding protein according to claim 21, wherein an N-terminus of said H-FR4 is linked to a C-terminus of said HCDR3, and said H-FR4 comprises an amino acid sequence as set forth in SEQ ID NO: 10.

**29.** The isolated antigen-binding protein according to claim 28, wherein said H-FR4 comprises an amino acid sequence as set forth in SEQ ID NO: 14.

**30.** The isolated antigen-binding protein according to any one of claims 1, 13-16, and 22-29, wherein said VH comprises an amino acid sequence as set forth in any one of SEQ ID NOs: 3, 5, 42, and 43.

**31.** The isolated antigen-binding protein according to claim 1 or 17, wherein said VL comprises framework regions L-FR1, L-FR2, L-FR3, and L-FR4.

**32.** The isolated antigen-binding protein according to claim 31, wherein a C-terminus of said L-FR1 is directly or indirectly linked to an N-terminus of said LCDR1, and said L-FR1 comprises an amino acid sequence as set forth in SEQ ID NO: 16.

**33.** The isolated antigen-binding protein according to claim 32, wherein said L-FR1 comprises an amino acid sequence as set forth in any one of SEQ ID NOs: 20 and 24.

**34.** The isolated antigen-binding protein according to claim 31, wherein said L-FR2 is located between said LCDR1 and said LCDR2, and said L-FR2 comprises an amino acid sequence as set forth in SEQ ID NO: 17.

**35.** The isolated antigen-binding protein according to claim 34, wherein said L-FR2 comprises an amino acid sequence as set forth in SEQ ID NO: 21.

**36.** The isolated antigen-binding protein according to claim 31, wherein said L-FR3 is located between said LCDR2 and said LCDR3, and said L-FR3 comprises an amino acid sequence as set forth in SEQ ID NO: 18.

**37.** The isolated antigen-binding protein according to claim 36, wherein said L-FR3 comprises an amino acid sequence as set forth in SEQ ID NO: 22.

**38.** The isolated antigen-binding protein according to claim 31, wherein an N-terminus of said L-FR4 is linked to a C-terminus of said LCDR3, and said L-FR4 comprises an amino acid sequence as set forth in SEQ ID NO: 19.

**39.** The isolated antigen-binding protein according to claim 38, wherein said L-FR4 comprises an amino acid sequence as set forth in SEQ ID NO: 23.

**40.** The isolated antigen-binding protein according to any one of claims 1, 17-20, and 32-39, wherein said VL comprises an amino acid sequence as set forth in any one of SEQ ID NOs: 4 and 6.

**41.** The isolated antigen-binding protein according to any one of claims 1-4, 6-11, 13-20, 22-29, and 32-39, comprising an antibody heavy-chain constant region, which is derived from a human IgG heavy-chain constant region.

**42.** The isolated antigen-binding protein according to claim 41, wherein said antibody heavy-chain constant region is derived from a human IgG1 heavy-chain constant region or a human IgG4 heavy-chain constant region.

**43.** The isolated antigen-binding protein according to claim 42, wherein said antibody heavy-chain constant region comprises an amino acid sequence as set forth in any one of SEQ ID NOs: 33 and 41.

**44.** The isolated antigen-binding protein according to any one of claims 1-4, 6-11, 13-20, 22-29, 32-39, and 42-43, comprising an antibody light-chain constant region, which comprises a human Igκ constant region.

**45.** The isolated antigen-binding protein according to claim 44, wherein said antibody light-chain constant region comprises an amino acid sequence as set forth in SEQ ID NO: 34.

**46.** The isolated antigen-binding protein according to any one of claims 1-4, 6-11, 13-20, 22-29, 32-39, 42-43, and 45, comprising an antibody heavy chain, which comprises an amino acid sequence as set forth in any one of SEQ ID NOs: 35 and 37.

**47.** The isolated antigen-binding protein according to any one of claims 1-4, 6-11, 13-20, 22-29, 32-39, 42-43, and 45, comprising an antibody light chain, which comprises an amino acid sequence as set forth in any one of SEQ ID NOs: 36 and 38.

**48.** The isolated antigen-binding protein according to any one of claims 1-4, 6-11, 13-20, 22-29, 32-39, 42-43, and 45, comprising an antibody or an antigen-binding fragment thereof.

**49.** The isolated antigen-binding protein according to claim 48, wherein said antibody is selected from the group consisting of a monoclonal antibody, a single chain antibody, a chimeric antibody, a multispecific antibody, a humanized antibody, and a fully human antibody.

**50.** The isolated antigen-binding protein according to claim 48, wherein said antigen-binding fragment is selected from the group consisting of: Fab, Fab', $F(ab)_2$, Fv, $F(ab')_2$, scFv, di-scFv, and dAb fragments.

**51.** An immunoconjugate, comprising said isolated antigen-binding protein of any one of claims 1-50.

**52.** The immunoconjugate according to claim 51, further comprising at least one additional agent selected from the group consisting of a chemotherapeutic agent, a radioactive element, a cytostatic agents, and a cytotoxic agent.

**53.** The immunoconjugate according to claim 52, wherein said isolated antigen-binding protein is linked to said additional agent by a linker molecule.

**54.** The immunoconjugate according to claim 53, wherein said isolated antigen-binding protein and said additional agent are covalently linked to said linker molecule, respectively.

**55.** The immunoconjugate according to any one of claims 52-54, wherein said additional agent comprises maytansine or a derivative thereof.

**56.** The immunoconjugate according to claim 55, wherein said maytansine derivative comprises a maytansine derivative DM1.

**57.** One or more isolated nucleic acid molecules, encoding said isolated antigen-binding protein of any one of claims 1-50.

**58.** A vector, comprising said nucleic acid molecule of claim 57.

**59.** A cell, comprising said nucleic acid molecule of claim 57 or said vector of claim 58.

**60.** A pharmaceutical composition, comprising said isolated antigen-binding protein of any one of claims 1-50, said immunoconjugate of any one of claims 51-56, said nucleic acid molecule of claim 57, said vector of claim 58, and/or

said cell of claim 59, and optionally a pharmaceutically acceptable adjuvant.

**61.** A preparation method for said isolated antigen-binding protein of any one of claims 1-50, wherein the method comprises culturing said cell of claim 59 under a condition of allowing the expression of said isolated antigen-binding protein of any one of claims 1-50.

**62.** Use of said isolated antigen-binding protein of any one of claims 1-50, said immunoconjugate of any one of claims 51-56, said nucleic acid molecule of claim 57, said vector of claim 58, said cell of claim 59, and/or said pharmaceutical composition of claim 60 in the preparation of a drug for preventing, relieving and/or treating a tumor.

**63.** The use according to claim 62, wherein said tumor comprises an AXL positive tumor.

**64.** The use according to claim 63, wherein said tumor is selected from the group consisting of a lung cancer, a skin cancer, a kidney cancer, a pancreatic cancer, a hematologic tumor, a breast cancer, an ovarian cancer, a lymphoma, and a myeloma.

**65.** The use according to claim 64, wherein said tumor is selected from the group consisting of a non-small cell lung cancer, a cutaneous squamous cell carcinoma, a renal clear cell adenocarcinoma, a pancreatic cancer, an erythroleukemia, an acute T cell leukemia, a breast cancer, an ovarian cancer, a lymphoma, and a myeloma.

**66.** Use of said isolated antigen-binding protein of any one of claims 1-50 in the preparation of a diagnostic agent for diagnosing a disease or condition associated with the expression of said AXL protein.

**67.** A method for diagnosing a disease or condition associated with the expression of an AXL protein in a subject, comprising: bringing a sample derived from the subject and said isolated antigen-binding protein of any one of claims 1-50 into contact, and determining the presence and/or amount of a substance capable of specifically binding said isolated antigen-binding protein, in the sample.

**68.** A method for detecting AXL in a sample, comprising administering said isolated antigen-binding protein of any one of claims 1-50.

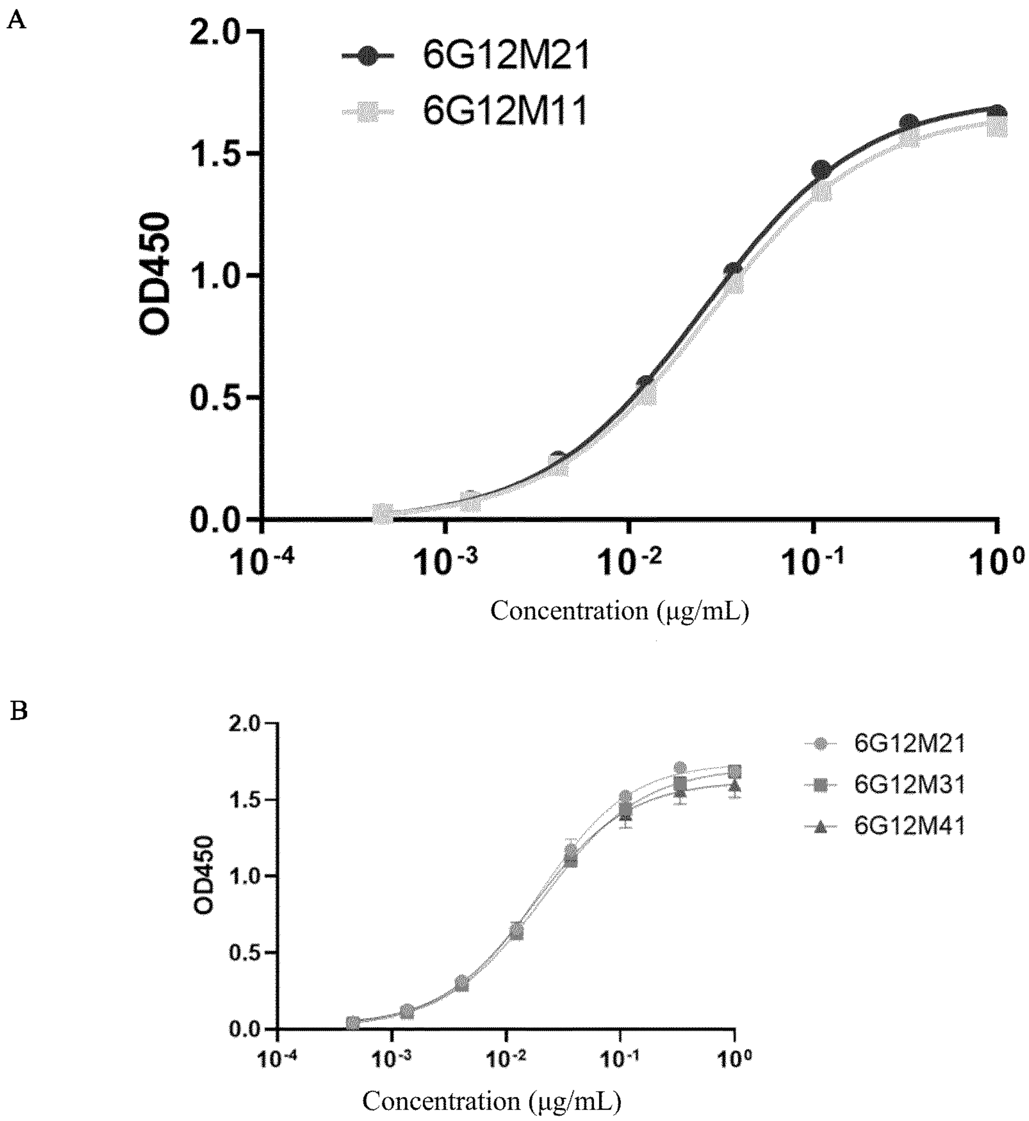
A
6G12M21
6G12M11
2.0
1.5
1.0
0.5
0.0
OD450
10-4
10-3
10-2
10-1
100
Concentration (μg/mL)
B
6G12M21
6G12M31
6G12M41
OD450
Concentration (μg/mL)

**FIG. 1**

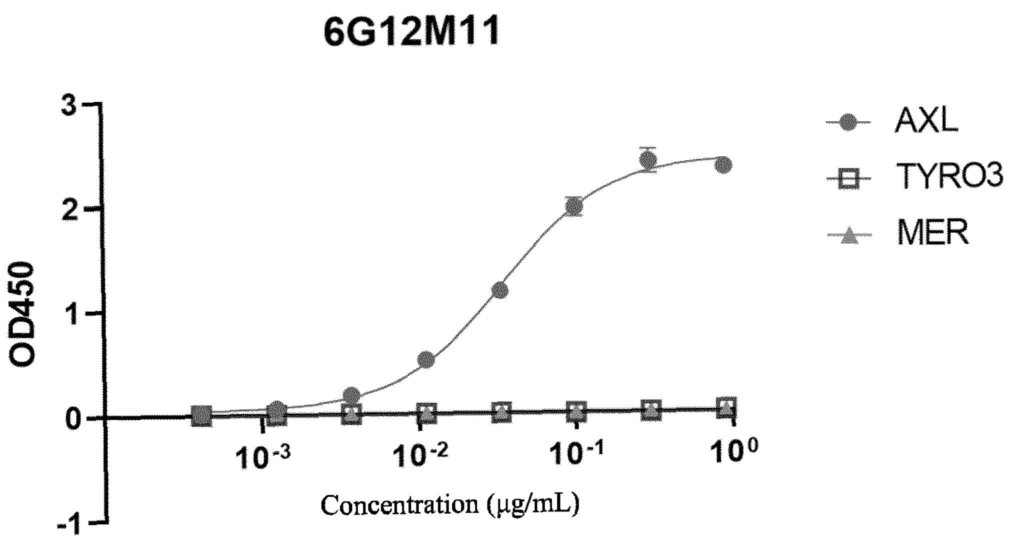
6G12M11
3
2
1
0
-1
OD450
10-3
10-2
10-1
100
Concentration (μg/mL)
AXL
TYRO3
MER

FIG. 2

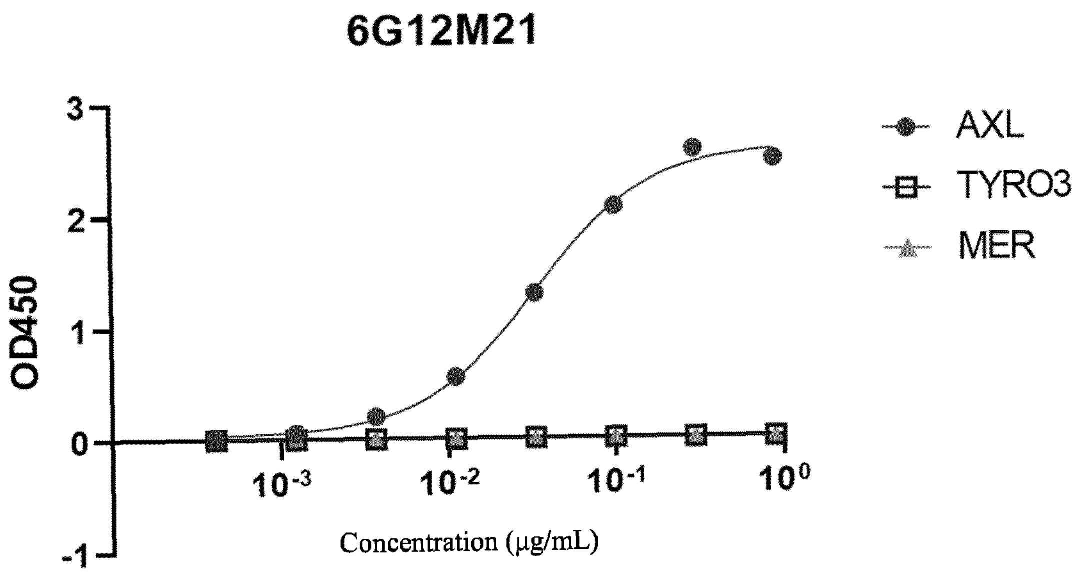
6G12M21
3
2
1
0
-1
OD450
10-3
10-2
10-1
100
Concentration (μg/mL)
AXL
TYRO3
MER

FIG. 3

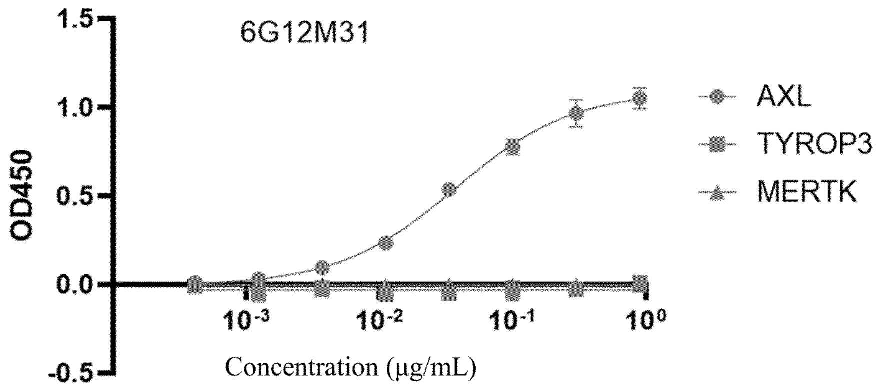


FIG. 4

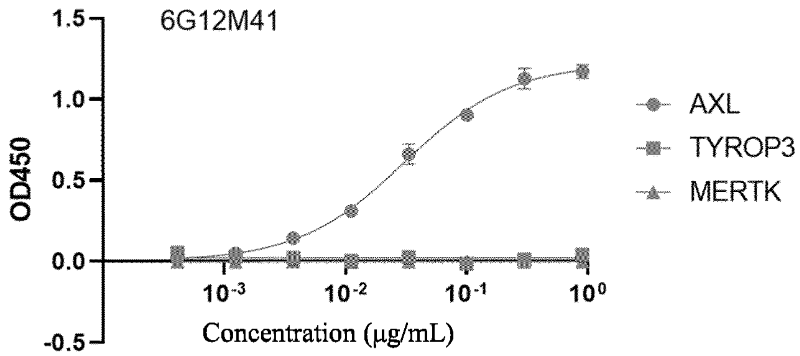


FIG. 5

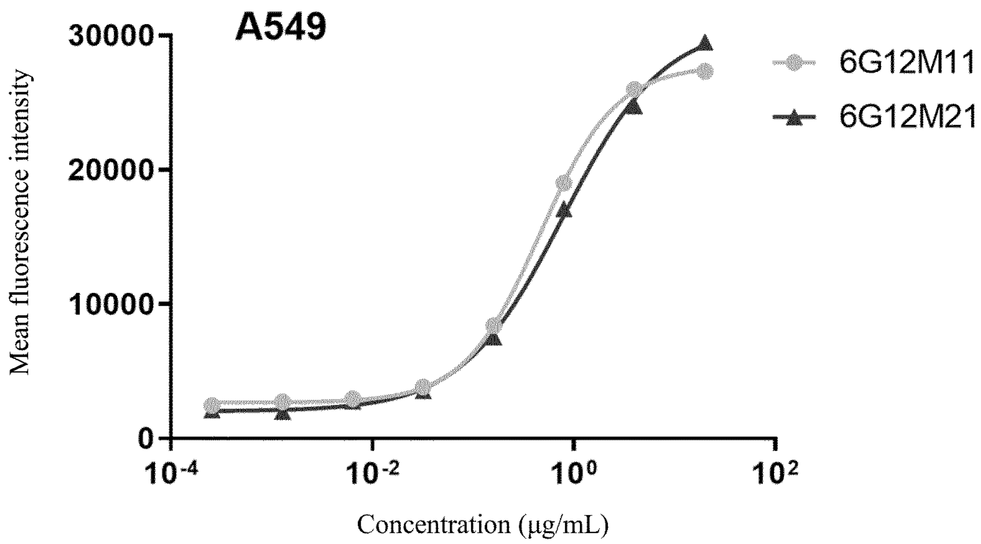


FIG. 6

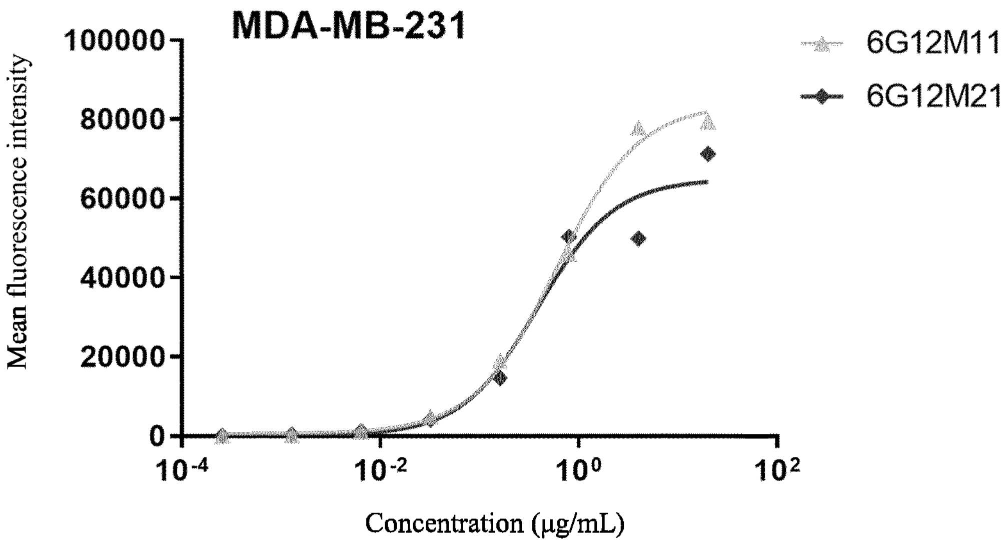
MDA-MB-231
6G12M11
6G12M21
Mean fluorescence intensity
100000
80000
60000
40000
20000
0
$10^{-4}$
$10^{-2}$
$10^{0}$
$10^{2}$
Concentration (μg/mL)

FIG. 7

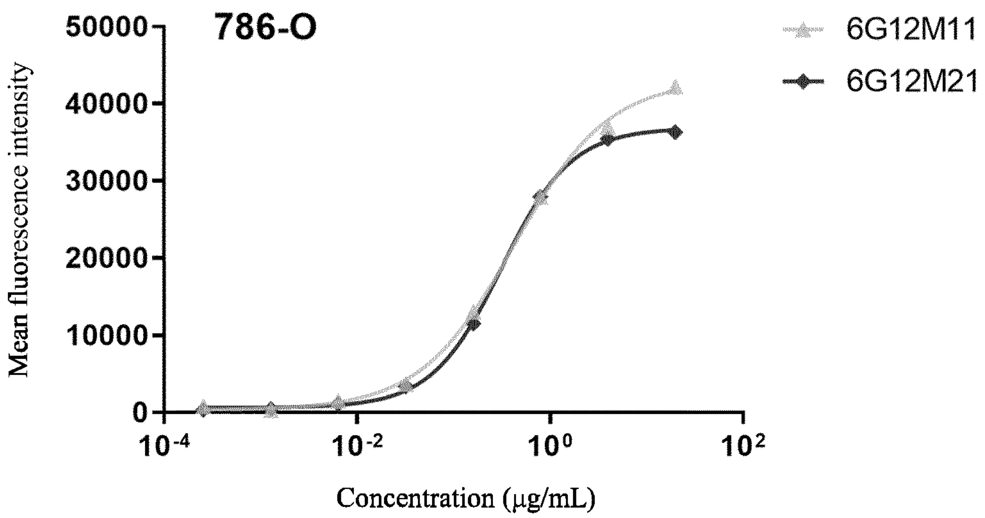
786-O
6G12M11
6G12M21
Mean fluorescence intensity
50000
40000
30000
20000
10000
0
$10^{-4}$
$10^{-2}$
$10^{0}$
$10^{2}$
Concentration (μg/mL)

FIG. 8

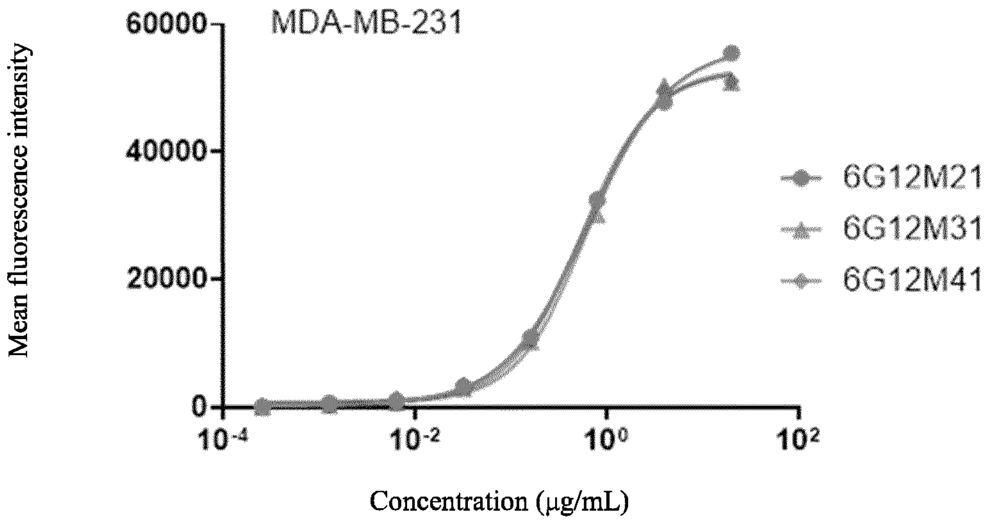
MDA-MB-231
Mean fluorescence intensity
60000
40000
20000
0
10⁻⁴
10⁻²
10⁰
10²
Concentration (μg/mL)
6G12M21
6G12M31
6G12M41

**FIG. 9**

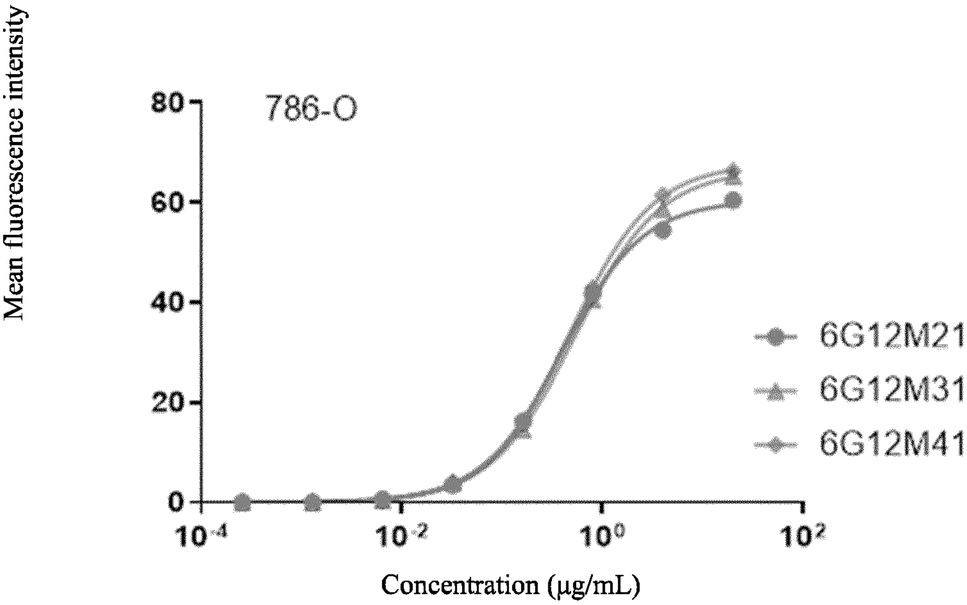
786-O
Mean fluorescence intensity
80
60
40
20
0
10⁻⁴
10⁻²
10⁰
10²
Concentration (μg/mL)
6G12M21
6G12M31
6G12M41

**FIG. 10**

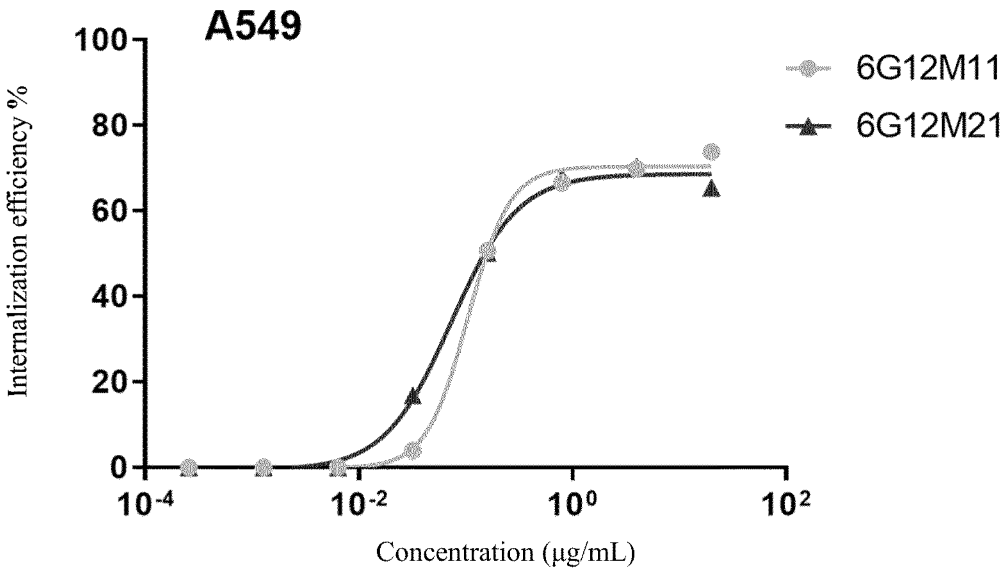
A549
100
80
60
40
20
0
Internalization efficiency %
10-4
10-2
100
102
Concentration (μg/mL)
6G12M11
6G12M21

FIG. 11

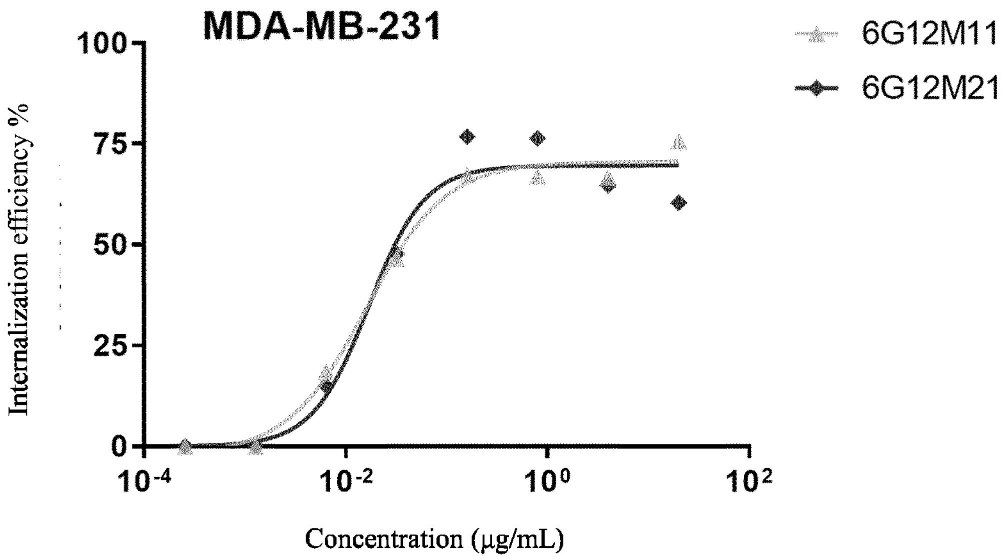
MDA-MB-231
100
75
50
25
0
Internalization efficiency %
10-4
10-2
100
102
Concentration (μg/mL)
6G12M11
6G12M21

FIG. 12

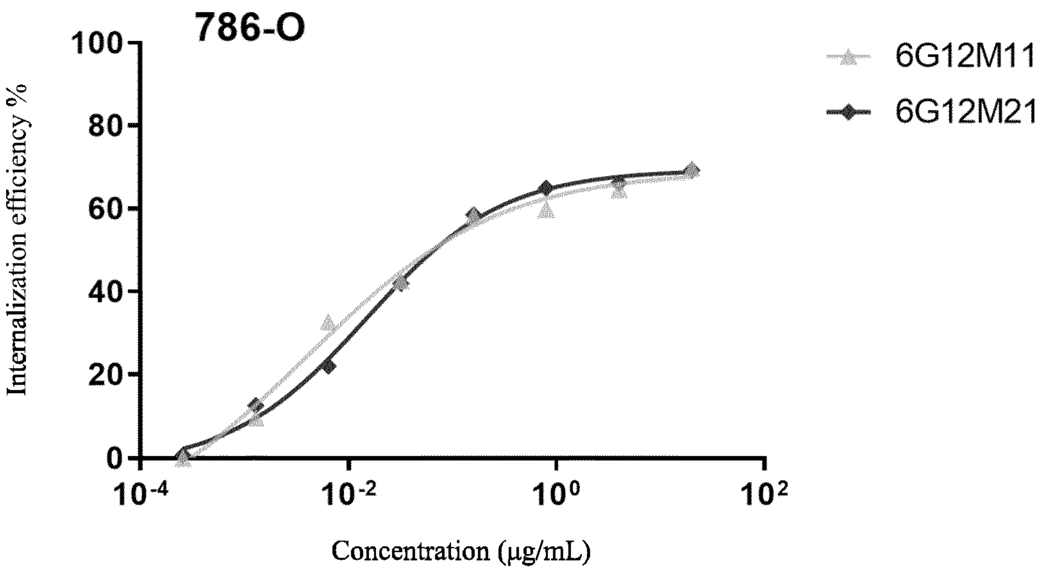
786-O
100
80
60
40
20
0
Internalization efficiency %
10-4
10-2
100
102
Concentration (μg/mL)
6G12M11
6G12M21

FIG. 13

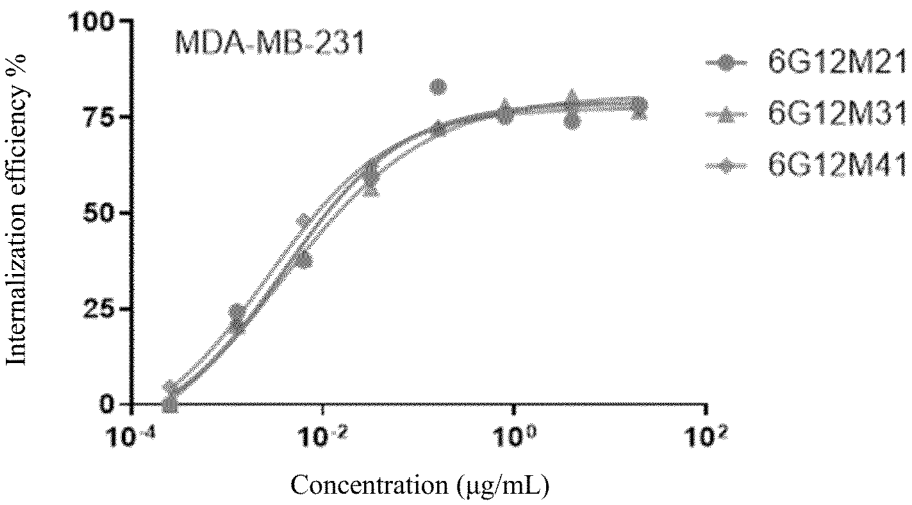
MDA-MB-231
100
75
50
25
0
Internalization efficiency %
10-4
10-2
100
102
Concentration (μg/mL)
6G12M21
6G12M31
6G12M41

FIG. 14

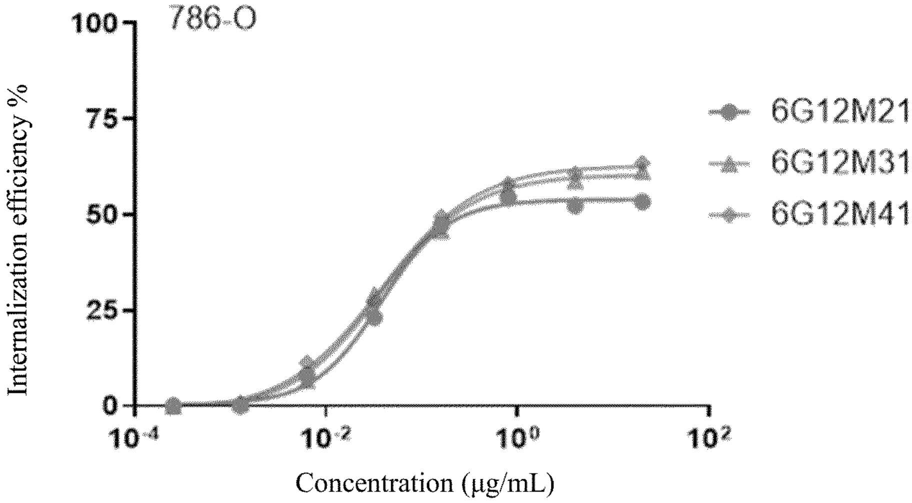
786-O
100
75
50
25
0
Internalization efficiency %
6G12M21
6G12M31
6G12M41
10-4
10-2
100
102
Concentration (μg/mL)

FIG. 15

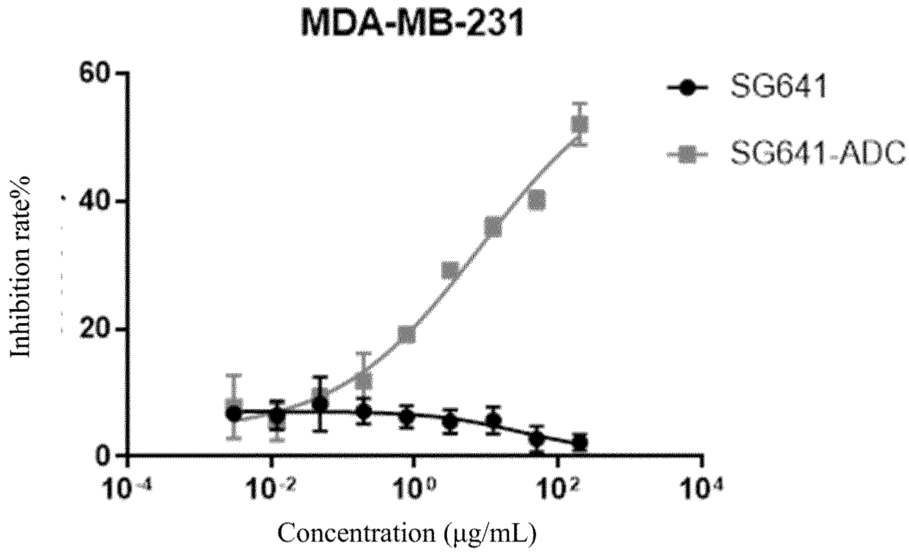
MDA-MB-231
60
40
20
0
Inhibition rate%
SG641
SG641-ADC
10-4
10-2
100
102
104
Concentration (μg/mL)

**FIG. 16**

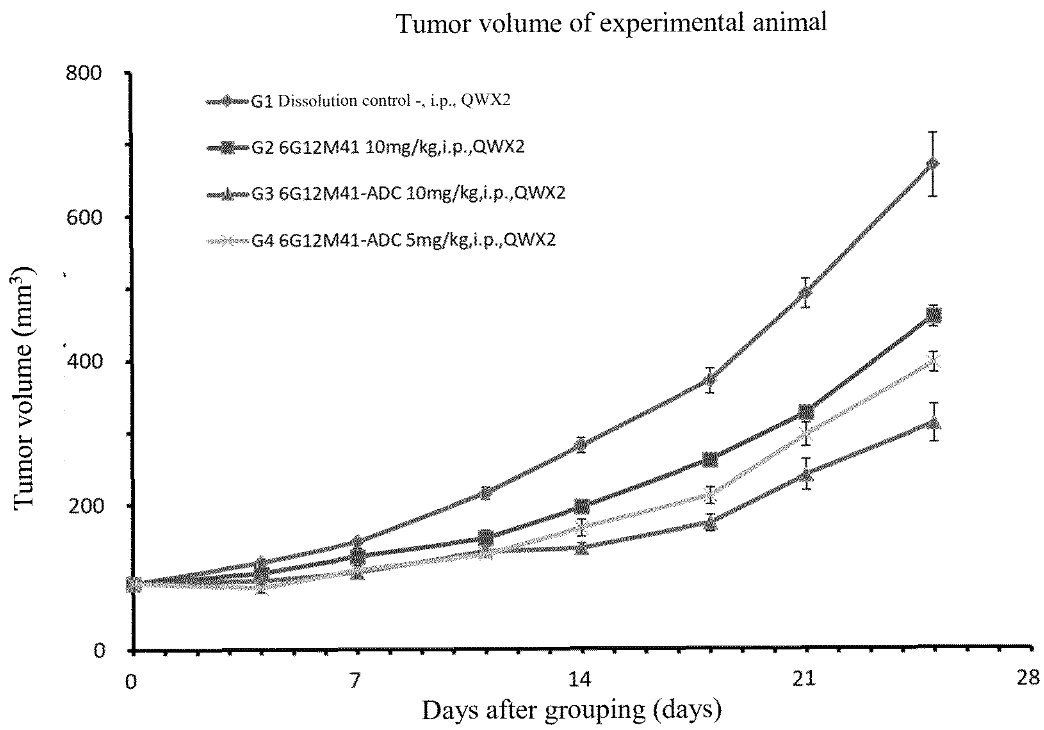
Tumor volume of experimental animal
G1 Dissolution control -, i.p., QWX2
G2 6G12M41 10mg/kg,i.p.,QWX2
G3 6G12M41-ADC 10mg/kg,i.p.,QWX2
G4 6G12M41-ADC 5mg/kg,i.p.,QWX2
Tumor volume ($mm^3$)
800
600
400
200
0
0
7
14
21
28
Days after grouping (days)

**FIG. 17**

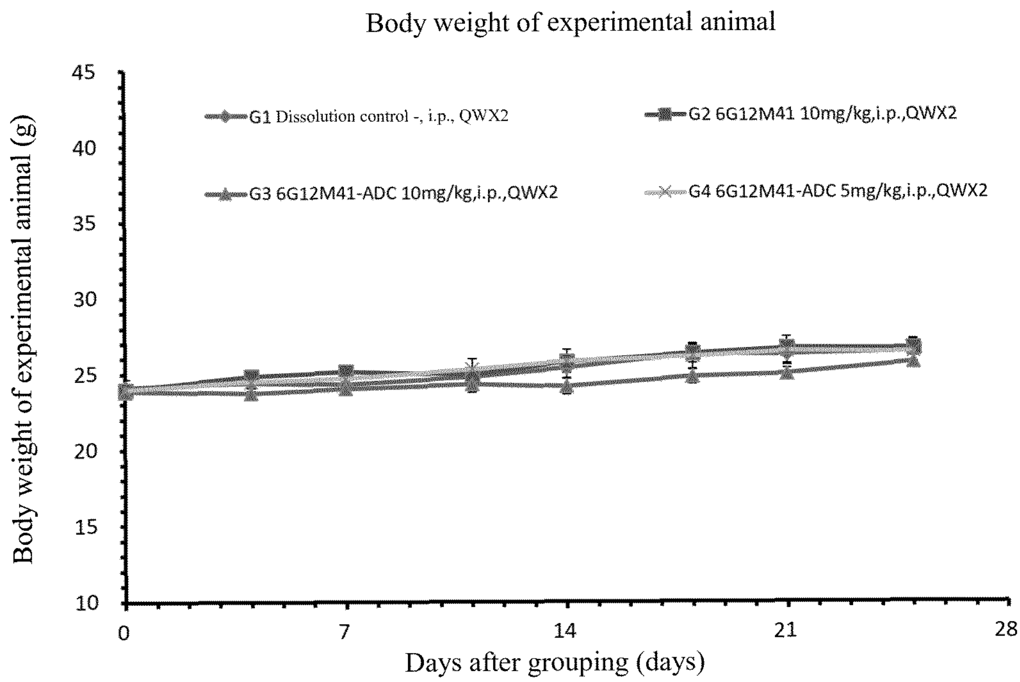
Body weight of experimental animal
G1 Dissolution control -, i.p., QWX2
G2 6G12M41 10mg/kg,i.p.,QWX2
G3 6G12M41-ADC 10mg/kg,i.p.,QWX2
G4 6G12M41-ADC 5mg/kg,i.p.,QWX2
Body weight of experimental animal (g)
45
40
35
30
25
20
15
10
0
7
14
21
28
Days after grouping (days)

**FIG. 18**

**INTERNATIONAL SEARCH REPORT**

International application No.
**PCT/CN2020/132456**

**A. CLASSIFICATION OF SUBJECT MATTER**

C07K 16/30(2006.01)i; C12N 15/13(2006.01)i; A61K 47/68(2017.01)i; G01N 33/68(2006.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; C12N; A61K; A61P; G01N

Documentation searched other than minimum documentation to the extent that such documents are included in the fields searched

Electronic data base consulted during the international search (name of data base and, where practicable, search terms used)

GBTXT; SGTXT; EPTXT; USTXT; WOTXT; VEN; PUBMED; ISI WEB OF SCIENCE: antibody, AXL, 受体酪氨酸激酶, 抗体, 肿瘤, SEQ ID NOs: 25-30, 3-24, 42-45

**C. DOCUMENTS CONSIDERED TO BE RELEVANT**

| Category* | Citation of document, with indication, where appropriate, of the relevant passages | Relevant to claim No. |
|---|---|---|
| PX | CN 110540592 A (HANGZHOU SUMGEN BIOTECHNOLOGY CO., LTD. et al.) 06 December 2019 (2019-12-06)<br>description, paragraphs 5-42, sequence table | 1-3, 5, 7-31, 34-35, 48-68 |
| X | CN 110003338 A (BEIJING MIANYI FANGZHOU MEDICINE TECHNOLOGY CO., LTD.) 12 July 2019 (2019-07-12)<br>description, paragraphs 7-31 | 1, 13, 17, 21, 31, 41-45, 48-62 |
| X | CN 105367657 A (SHANGHAI INSTITUTE OF BIOLOGICAL PRODUCTS CO., LTD.) 02 March 2016 (2016-03-02)<br>description, paragraphs 4-96, embodiment 9 | 1, 13, 17, 19, 21, 31, 41-45, 48-62 |
| A | WO 2016091891 A1 (INSERM et al.) 16 June 2016 (2016-06-16)<br>entire document | 1-68 |
| A | WO 2016187354 A1 (AGENSYS, INC.) 24 November 2016 (2016-11-24)<br>entire document | 1-68 |
| A | WO 2019051586 A1 (NATIONAL RESEARCH COUNCIL OF CANADA) 21 March 2019 (2019-03-21)<br>entire document | 1-68 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 February 2021** | **26 February 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/132456**

**C. DOCUMENTS CONSIDERED TO BE RELEVANT**

| Category* | Citation of document, with indication, where appropriate, of the relevant passages | Relevant to claim No. |
|---|---|---|
| A | MOODY, G. et al. "Antibody-mediated neutralization of autocrine Gas6 inhibits the growth of pancreatic ductal adenocarcinoma tumors in vivo" *International Journal of Cancer,* Vol. 139, 11 May 2016 (2016-05-11), pp. 1340-1349 | 1-68 |
| A | YE, X. et al. "An anti-Axl monoclonal antibody attenuates xenograft tumor growth and enhances the effect of multiple anticancer therapies" *Oncogene,* Vol. 29, 05 July 2010 (2010-07-05), pp. 5254-5264 | 1-68 |

Form PCT/ISA/210 (second sheet) (January 2015)

**INTERNATIONAL SEARCH REPORT**

International application No.
**PCT/CN2020/132456**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/CN2020/132456**

| Patent document cited in search report | | | Publication date (day/month/year) | Patent family member(s) | | | Publication date (day/month/year) |
|---|---|---|---|---|---|---|---|
| CN | 110540592 | A | 06 December 2019 | WO | 2019228345 | A1 | 05 December 2019 |
| CN | 110003338 | A | 12 July 2019 | | None | | |
| CN | 105367657 | A | 02 March 2016 | CN | 105367657 | B | 13 September 2019 |
| | | | | CN | 110642952 | A | 03 January 2020 |
| WO | 2016091891 | A1 | 16 June 2016 | EP | 3229836 | B1 | 13 November 2019 |
| | | | | JP | 2017538415 | A | 28 December 2017 |
| | | | | US | 10398774 | B2 | 03 September 2019 |
| | | | | US | 2017340734 | A1 | 30 November 2017 |
| | | | | ES | 2764299 | T3 | 02 June 2020 |
| | | | | EP | 3229836 | A1 | 18 October 2017 |
| WO | 2016187354 | A1 | 24 November 2016 | EP | 3297662 | A1 | 28 March 2018 |
| | | | | EP | 3297662 | A4 | 13 March 2019 |
| | | | | JP | 2018525320 | A | 06 September 2018 |
| | | | | US | 2018134792 | A1 | 17 May 2018 |
| | | | | US | 10787516 | B2 | 29 September 2020 |
| WO | 2019051586 | A1 | 21 March 2019 | CA | 3075531 | A1 | 21 March 2019 |
| | | | | US | 2020277386 | A1 | 03 September 2020 |
| | | | | EP | 3681912 | A1 | 22 July 2020 |
| | | | | SG | 11202002331 Q | A | 29 April 2020 |
| | | | | KR | 20200067145 | A | 11 June 2020 |
| | | | | IL | 273157 | D0 | 30 April 2020 |
| | | | | AU | 2018333290 | A1 | 16 April 2020 |
| | | | | CN | 111263770 | A | 09 June 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3896111 A
- WO 2015036583 A


### Non-patent literature cited in the description


- **FRANKE et al.** *Oncogene,* 2003, vol. 22, 8983-8998
- **GORUPPI et al.** *Mol. Cell Biol.,* 2001, vol. 21, 902-915
- **LINGER R.M.** *Adv.Cancer Res.,* 2008, vol. 100, 35-83
- **VERMA A.** *Mol.Cancer Ther.,* 2011, vol. 10, 1763-1773
- **KORNDORFER et al.** *Proteins: Structure, Function, and Bioinformatics,* 2003, vol. 53 (1), 121-129
- **ROQUE et al.** *Biotechnol. Prog.,* 2004, vol. 20, 639-654
- **SASAKI T. et al.** *EMBO J.,* 2006, vol. 25, 80-87
- **KABAT et al.** Sequences of Immunological Interest. National Institute of Health, 1991
- Basic and Clinical Immunology. Appleton & Lange, 1994, 71
- **XU et al.** *Immunity,* 2000, vol. 13, 37-45
- **JOHNSON ; WU.** Methods in Molecular Biology. Human Press, 2003, vol. 248, 1-25
- **HAMERS-CASTERMAN et al.** *Nature,* 1993, vol. 363, 446-448
- **SHERIFF et al.** *Nature Struct. Biol.,* 1996, vol. 3, 733-736
- *CHEMICAL ABSTRACTS,* 35846-53-8
- *CHEMICAL ABSTRACTS,* 139504-50-0
- **BRUMMELL et al.** *Biochem,* 1993, vol. 32, 1180-8
- **DE WILDT et al.** *Prot. Eng.,* 1997, vol. 10, 835-41
- **KOMISSAROV et al.** *J. Biol. Chem.,* 1997, vol. 272, 26864-26870
- **HALL et al.** *J. Immunol.,* 1992, vol. 149, 1605-12
- **KELLEY ; O'CONNELL.** *Biochem.,* 1993, vol. 32, 6862-35
- **ADIB-CONQUY et al.** *Int. Immunol.,* 1998, vol. 10, 341-6
- **BEERS et al.** *Clin. Can. Res.,* 2000, vol. 6, 2835-43
- **W. R. PEARSON ; D. J. LIPMAN.** Improved Tools for Biological Sequence Comparison. *Proc. Natl. Acad. Sci.,* 1988, vol. 85, 2444-2448
- **D. J. LIPMAN ; W. R. PEARSON.** Rapid and Sensitive Protein Similarity Searches. *Science,* 1989, vol. 227, 1435-1441
- **S. ALTSCHUL ; W. GISH ; W. MILLER ; E. W. MYERS ; D. LIPMAN.** Basic Local Alignment Search Tool. *J. Mol. Biol.,* 1990, vol. 215, 403-410
- **SAMBROOK, J. ; FRITSCH, E. F. ; MANIAIS, T.** Molecular Cloning: A Laboratory Manual. Cold spring Harbor Laboratory Press, 1989